# EUROPEAN PATENT SPECIFICATION

(11) **EP 1 941 060 B1**
(45) Date of publication and mention of the grant of the patent: **29.02.2012**
(21) Application number: 06836609.5
(22) Date of filing: 27.10.2006
(51) Int. Cl.: C12N 9/10, C12Q 1/48, C12Q 1/68, G01N 33/567, A61K 48/00

(54) **PROTEIN DEMETHYLASES COMPRISING A JMJC DOMAIN**
PROTEIN-DEMETHYLASEN MIT EINER JMJC-DOMÄNE
PROTÉINE DÉMÉTHYLASE À DOMAINE JMJC

(30) Priority: 28.10.2005 US 731053 P; 05.04.2006 US 789437 P
(43) Date of publication of application: 09.07.2008
(73) Proprietor: The University of North Carolina At Chapel Hill, Chapel Hill, NC 27599-4105 (US)
(72) Inventor: ZHANG, Yi, Chapel Hill, North Carolina 27514 (US); TSUKADA, Yuichi, Chapel Hill, North Carolina 27514 (US); YAMANE, Kenichi, Chapel Hill, North Carolina 27516 (US); KLOSE, Robert John, Oxford OX2 7QR (GB)
(74) Representative: Markham, Lisouschka
(86) International application number: PCT/US2006/042126
(87) International publication number: WO 2007/053480

(56) References cited:
- WO-A1-99/24583
- US-A1- 2005 003 378
- US-B1- 7 098 012
- TREWICK S C ET AL: "Methylation: Lost in hydroxylation?" EMBO REPORTS, NATURE PUBLISHING GROUP, LONDON, GB, vol. 6, no. 4, 1 January 2005 (2005-01-01), pages 315-320, XP002446862 ISSN: 1469-221X
- AYOUB NABIEH ET AL: "A novel jmjC domain protein modulates heterochromatization in fission yeast." MOLECULAR AND CELLULAR BIOLOGY, vol. 23, no. 12, June 2003 (2003-06), pages 4356-4370, XP002548387 ISSN: 0270-7306
- KLOSE ROBERT J ET AL: "JmjC-domain-containing proteins and histone demethylation" NATURE REVIEWS GENETICS, MACMILLAN MAGAZINES, GB, vol. 7, no. 9, 1 September 2006 (2006-09-01), pages 715-727, XP002459709

## Description

### Field of the Invention

The present invention relates to a newly-identified family of demethylases as well as a novel demethylase assay; also disclosed are methods for identifying compounds that modulate the activity of a demethylase, methods of identifying candidate compounds for the treatment of cancer or hair loss, use of the demethylases of the invention to demethylate a protein, methods of modulating demethylase activity, methods of treating cancer, methods of treating hair loss, methods of modulating expression of pluripotency or differentiation markers, and methods of modulating gene expression including steroid hormone regulated genes.

### Background of the Invention

Proteins can post-translationally be N-methylated on amino groups of lysines and guanidino groups of arginines or carboxymethylated on aspartate, glutamate, or the protein C-terminus. Recent studies have provided indirect evidence suggesting roles for methylation in a variety of cellular processes such as RNA processing, receptor mediated signaling, and cellular differentiation (Aletta, J. M. et al. (1998) Trends Biochem. Sci. 23:89). However, for the most part, the specific methyltransferases, protein substrates, and specific roles played by methylation in these phenomena have not been identified. Protein methylation has been most widely studied in histones. The transfer of methyl groups from S-adenyosyl methionine (SAM) to histones is catalyzed by enzymes known as histone methyltransferases.

Covalent histone modifications play an important role in regulating chromatin dynamics and function (Strahl and Allis, (2000) Nature 403:41-45). One such modification, methylation, occurs on both lysine and arginine residues and participates in a diverse range of biological processes including heterochromatin formation, X-chromosome inactivation, and transcriptional regulation (Lachner et al., (2003) J. Cell Sci. 116:2117-2124; Margueron et al., (2005) Curr. Opin. Genet. Dev. 15:163-176). Unlike acetylation, which generally correlates with transcriptional activation, histone lysine methylation can signal either activation or repression depending on the particular lysine residue which is methylated (Zhang and Reinberg, (2001) Genes Dev. 15:2343-2360). Even within the same lysine residue, the biological consequence of methylation can differ depending on whether it is a mono-, di, or tri-methylation (Santos-Rosa et al., (2002) Nature 419:407-411; Wang et al., (2003) Mol. Cell. 12:475-487).

The steady state level of a covalent histone modification is controlled by a balance between enzymes that catalyze the addition and removal of a given modification. While this notion is generally true for acetylation, phosphorylation, and ubiquitylation, an enzyme capable of removing methyl-groups from a methyl-lysine residue has remained elusive until recently (Shi et al., (2004) Cell 119:941-953). Using a candidate approach, Shi and colleagues demonstrated that LSD1/BHC110, a nuclear amine oxidase homolog previously found in several histone deacetylase complexes (Hakimi et al., (2002) Proc. Natl. Acad. Sci. USA 99:7420-7425); Shi et al., (2003) Nature 422:735-738; You et al., (2001) Proc. Natl. Acad. Sci. USA 98:1454-1458), can specifically demethylate mono- and di-methyl H3 K4 in a FAD (flavin adenine dinucleotide)-dependent oxidative reaction. Although there are potential LSD1 homologs in *S*. *pombe,* no apparent LSD1 homologs exist in *S*. *cerevisiae* even though at least three distinct lysine residues on H3 can be methylated in this organism.

### Summary of the Invention

Protein methylation and carboxymethylation are mechanisms for modulating protein function through post-translational covalent modification. Methylation of histones plays an important role in regulating chromatin dynamics and transcription. While most covalent histone modifications are reversible, it was unknown whether methyl groups could be actively removed from histones until recently. The present invention is based in part on the discovery of JmjC domain-containing proteins, which the inventors have named JHDM1A, JHDM2 and JHDM3A (JmjC containing histone demethylase 1A, 2A and 3A) as well as Retinoblastoma Binding Protein-2 (RBP2/JARID1A) and JARID1B (PLU1), which specifically demethylate histone H3 at lysine 36 (H3-K36), H3-K9 or H3-K4. The function of the JmjC domain in histone demethylation is conserved. For example, a *S*. *cerevisiae* homologue of JHDM1 also has H3-K36 demethylase activity. Further, a mutation that mimics a loss of function mutant in the *S. pombe* JHDM1 homologue impaired the demethylase activity. In addition, both RBP2 and the *Drosophila* orthologue *Lid* (Little Imaginal Discs) are H3-K4 demethylases. Thus, the inventors have uncovered a novel protein demethylation mechanism and identified the JmjC domain as a signature motif for demethylases that is found in organisms from yeast to humans.

Accordingly, as a first aspect, the invention provides a method of detecting demethylase activity in a composition, the method comprising:
(a) contacting the composition with (i) a methylated protein substrate, (ii) Fe(II) and (iii) α-ketoglutarate under conditions sufficient for demethylation of the methylated protein substrate; and
(b) detecting the release of formaldehyde and/or succinic acid from the demethylation reaction; wherein the release of formaldehyde and/or succinic acid is an indicator of demethylase activity.

As a further aspect, the invention comprises a method of detecting demethylase activity, the method comprising:
(a) contacting a protein with (i) a methylated protein substrate, (ii) Fe(II), and (iii) α-ketoglutarate under conditions sufficient for demethylation of the methylated protein substrate; and
(b) detecting the release of formaldehyde and/or succinic acid from the demethylation reaction; wherein the release of formaldehyde and/or succinic acid is an indicator of demethylase activity.

As another aspect, the invention provides a method of identifying a compound that modulates the demethylase activity of a demethylase comprising a JmjC domain, the method comprising:
(a) contacting the demethylase with a methylated protein substrate in the presence of a test compound; and
(b) detecting the level of demethylation of the protein substrate under conditions sufficient for demethylation, wherein a change in demethylation of the protein substrate as compared with the level of demethylation in the absence of the test compound indicates that the test compound is a modulator of the demethylase activity of the demethylase.

As yet a further aspect, the present invention provides a method of identifying a candidate compound for treating cancer, the method comprising:
(a) contacting a demethylase (*e.g*., histone demethylase) comprising a JmjC domain with a methylated protein substrate (*e.g*., methylated histone substrate) in the presence of a test compound; and
(b) detecting the level of demethylation of the protein substrate under conditions sufficient for demethylation, wherein a change in demethylation of the protein substrate as compared with the level of demethylation in the absence of the test compound indicates that the test compound is a candidate compound for the treatment of cancer.

As still another aspect, the invention provides a method of identifying a candidate compound for treating hair loss, the method comprising:
(a) contacting a Hairless protein with a methylated protein substrate in the presence of a test compound; and
(b) detecting the level of demethylation of the protein substrate under conditions sufficient for demethylation, wherein a change in demethylation of the protein substrate as compared with the level of demethylation in the absence of the test compound indicates that the test compound is a candidate compound for the treatment of hair loss.

Further encompassed by the present invention is a method of demethylating a methylated protein, the method comprising contacting the methylated protein with a demethylase comprising a JmjC domain under conditions sufficient for demethylation, as well as the use of a JmjC domain-containing protein as a demethylase.

In particular embodiments of the foregoing methods, the demethylase is a histone demethylase and the methylated protein substrate is a methylated histone substrate.

The invention also encompasses a kit, the kit comprising:
(a) a demethylase comprising a JmjC domain; and
(b) written instructions for methods of using the JmjC domain-containing protein to carry out a demethylation reaction, and optionally additional reagents or apparatus for using the JmjC domain-containing protein to carry out a demethylation reaction.

These and other aspects of the invention are set forth in more detail in the description of the invention that follows.

### Brief Description of the Drawings

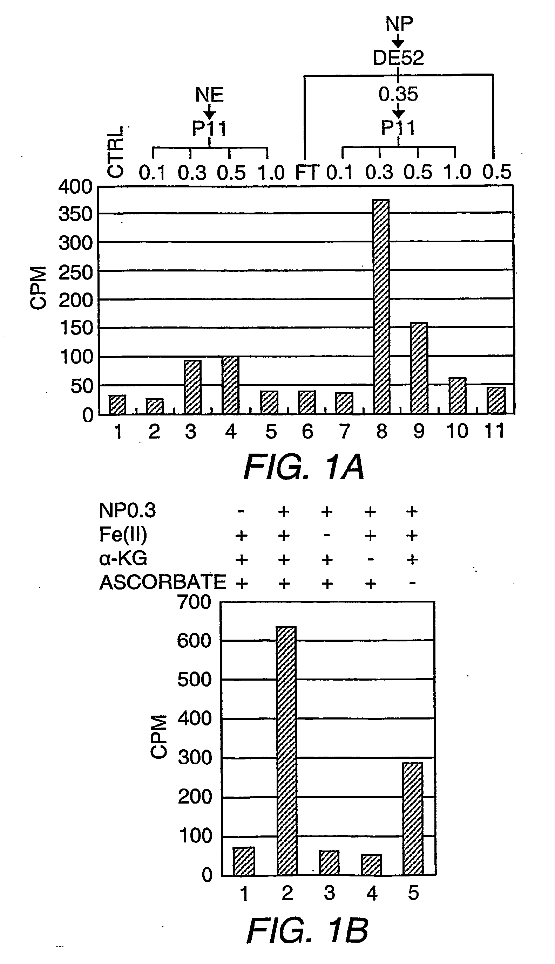

**Figures 1A-1B** demonstrate the establishment of a histone demethylase assay and identification of a demethylase activity in HeLa cells. **Figure 1A** shows the relative histone demethylase activities of the P11 column fractions derived from HeLa nuclear extracts and nuclear pellet fractions. The numbers above the panel represent the molar concentration of KCI in the elution buffers. **Figure 1** **B** shows that the demethylase activity depends on the presence of Fe(II), α-KG and proteins present in the 0.3 M P11 nuclear pellet fraction.

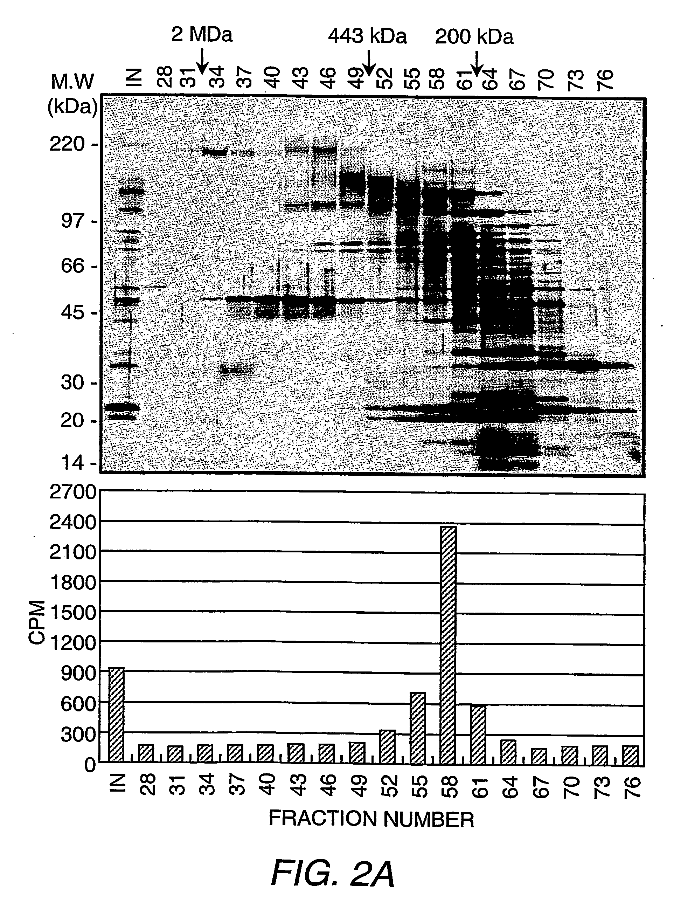

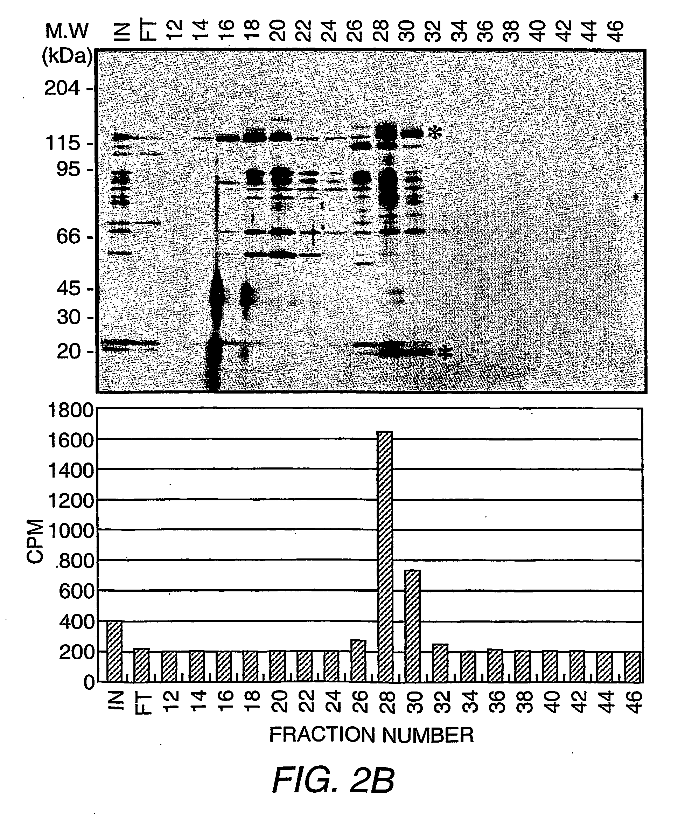

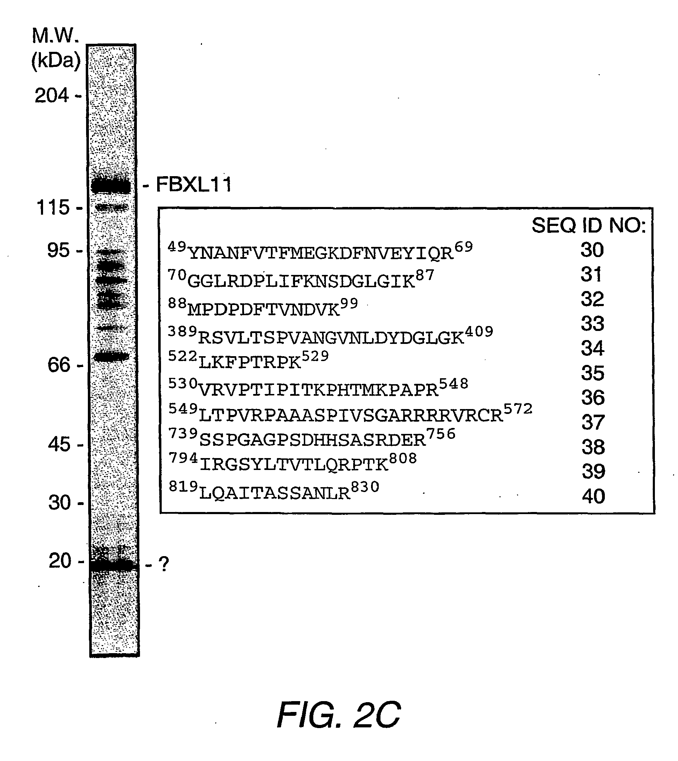

**Figures 2A-2C** show the purification and identification of a histone demethylase activity. **Figure 2A** shows a silver-stained protein gel (top panel) and histone demethylase activities (bottom panel) of the protein fractions derived from the gel-filtration Superose^{®} 6 column. The elution profile of the protein markers is indicated on top of the panel. **Figure 2B** is a silver-stained protein gel (top panel) and histone demethylase activities (bottom panel) of the protein fractions derived from a mini-MonoQ^{®} column. The candidate proteins that co-fractionated with the demethylase activity are indicated by *. The positions of the protein size markers on SDS-PAGE are indicated to the left of the panel. **Figure 2C** is a silver-stained protein gel containing the samples for protein identification. The top protein band was identified as FBXL11 (NP_036440). The peptides identified from mass spectrometry are listed. The numbers correspond to the amino acid numbers in the FBXL11 protein. The "?" represents an unidentified FBXL11-associated protein.

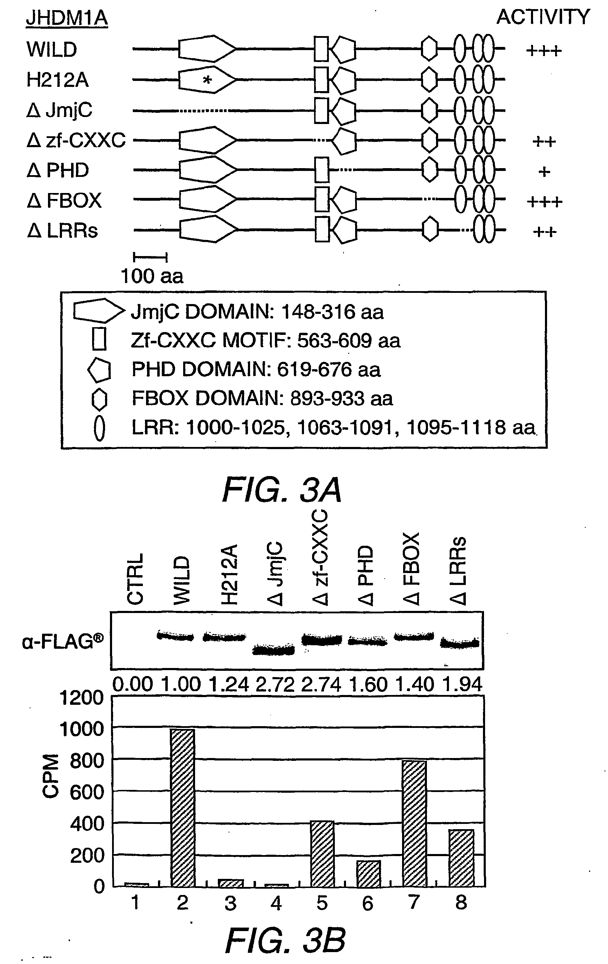

**Figures 3A-3B** show the characterization of the functional domains of the histone demethylase JHDM1A/FBXL11. **Figure 3A** is a schematic representation of the wild-type and mutant protein with their relative activities (right). The number of represents the relative activity. The identified functional domains are shown in the box using the SMART program. **Figure 3B** shows western blot (top panel) and demethylase assay (bottom panel) analysis of wild-type and mutant Flag^{®}-JHDMlA proteins expressed in COS-7 cells and immunoprecipitated prior to analysis. The relative amount of each immunoprecipitated protein, indicated with the numbers below the western blot, was quantified with the NIH ImageJ program and used for normalization of the activities.

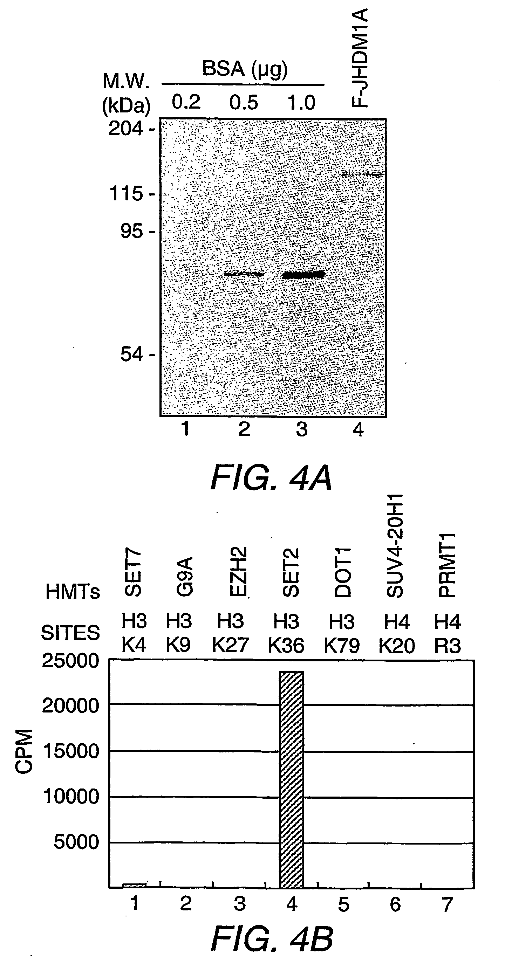

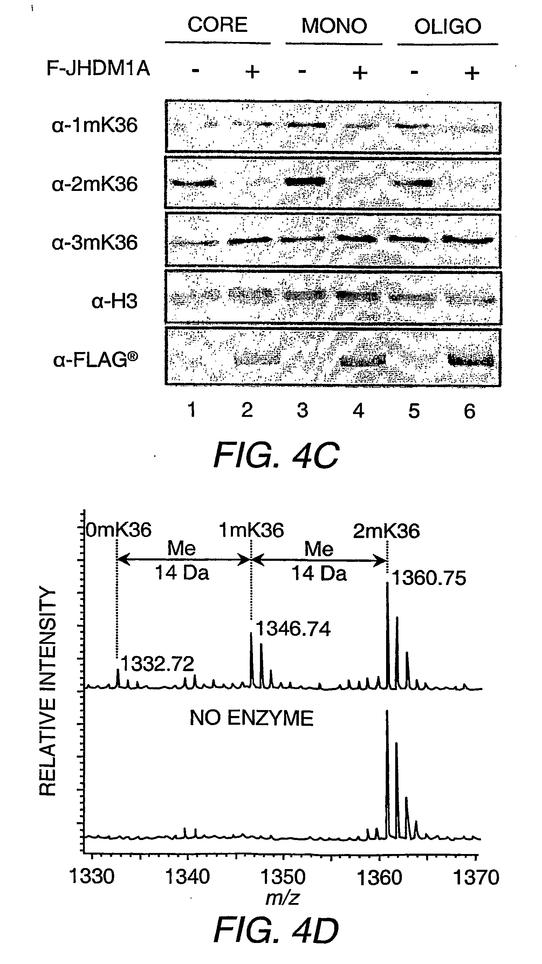

**Figures 4A-4D** show the characterization of the site- and methylation state-specificity of JHDM1A. **Figure 4A** is a Coomassie^{®}-stained protein gel of the purified Flag^{®}-JHDM1 protein expressed in Sf9 cells. **Figure 4B** shows the histone demethylase activity of Flag^{®}-JHDM1 toward various methylated histone substrates. The histone methyltransferases (HMTs) and their sites of methylation are indicated on top of the panel. **Figure 4C** shows western blot analysis of demethylation reactions using vanous histone substrates. Antibodies used are indicated to the left of the panel. **Figure 4D** shows mass spectrometry analysis of demethylation of a dimethyl-K36 peptide (STGGV2mKKPHRY-C; **SEQ ID NO:1**) by Flag^{®}-JHDM1. The enzyme/substrate molar ratio of the reaction was 1:40. Numbers represent the masses of the substrate and product peptides.

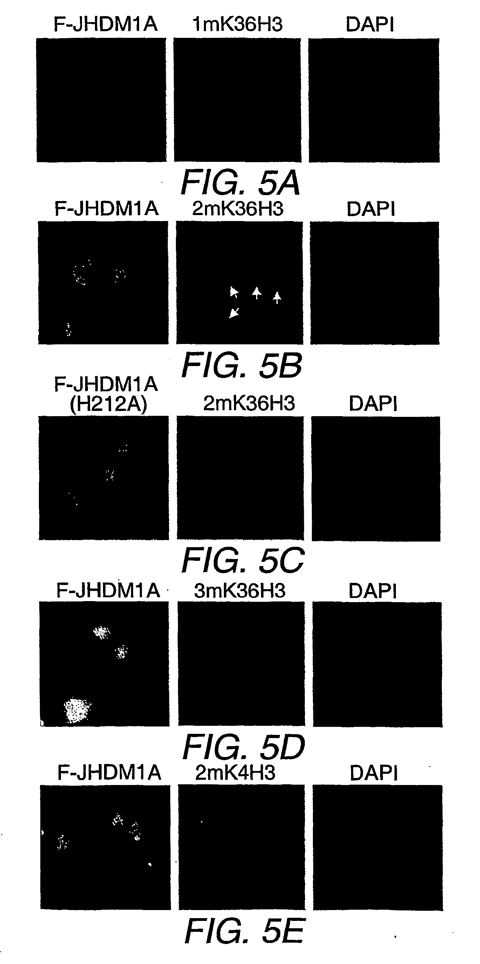

**Figures 5A-5E** show that JHDM1A demethylates dimethyl-H3-K36 *in vivo.* 293T cells were transfected with wild-type (**Figure 5A, 5B, 5D** and **5E**) or mutant (**Figure 5C**) Flag^{®}-JHDM1A. Cells were co-stained with Flag^{®} antibody and different methylated H3-K36 antibodies as indicated in the figures. Staining with a dimethyl-K4 antibody was performed to serve as a control. Arrows in the middle **Figure 5B** point to the cells that express wild-type Flag^{®}-JHDM1A.

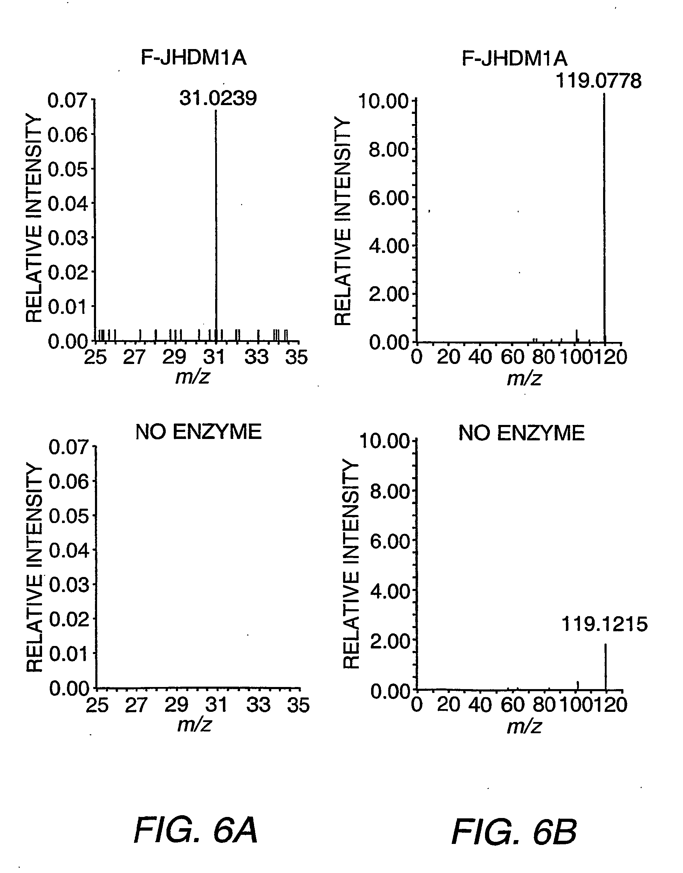

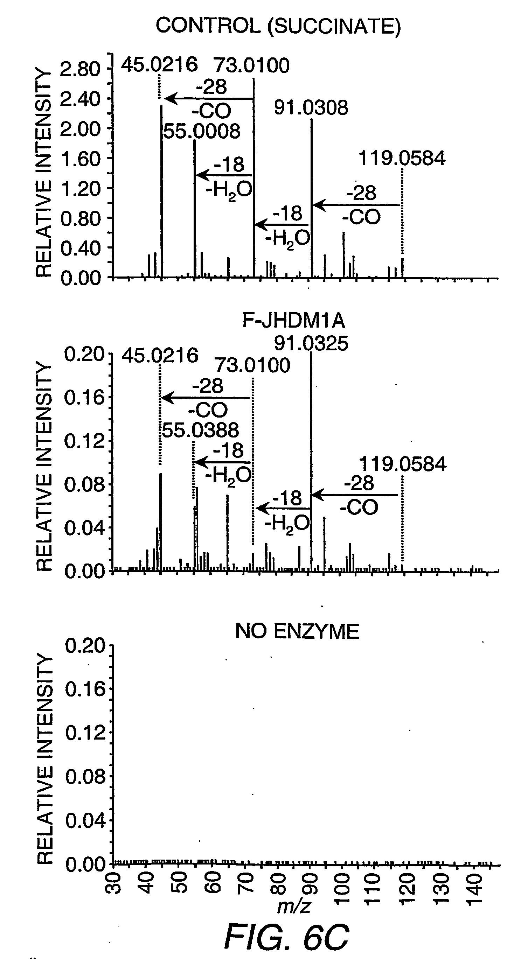

**Figures 6A-6C** show that JHDM1-mediated histone demethylation generates formaldehyde and succinate. ESI-MS detection of formaldehyde **(****Figure 6A****,** [M+H]⁺_{theoretical} = 31.0184) and succinate **(****Figure 6B****,** [M+H]⁺_{theoretical} = 119.0344) in the JHDM1 reaction samples (upper panels) and in the negative controls (lower panels), respectively, was demonstrated. **Figure 6C** shows ESI-MS/MS analysis of ions at m/z 119 from a standard solution of succinate (600 nM) (upper panel), the JHDM1 reaction sample (middle panel) and the negative control (lower panel). Suggested structures of the succinate fragment ions are shown on the MS/MS spectrum.

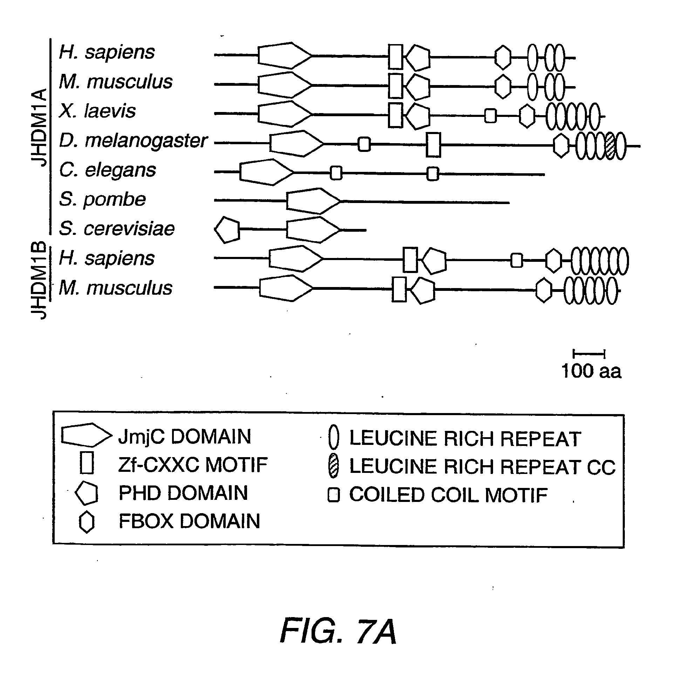

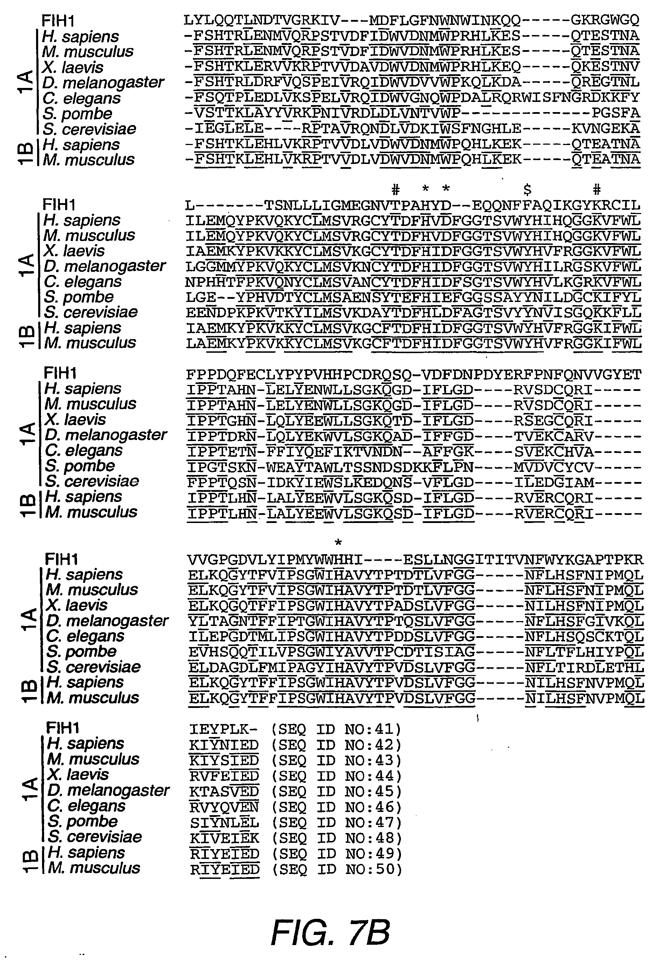

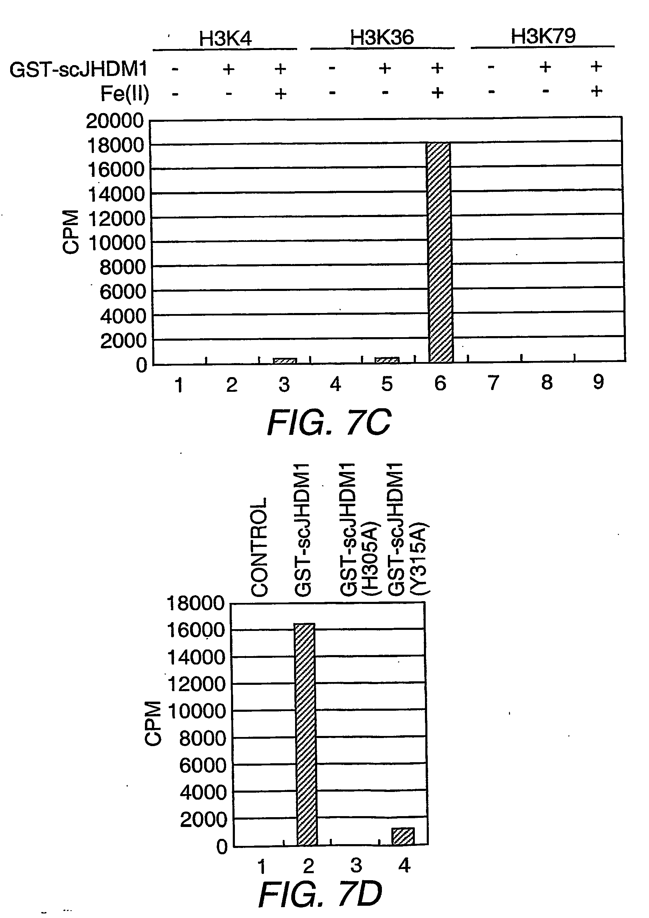

**Figures 7A-7D** show that the H3-K36 demethylase activity of the JHDM1 family proteins is conserved during evolution. **Figure 7A** is a diagrammatic representation of the JHDM1 family proteins in different organisms. Two highly related proteins were identified in human and mouse, but only one homolog was found in each of the other organisms. The various functional domains present in this family of proteins are shown based on analysis using the SMART program. **Figure 7B** is an alignment of the JmjC domain of the JHDM1 family members with that of FIH1 (Q9NWT6) using the PAPIA system. The NCBI accession numbers for the JHDM1A ("1A") proteins are as follows: NP_036440 (human), XP_355123 (mouse), AAH82636 (*Xenopus*), NP_649864 (*Drosophila*), AAN65291 (*C. elegans*), CAA21872 (*S. pombe*), NP_010971 (*S. cerevisiae*). The NCBI accession numbers for JHDM1 B ("1B") proteins are NP_115979 (human) and NP_001003953 (mouse). The numbers represent the amino acid numbers of each protein. The amino acids in FIH1 that are involved in Fe(II) and α-KG binding are indicated by "*" and "#", respectively. The conserved tyrosine that is critical for Epe1 function is indicated by a "$". Conservation of four out of seven sequences is underlined. **Figure 7C** shows demethylase activity and site specificity of the S. *cerevisiae* protein. **Figure 7D** shows mutational analysis of the JmjC domain of the scJHDM1 protein. Equal amounts of wild-type and mutant GST-scJHDM1 were used in the demethylase assays. A mutation in the Fe(II) binding site (H305A) completely abolished the H3-K36 demethylase activity, while a mutation (Y315A) that mimics the loss of function Epe1 (Y307A) mutation significantly reduced the H3-K36 demethylase activity.

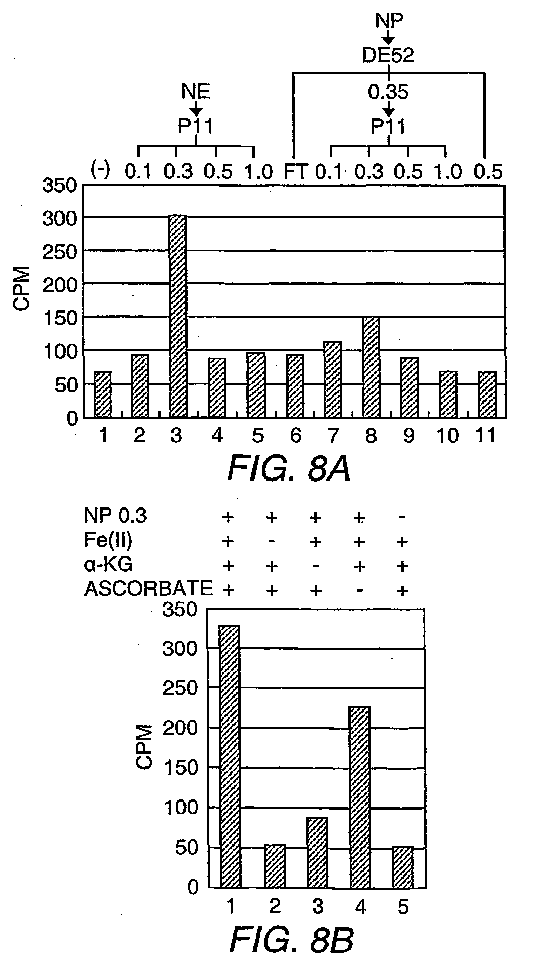

**Figures 8A-8B** show the identification of histone demethylase activity in HeLa cells using G9a-methylated histone substrates. **Figure 8A** depicts monitoring of histone demethylase activity from P11 column fractions derived from HeLa nuclear extract and nuclear pellet fractions against G9a-methylated histone substrates. The numbers above the panel represent the molar concentration of KCI in the elution buffers. **Figure 8B** shows that the 0.3M P11 demethylase activity depends on the presence of Fe(II) and α-KG.

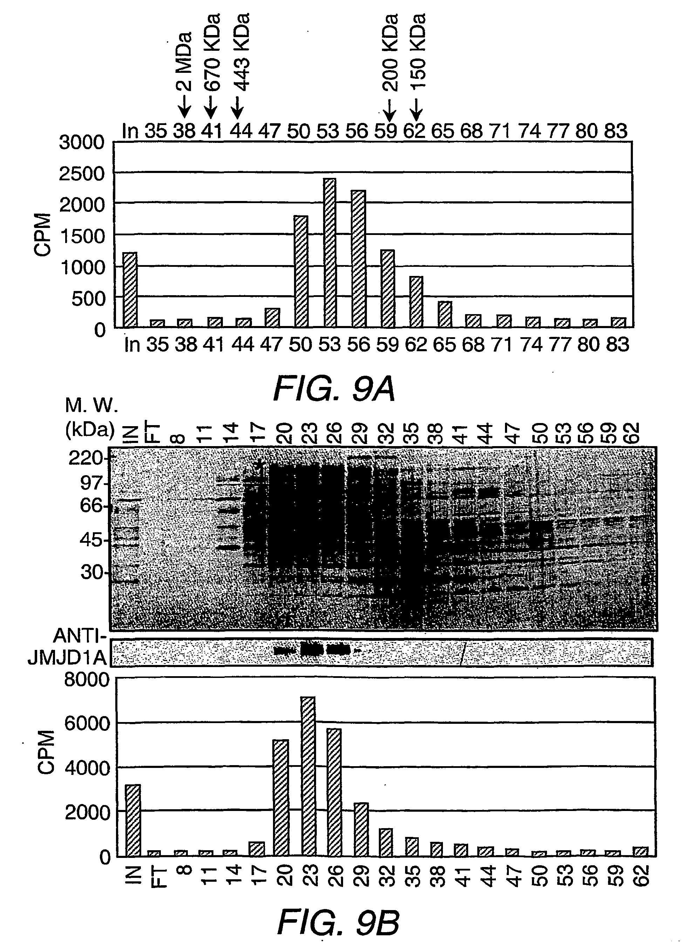

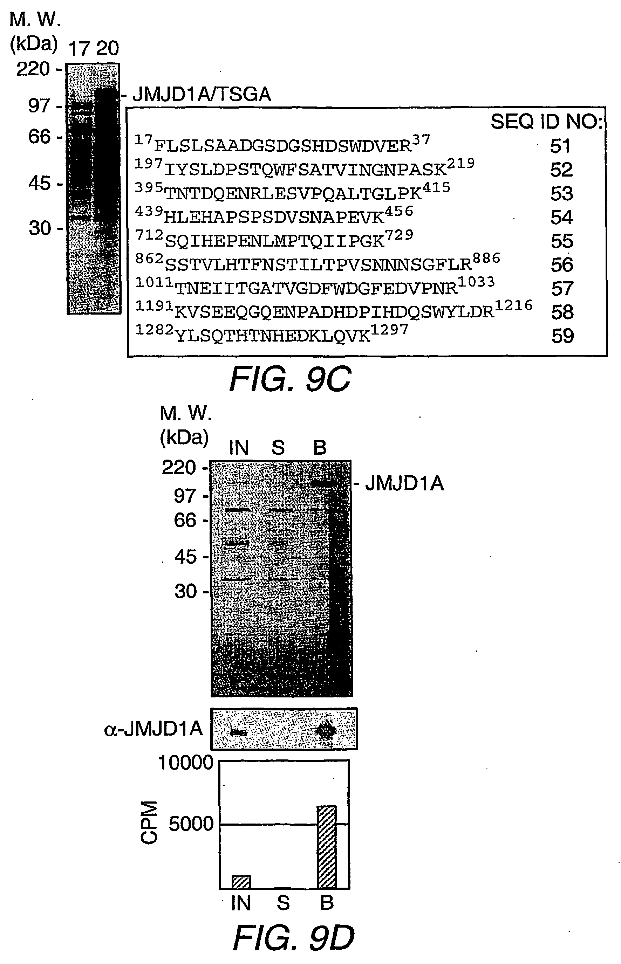

**Figures 9A-9D** show the purification and identification of histone demethylase activity. **Figure 9A** shows the histone demethylase activity of the protein fractions derived from a Sephacyl^{®} S300 gel-filtration column. The elution profile of the protein markers is indicated on top of the panel. **Figure 9B** is a silver-stained protein gel (top panel) and histone demethylase activities (bottom panel) of the protein fractions derived from a MonoS^{®} column. The candidate protein that co-fractionated with the demethylase activity is indicated by *. The positions of the protein size markers on SDS-PAGE are indicated to the left of the panel. **Figure 9C** is a silver-stained protein gel comparing the protein compositions of the histone demethylase positive fraction 20 with the adjacent histone demethylase negative fraction 17. The candidate protein was identified by mass spectrometry. A total of 63 peptides covering 53% of the JMJD1A protein (NCBI Accession No. NP_060903) were identified. Representative peptides identified from mass spectrometry are listed. The numbers correspond to the amino acid numbers of the JMJD1A protein. **Figure 9D** is silver staining (top panel), western blot (middle panel), and histone demethylase assay (bottom panel) analysis of the immunoprecipitated sample using a JMJD1A antibody. "In", "S" and "B" represent input, supernatant, and bound, respectively. The input sample was derived by pooling fractions 21-29 of the MonoS^{®} column.

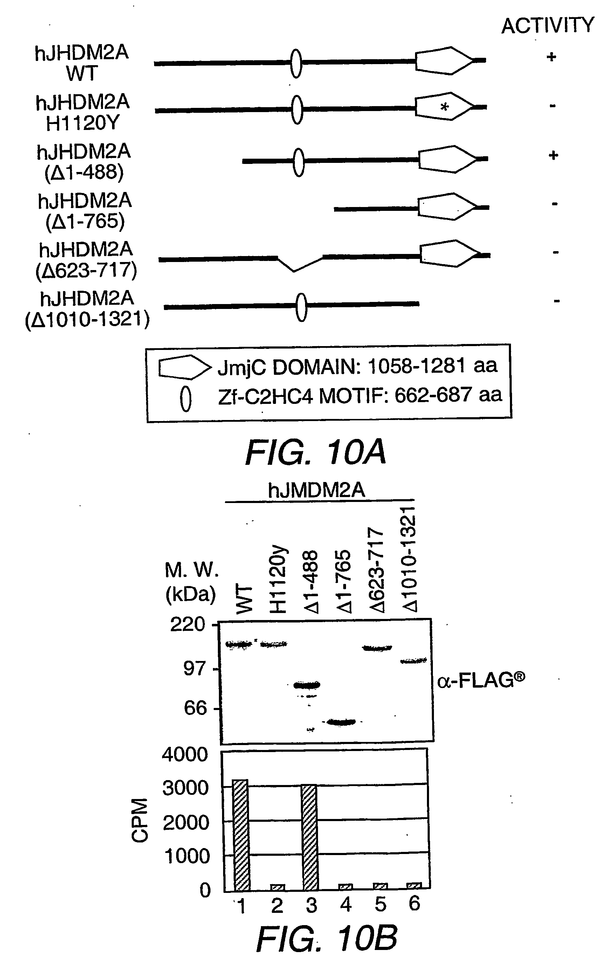

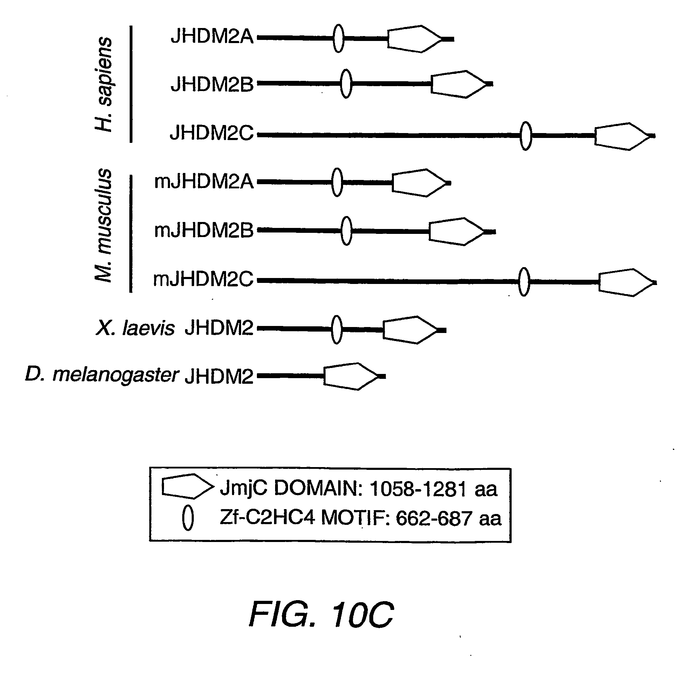

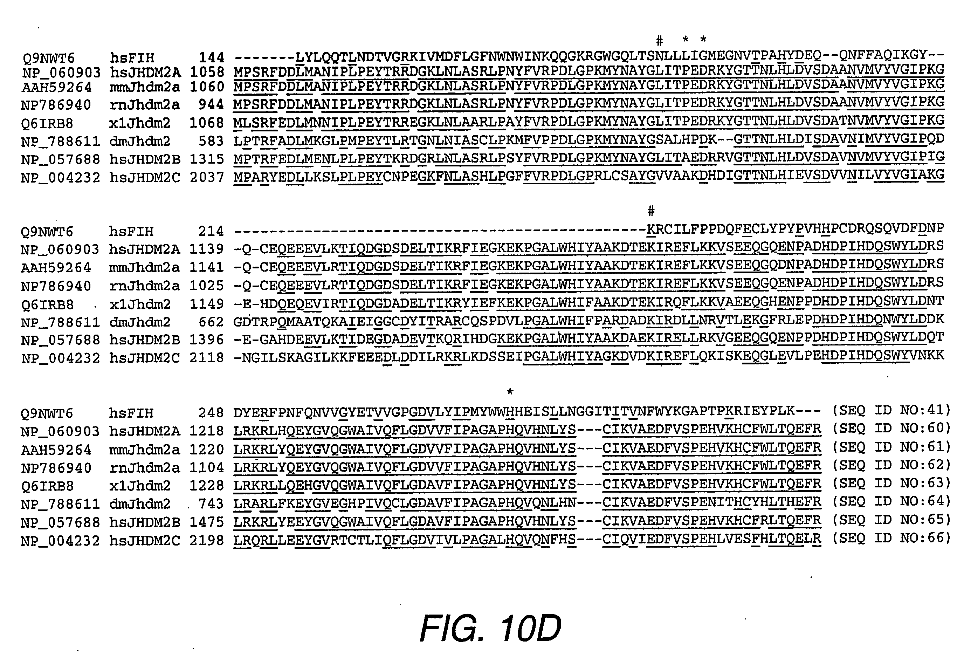

**Figures 10A-10D** shows that JmjC and Zinc-finger domains of JHDM2A are both required for histone demethylase activity. **Figure 10A** is a schematic representation of the wild-type and mutant JHDM2A proteins with their activities (right). "+" represents active and "-" represents inactive. **Figure 10B** shows western blot (top panel) and demethylase assay (bottom panel) analysis of wild-type and mutant Flag^{®}-JHDM1A proteins expressed in COS-7 cells and immunoprecipitated prior to analysis. **Figure 10C** is a diagrammatic representation of the JHDM2 family of proteins from different organisms. Three related proteins were identified in human and mouse, but only one homolog was found in *Drosophila* and *Xenopus.* The JmjC and Zinc-finger domains present in this family of proteins are shown based on analysis using the SMART program. **Figure 10D** is an alignment of the JmjC domain of the JHDM2 family members with that of FIH1 using the PAPIA system. The accession number for each of the proteins in the alignment is listed. The numbers represent the amino acid numbers of each protein. The amino acids in FIH1 that are involved in Fe(II) and α-KG binding are indicated by "*" and "#", respectively. Conserved sequences are underlined.

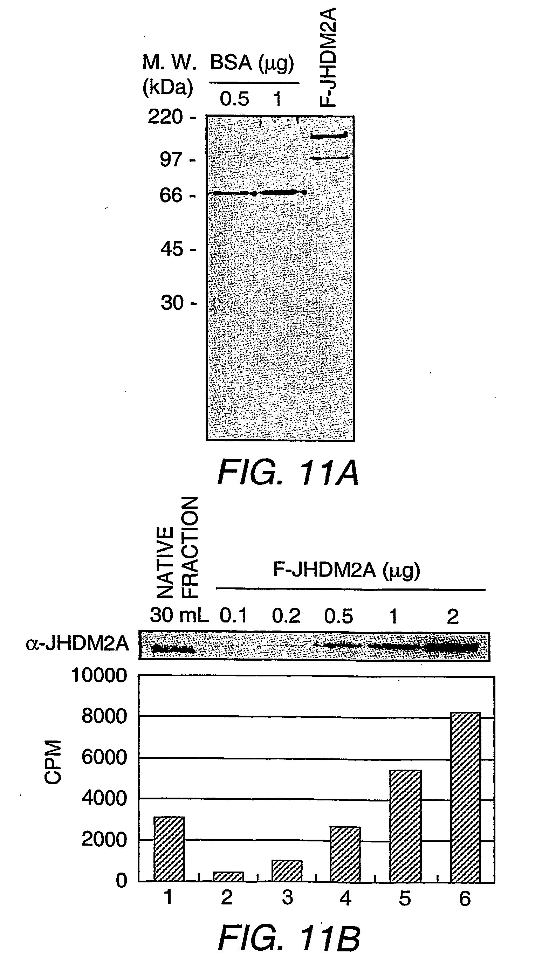

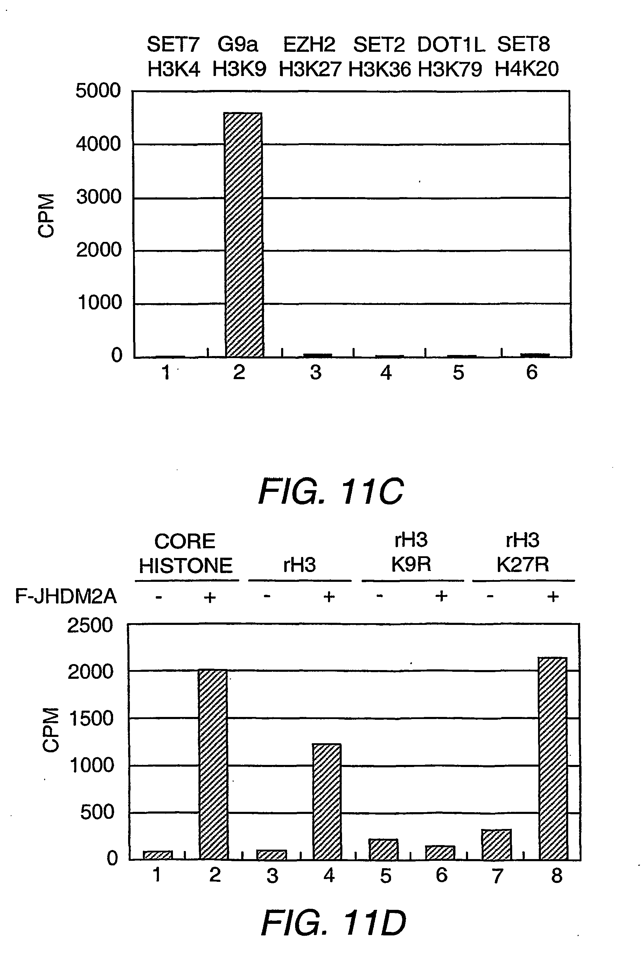

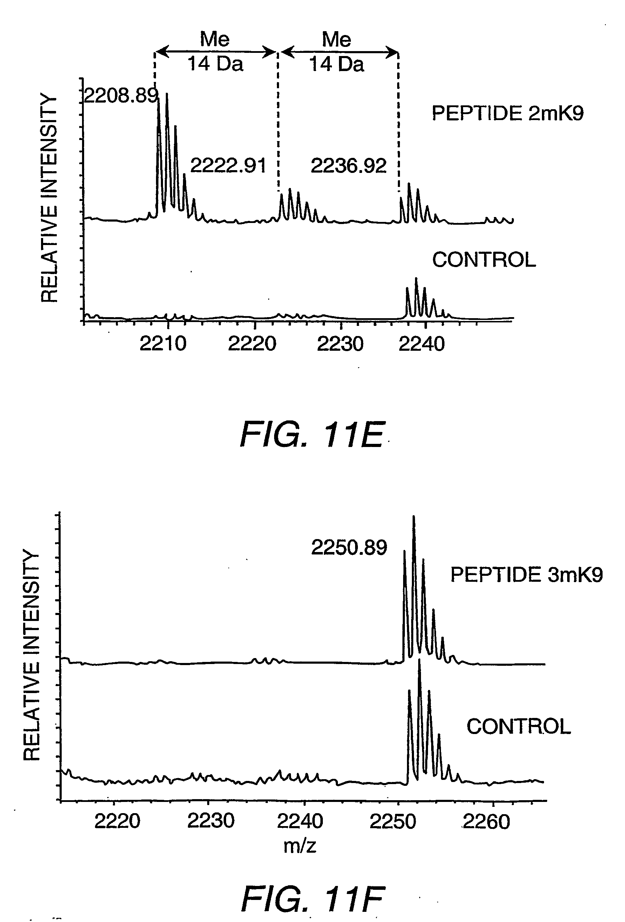

**Figures 11A-11** **F** show the characterization of the site and methylation state specificity of JHDM2A. **Figure 11A** is a Coomassie^{®}-stained protein gel of the purified Flag^{®}-JHDM2A protein expressed in Sf9 cells compared with BSA. The lower band in lane 3 is a degradation product verified by western blot analysis. **Figure 11B** shows the comparable histone demethylase activity of recombinant JHDM2A made in Sf9 cells and the native JHDM2A purified from HeLa cells. Fixed amounts of native JHDM2A were compared with varying amounts of recombinant JHDM2A, quantified by western blot analysis using JHDM2A antibody (top panel), in histone demethylase activities (bottom panel). **Figure 11C** shows the histone demethylase activity of Flag^{®}-JHDM2A toward equal counts of various methylated histone substrates input. Similar results were obtained when equal amounts of substrates in micrograms were used. The histone methyltransferases (HMTs) and their sites of methylation are indicated on top of the panel. **Figure 11D** shows that Flag^{®}-JHDM2A demethylates G9a-methylated H3-K9, but not H3-K27. Native histones purified from HeLa cells, and recombinant histone H3, wild-type or mutant (K9R, K27R), purified from *E. coli,* were methylated by G9a and subjected to demethylase assays in the presence (+) or absence (-) of Flag^{®}-JHDM2A. Mass spectrometry analysis of demethylation of a dimethyl-K9 (**Figure 11E**) or a trimethyl-K9 (**Figure 11F**) peptide (acetyl-ARTKQTARmeKSTGGKAPRK-biotin; **SEQ ID NO:77**) by Flag^{®}-JHDM2A was conducted. The enzyme/substrate molar ratio of the reaction was 1:40. Numbers represent the masses of the substrate and product peptides.

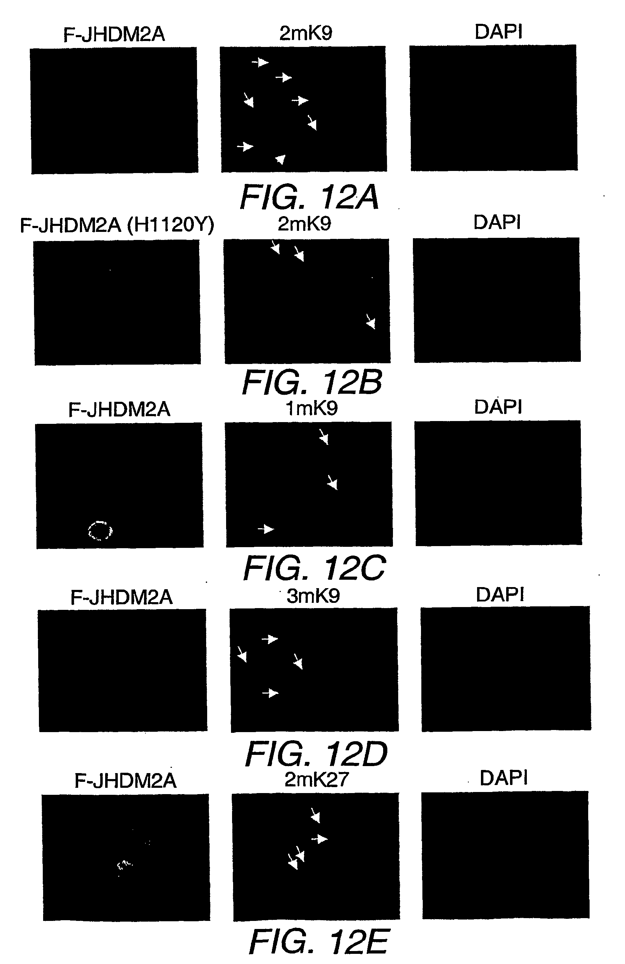

**Figures 12A-12E** show that JHDM2A demethylates mono-, and dimethyl-H3-K9 *in vivo.* COS7 cells were transfected with wild-type **(****Figures 12A, 12C, 12D, 12E****)** or mutant **(****Figure 12B****)** Flag^{®}-JHDM2A. Cells were co-stained with Flag^{®} antibody and different methyl-H3-K9 or dimethyl-H3-K27 antibodies were used as indicated in the figure. Arrows point to the transfected cells.

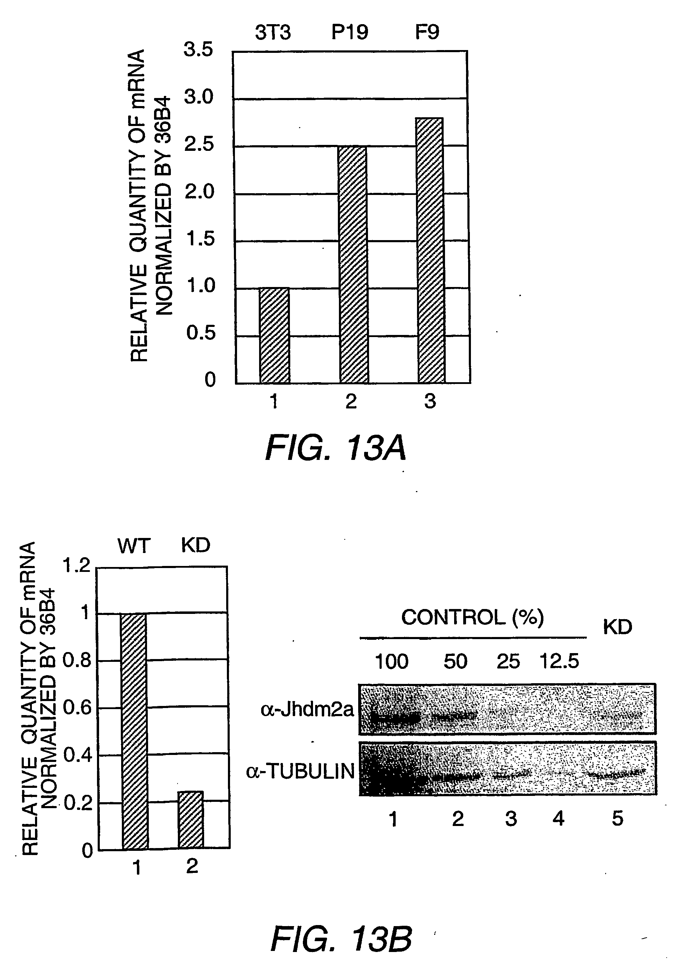

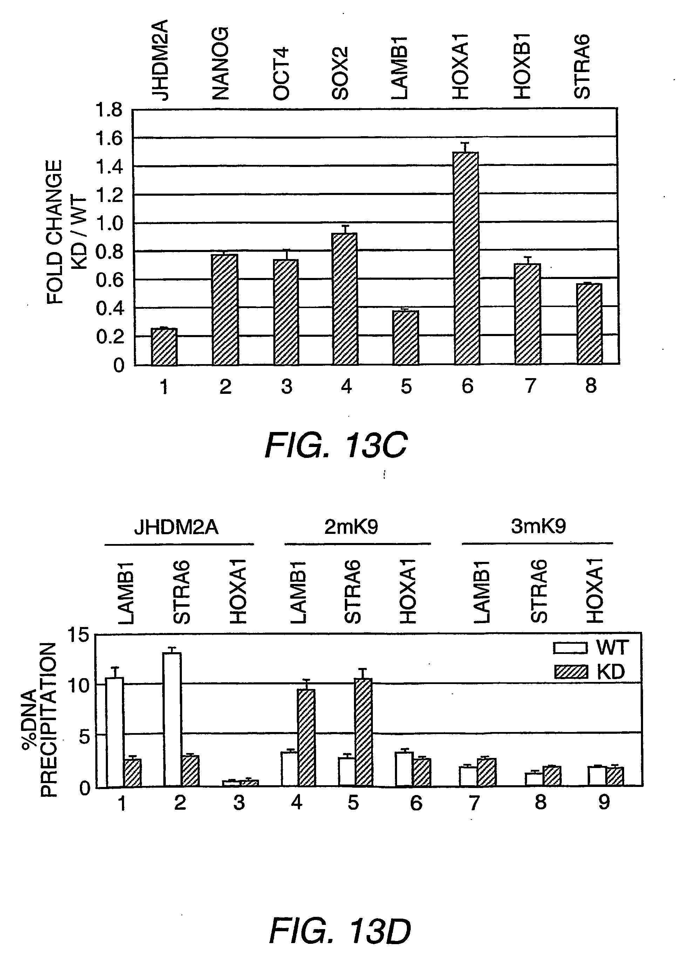

**Figure 13** shows that knockdown of *Jhdm2a* in F9 cells results in decreased transcription concomitant with increased promoter H3-K9 dimethylation. Relative expression level of *Jhdm2a* in NIH3T3, P19, and F9 cells was analyzed by quantitative RT-PCR **(****Figure 13A****).** Characterization of a *Jhdm2a* knockdown F9 cell line was also carried out **(****Figure 13B****).** Quantitative RT-PCR (left panel) and western blot analysis (right panel) demonstrated that about 80% knockdown on *Jhdm2a* at the RNA level and 70% knockdown at the protein level were achieved. **Figure 13C** shows the quantitative RT-PCR analysis of the changes at the RNA level of several pluripotency and differentiation mark genes in response to *Jhdm2a* knockdown. The changes are expressed as the ratio of the expression level in knockdown cells to that of the wild-type control. **Figure 13D** shows the Q-PCR results of ChlPed DNA in control (open bars) and *Jhdm2a* knockdown (filled bars) cells. The gene promoters analyzed, and the antibodies used are indicated. All Q-PCR have been repeated for three times. The average with standard deviation is presented.

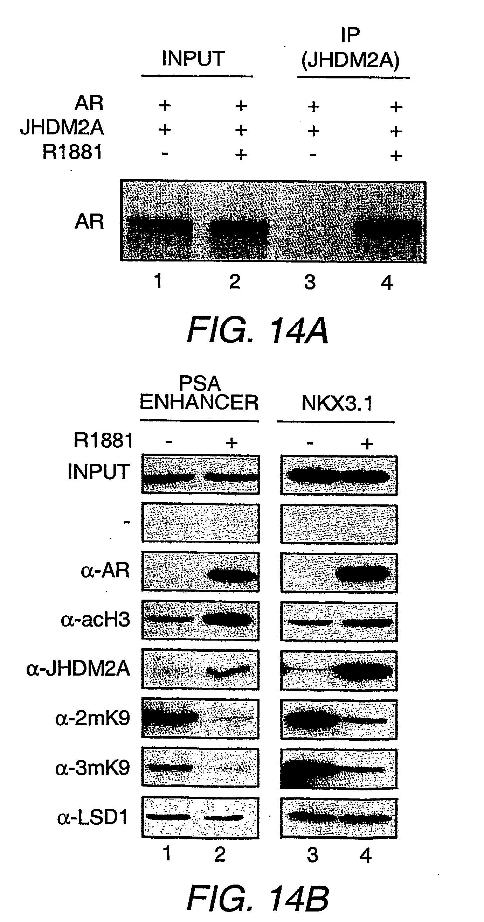

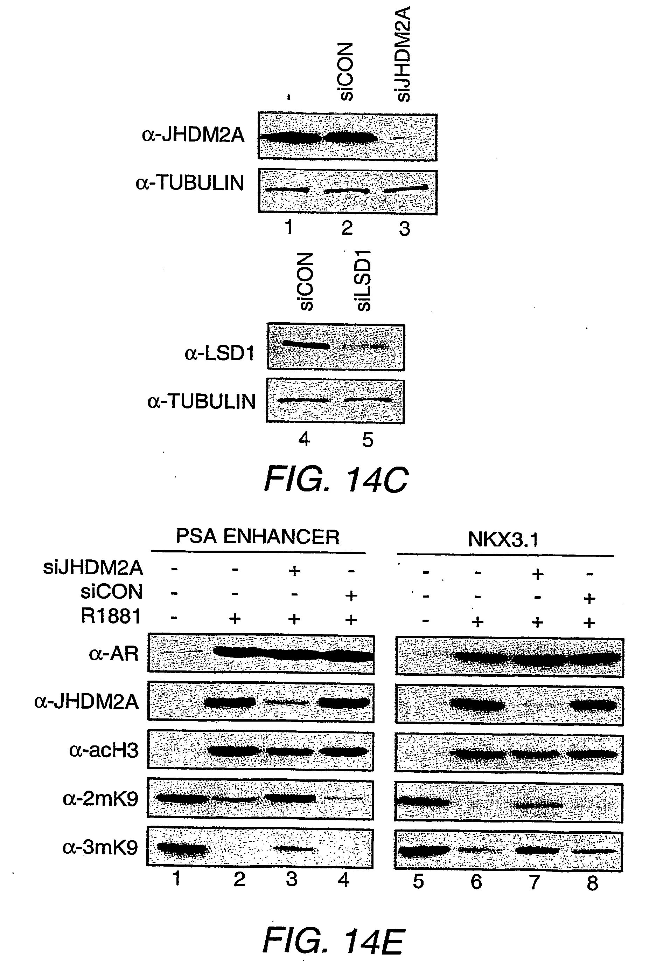

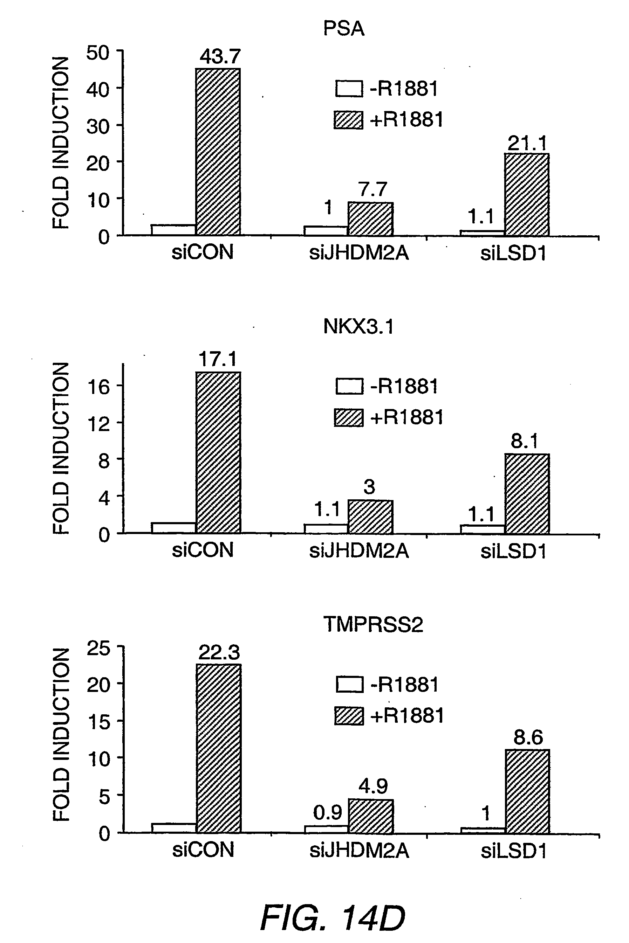

**Figure 14** demonstrates that JHDM2A is involved in transcriptional activation by AR. JHDM2A was found to interact with androgen receptor (AR) in a hormone-dependent manner *in vitro* (**Figure 14A**). Recombinant JMJD1A was mixed with *in vitro* translated ³⁵S-labeled AR in the absence or presence of R1881 (100 nM). After immunoprecipitation with anti-JHDM2A antibody, AR was detected by autoradiography. **Figure 14B** shows hormone-dependent recruitment of JHDM2A to PSA and NKX3.1 enhancers *in vivo.* LNCaP cells were cultured in charcoal-stripped serum medium for three days and then treated without or with R1881 (50 nM) for 1 hour before being processed for CHIP analysis. **Figure 14C** shows knockdown of JHDM2A and LSD1 in LNCaP cells by siRNA. LNCaP cells were transfected with a scramble (siCON), siJHDM2A or siLSD1 as indicated. Tubulin served as a loading control. **Figure 14D** shows quantitative RT-PCR analysis showing the effect of knocking down JHDM2A or LSD1 on R1881-dependent activation of three AR target genes. The cells were treated as in **Figure 14B** except the cells were collected for RNA isolation 8 hours after the R1881 treatment. **Figure 14E** shows that knocking down JHDM2A impaired hormone-induced H3K9 demethylation. The LNCaP cells were treated with siJHDM2A or siCON for three days and then 50 nM R1881 for 1 hour before processed for ChIP assays using antibodies as indicated.

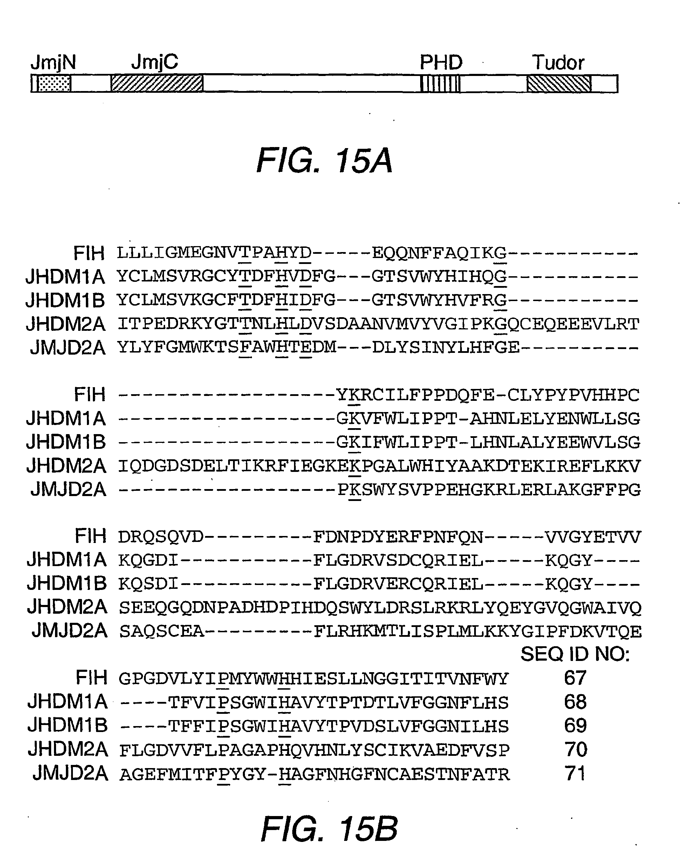

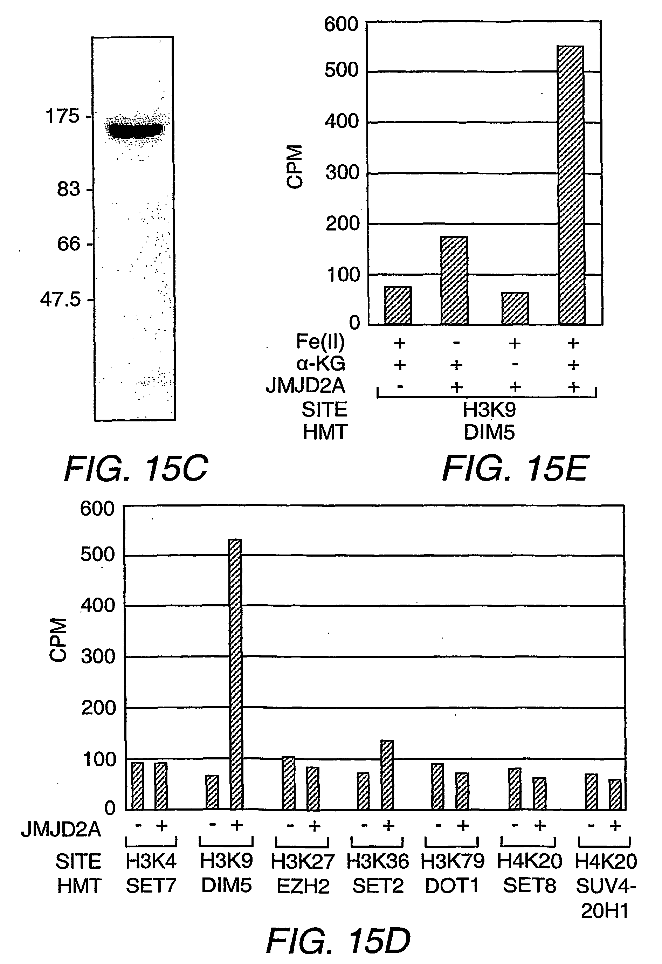

**Figures 15A-15E** demonstrate that JMJD2A is a histone H3K9 demethylase. As depicted in **Figure 15A****,** JMJD2A contains a JmjC domain and several other domains found in chromatin associated proteins. **Figure 15B** is an alignment detailing similarities in the JmjC domain of FIH, JHDM1A, JHDM1B, JHDM2A, and JMJD2A. The Fe(II) and (α-KG) binding domains are indicated by "*" and "#", respectively. Underlining indicates regions of conservation. **Figure 15C** is a Coomassie^{®}-stained gel containing recombinant JMJD2A purified from baculovirus-infected Sf9 cells. In **Figure 15D** histones were labeled using various histone methyltransferases as indicated below the bar graph and incubated with recombinant JMJD2A. The release of labeled methyl groups was measured to assay for histone demethylase activity, in the presence (+) or absence (-) of enzyme. JMJD2A specifically demethylates H3K9 labeled by the histone methyltransferase Dim5. To demonstrate that JMJD2A carries out demethylation using the an oxidative mechanism, the co-factors Fe(II) and α-KG were omitted from the reaction as indicated (-) below the bar graph shown in **Figure 15E****.** Full enzymatic activity of JMJD2A requires the complete complement of co-factors and enzyme.

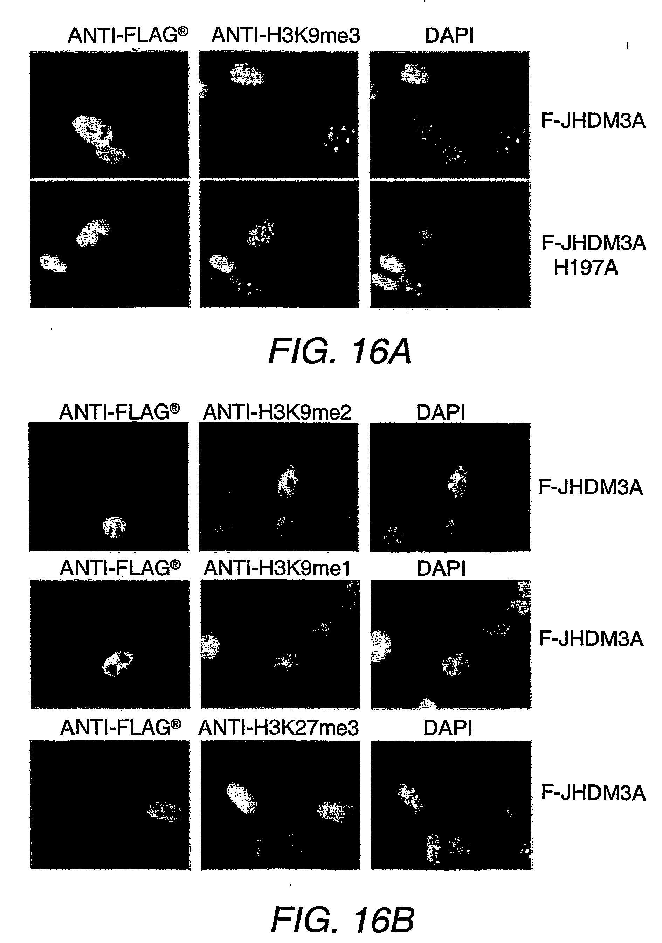

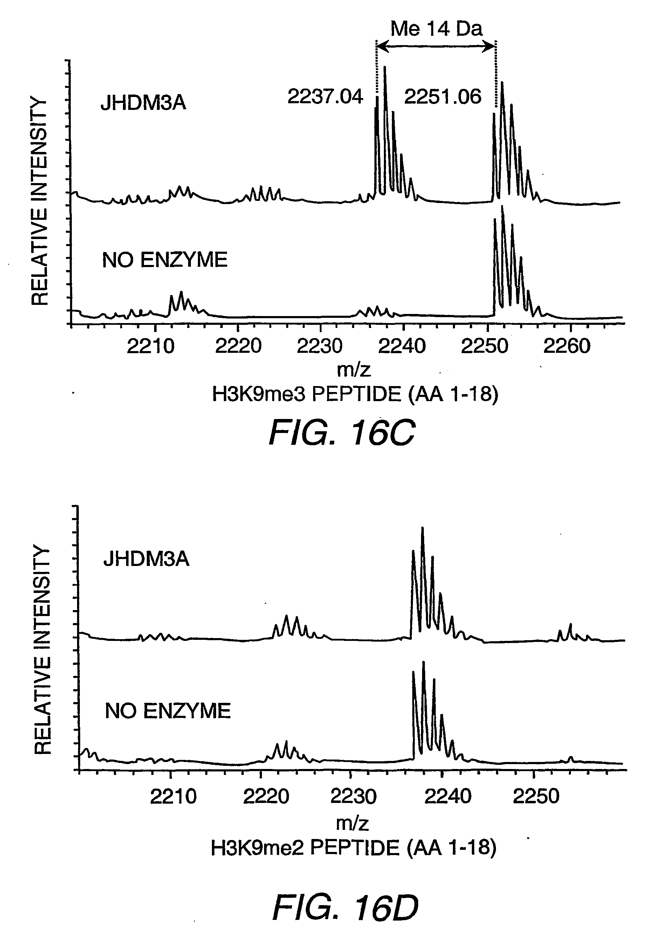

**Figures 16A-16D** show that JHDM3A is a trimethyl-specific histone demethylase. In **Figure 16A****,** JHDM3A was expressed in mouse 3T3 cells as a Flag^{®} fusion protein. Indirect immuno-fluoresce with antibodies against Flag^{®} (left panel) or trimethylated H3K9 (middle panel) were used to analyze the substrate specificity of JHMD3A *in vivo.* DAPI staining (right) indicates location of nuclei in each field. Cells transfected with JHDM3A showed a pronounced loss of trimethyl H3K9 staining (top panels), and this activity was dependent on an intact JmjC domain as staining was unaffected when a mutation in the predicted Fe(II) binding site was introduced into JHMD3A (bottom panel H197A). **Figure 16B** shows that the demethylation of trimethyl H3K9 by JHDM3A was specific, as antibodies against di-methyl H3K9, mono-methyl H3K9, or trimethyl H3K27 indicate no alterations in the level of these modifications in cells transfected with JHDM3A. A trimethyl K9 (**Figure 16C**) or a di-methyl K9 (**Figure 16D**) peptide was incubated in the presence (top) or absence (bottom) of JHDM3A before analysis by mass spectrometry. Incubation of JHDM3A with the dimethyl H3K9 peptide resulted in the removal of one methyl group (1 Da) producing a dimethyl-H3K9 peptide (**Figure 16C**) while incubation of JHDM3A with the di-methyl H3K9 resulted in no change in the peptide modification state (**Figure 16C**)**.**

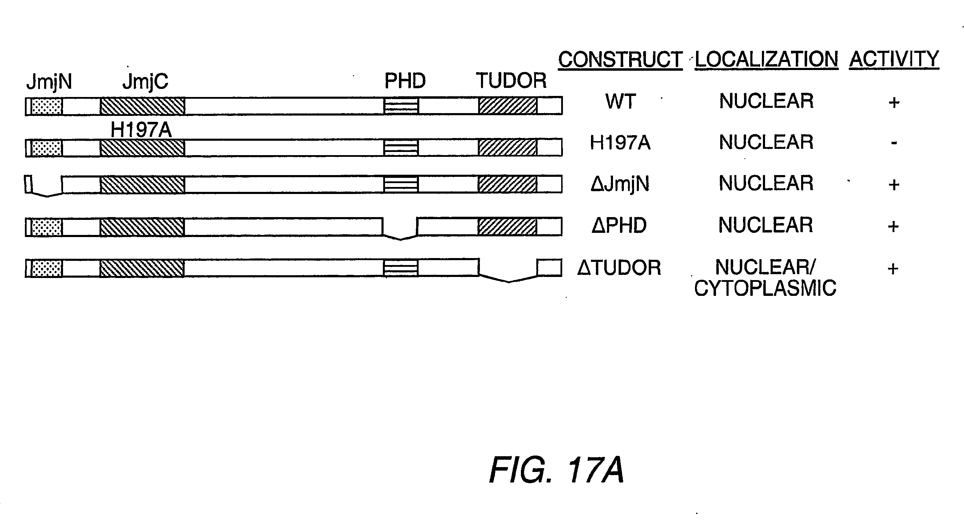

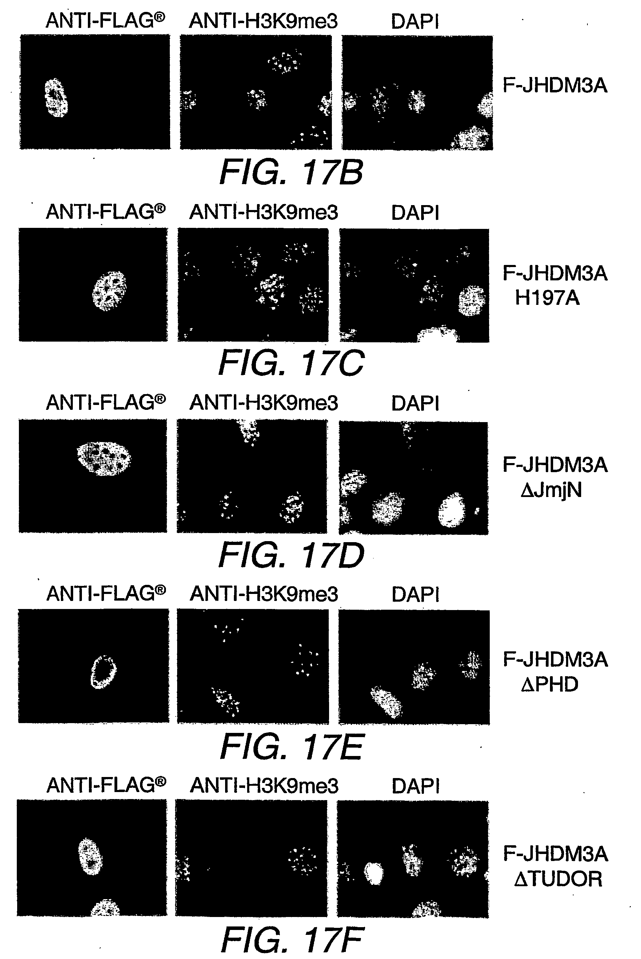

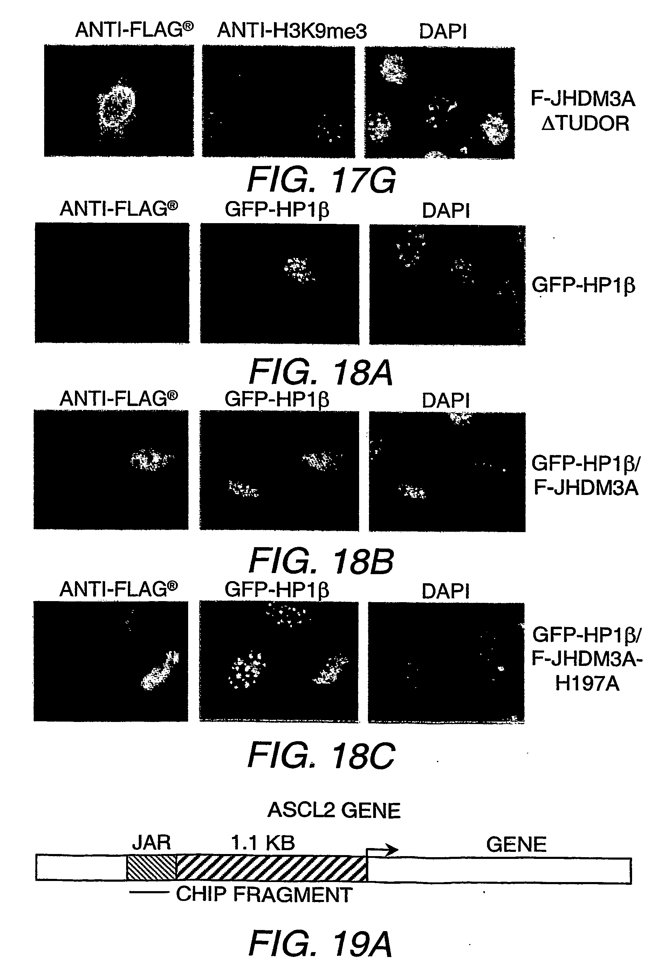

**Figures 17-17G** show that only the JmjC domain is required for trimethyl H3K9 demethylation *in vivo.* **Figure 17A** is a diagramatic representation indicating the deletion constructs used in the transfection assays. Subcellular localization and demethylase activity of the mutants are indicated. **Figures 17B-17G** show the effects of the deletion mutants on trimethyl H3K9 levels as determined by immunofluorescence. By analyzing trimethyl H3K9 staining in transfected cells (**Figures 17B-17G**), it was observed that only mutation of the proposed iron binding domain (H197A) abrogates loss of trimethyl H3K9 staining. Deletion of the TUDOR domain caused variable localization of JHDM3A in both the nucleus and cytoplasm, but still resulted in trimethyl H3K9 demethylation (**Figures 17F****,** **17G**).
**Figures 18A-18C** show that the expression of JHDM3A antagonizes HP1 recruitment. As shown in **Figure 18A****,** GFP-HP1 displays punctuate fluorescence corresponding to trimethyl H3K9 containing pericentric heterochromatin in mouse cells. **Figure 18B** demonstrates that the expression of JHDM3A causes trimethyl H3K9 demethylation resulting in a diffuse re-distribution of GFP-HP1 within the nucleus. **Figure 18C** shows that an intact JmjC domain is essential for antagonizing HP1 recruitment to pericentric heterochromatin as a mutation in the predicted Fe(II) binding domain abrogated the affect of JHDM3A on GFP-HP1 localization.

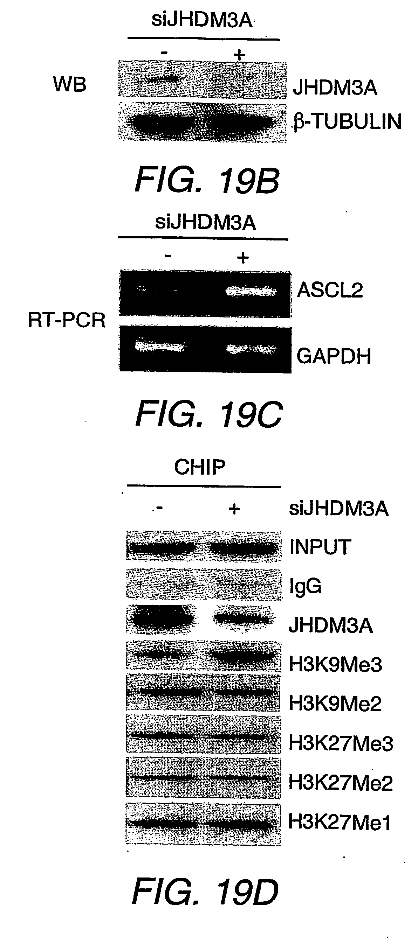

**Figures 19A-19D** demonstrate that JHDM3A demethylates trimethyl H3K9 at a euchromatic target gene. **Figure 19A** is a diagram of the human ASCL2 gene structure. JAR represents the JHDM3A associated region. **Figure 19B** shows that JHDM3A protein levels were efficiently reduced by treatment of cells with JHDM3A siRNA. **Figure 19C** shows that siRNA-mediated knockdown of JHDM3A results in increased expression of the ASCL2 gene. Untreated (-) and JHDM3A siRNA treated (+) cells were used in CHIP assays to analyze histone modifications at the ASCL2 gene (**Figure 19D**). Reduced levels of JHDM3A caused reduced occupancy of JHDM3A at the ASCL2 gene and increased levels of trimethyl H3K9 (H3K9me3). Other histone modifications at the ASCL2 gene remained unchanged after siRNA treatment verifying the specificity of the JHDM3A.

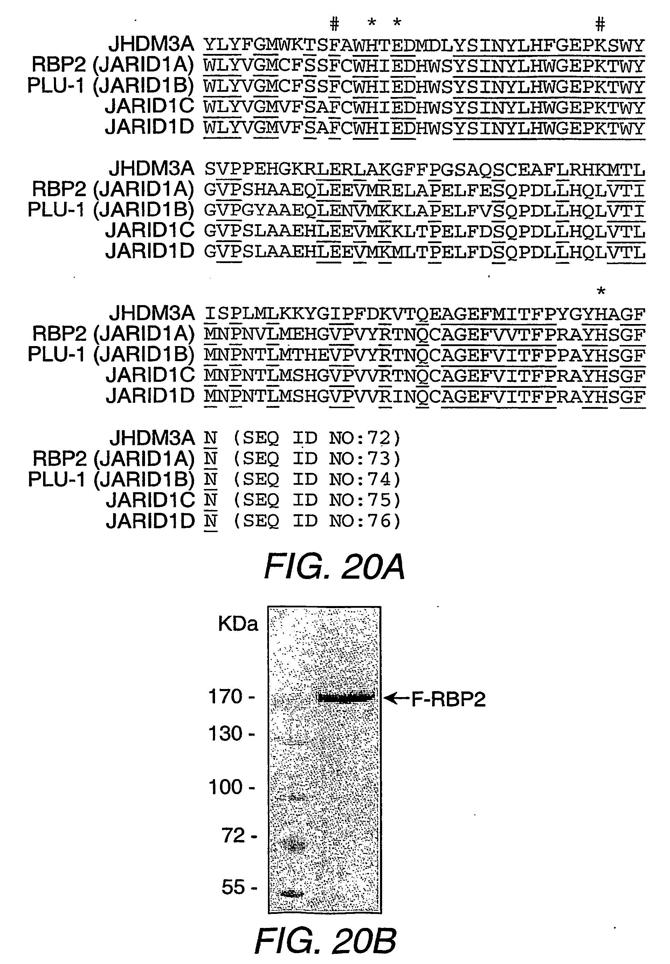

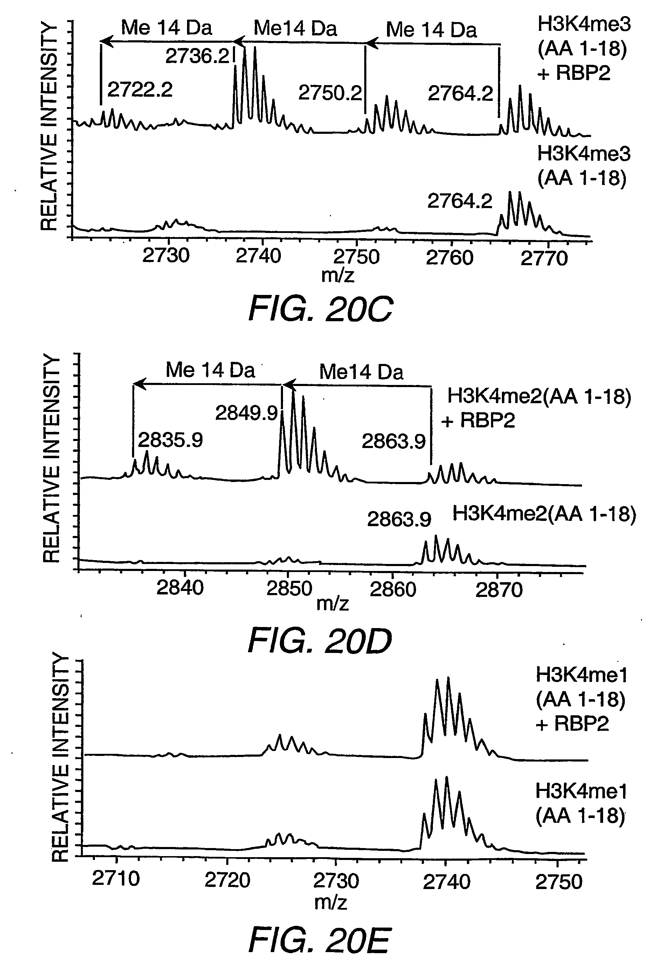

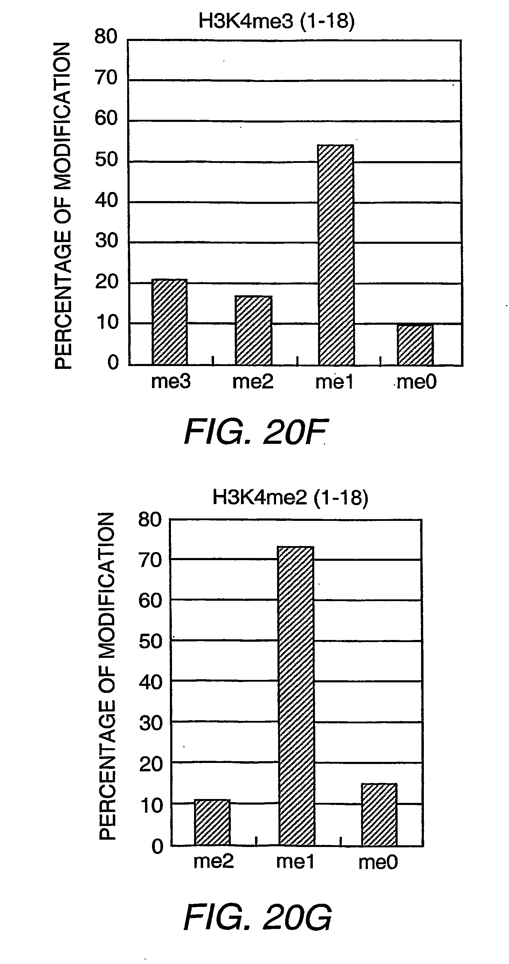

**Figures 20A-20G** show that RBP2 is an H3K4 demethylase with the capacity to remove H3K4me3. **Figure 20A** shows that the JmjC domain of the JARID1 subfamily is highly similar to the JmjC domain of JHDM3 demethylase enzymes. The predicted Fe(II) ("*") and α-KG binding ("#") residues are conserved in RBP2 and other JARID1 members. Recombinant Flag^{®}-RBP2 was expressed in insect cells, affinity purified by Flag^{®} chromatography, and analyzed by SDS-page **(****Figure 20B****).** **Figures 20C-20E** show mass spectrometry analysis of RBP2 activity toward H3K4me3 (**Figure 20C**), H3K4me2 (**Figure 20D**), and H3K4me1 (**Figure 20E**) peptides. Quantification of demethylation levels observed for RBP2 on H3K4me3 (**Figure 20F**) and H3K4me2 (**Figure 20G**) substrates.

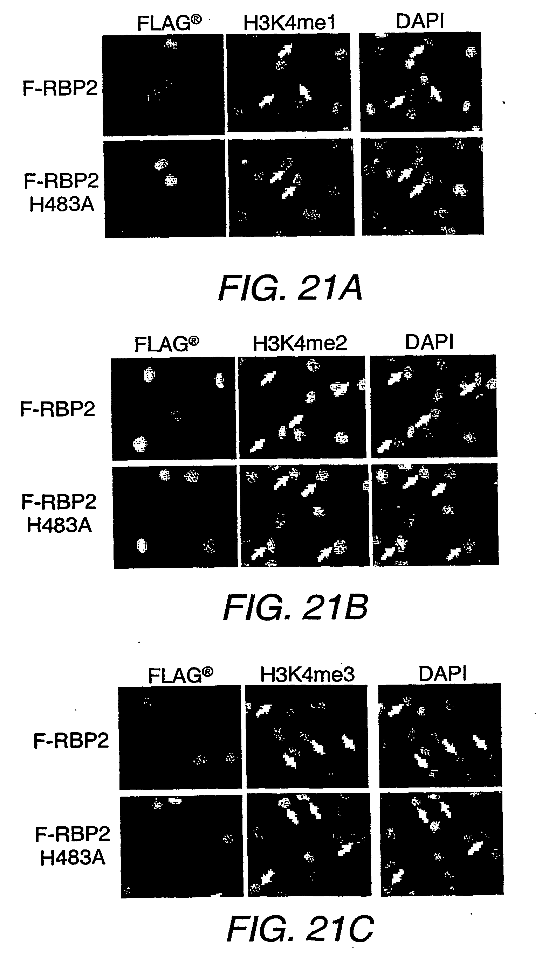

**Figures 21A-21C** show that RBP2 demethylates H3K4 *in vivo*. Flag^{®}-RBP2 or Flag^{®}-RBP2 containing a mutation in the proposed iron-binding site (H483A) were expressed in NIH3T3 cells **(****Figures 21A-21C****).** The levels of H3K4 methylation were analyzed using modification-specific antibodies against H3K4me1 (**Figure 21A**), H3K4me2 (**Figure 21B**), and H3K4me3 (**Figure 21C**) by indirect immunofluorescence (middle panels). Cells expressing wild-type and mutant RBP2 were identified by Flag^{®} immunofluorescence (left panels) and nuclei were identified by DAPI staining (right panels). Arrowheads in the middle and right panels indicate transfected cells. RBP2 demethylates all three H3K4 methylation states *in vivo.*

### Detailed Description of Embodiments of the Invention

The present invention is based, in part, on the discovery of a novel protein demethylase motif and assay for evaluating demethylation activity. Using this assay, the inventors have identified a histone demethylase activity from HeLa cells. It has further been demonstrated that a protein comprising a JmjC domain (Clissold and Ponting, (2001) Trends Biochem. Sci. 26:7-9), that has been named JHDM1, is responsible for the demethylase activity. In the presence of cofactors Fe(II) and α-ketoglutarate, JHDM1 demethylates histone H3 lysine 36 (H3-K36) and generates formaldehyde and succinate. The JmjC domain present in JHDM1 is responsible for the enzymatic activity as a mutation in this domain completely abolished its enzymatic activity. The function of the JmjC domain in histone demethylation is conserved as a *S*. *cerevisiae* homolog is also capable of demethylating H3-K36. Importantly, a mutation that mimics a loss of function mutation in the *S*. *pombe* JHDM1 homologue abolished the demethylase activity. Thus, the inventors have uncovered a novel histone demethylation mechanism and identified the JmjC domain as a signature motif for demethylases that is found in organisms from yeast to human.

In a parallel study using G9a-methylated histone substrates, the inventors have purified and characterized a second JmjC domain-containing histone demethylase, named JHDM2A, that demethylates H3-K9. The enzymatic activity of JHDM2A depends on an intact JmjC domain and requires cofactors Fe(II) and α-ketoglutarate.

Further, to identify additional histone demethylases, the inventors compared the JmjC domains of other JmjC family members to JHDM1A/B and JHDM2A, focusing on similarities in the proposed Fe(II) and α-KG binding sites. A related protein hydroxylase, FIH (factor-inhibiting hypoxia-inducible factor), was included in the alignment because the structure of FIH complexed with Fe(II) and α-KG is available and thus can serve as a reference point. The protein JMJD2A was identified as a tri-methylated H3-K9 and H3-K36 demethylase, which demethylates trimethyl H3-K9 and trimethyl H3-K36 to dimethyl H3-K9 and H3-K36, respectively. This protein has been redesignated as JHDM3A to reflect its enzymatic function and conform to the inventors' existing naming convention.

Further studies have demonstrated that Retinoblastoma Binding Protein-2 (RBP2/JARID1A), a member of the JARID1 JmjC family, as well as the *Drosophila* orthologue *Lid* (Little Imaginal Discs), are H3-K4 demethylases and can process mono-, di- or tri-methylated substrates to the unmethylated form. JARID1B (PLU1) is also a H3-K4 demethylase, with a similar substrate specificity to RBP2.

Subjects for which implementation of the present invention is applicable include, but are not limited to, avians and mammals, with mammals being preferred. The term "avian" as used herein includes, but is not limited to, chickens, ducks, geese, quail, turkeys and pheasants. In some embodiments, the subject is a human subject. Human subjects include subjects of both genders and at any stage of development (*i.e*., neonate, infant, juvenile, adolescent, adult). While some embodiments of the present invention are primarily concerned with implementation regarding human subjects, the invention can also be carried out on animal subjects, particularly mammalian subjects such as non-human primates, bovines, ovines, caprines, equines, felines, canines, lagomorphs, rats, mice, *etc*. The present invention can be carried out on animals for veterinary purposes, for drug screening and/or drug development purposes.

The present invention will now be described in more detail with reference to the accompanying drawings, in which embodiments of the invention are shown. This invention may, however, be embodied in different forms and should not be construed as limited to the embodiments set forth herein. Rather, these embodiments are provided so that this disclosure will be thorough and complete, and will fully convey the scope of the invention to those skilled in the art.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention. All publications, patent applications, patents, and other references mentioned herein are incorporated by reference in their entirety.

Except as otherwise indicated, standard methods known to those skilled in the art may be used for cloning genes, expressing proteins, amplifying and detecting nucleic acids, and the like. Such techniques are known to those skilled in the art. *See, e.g.,* Sambrook et al., Molecular Cloning" A Laboratory Manual 2nd Ed. (Cold Spring Harbor, NY, 1989); F. M. Ausubel et al. Current Protocols in Molecular Biology (Green Publishing Associates, Inc. and John Wiley & Sons, Inc., New York).

### Definitions.

As used in the description of the invention and the appended claims, the singular forms "a," "an" and "the" are intended to include the plural forms as well, unless the context clearly indicates otherwise.

As used herein, "and/or" refers to and encompasses any and all possible combinations of one or more of the associated listed items, as well as the lack of combinations when interpreted in the alternative ("or").

Moreover, the present invention also contemplates that in some embodiments of the invention, any feature or combination of features set forth herein can be excluded or omitted.

The term protein or histone "substrate" as used herein refers to a starting reagent in an enzymatic reaction that is acted upon to produce the reaction product(s). According to the present invention, the protein or histone substrate can be directly acted upon by the demethylase (typically by binding to the active site and undergoing a chemical reaction catalyzed by the enzyme) or can first be modified prior to being acted upon by the enzyme.

The terms "JHDM protein," "JHDM proteins" and "demethylase comprising a JmjC domain" (and similar terms) as used herein encompass any JmjC domain containing demethylase (including histone demethylases), which includes without limitation proteins in the JHDM1 family, the JHDM2 family, the JHDM3 [JMJD2] family, the PHF2/PHF8 family, the JARID family (*e.g*., the JARID1 subfamily including without limitation RBP2 [JARID1A], JARID1B [PLU1], JARID1C [SMCX] and JARID1D [SMCY] and the JARID2 subfamily), the UTX/UTY family, and the JmjC domain only family (including the MINA53/NO66 subfamily, the JMJD5 subfamily, the PLA2G4B subfamily, the FIH subfamily, the HSPBAP1 subfamily, the LOC339123 subfamily, the PTDSR subfamily and the JMJD4 subfamily) (*see*, *e.g*., Klose et al., (2006) Nature Reviews/Genetics 7:715-727) and further includes variants and functional fragments of any of the foregoing that retain substantial demethylase activity (*e.g*., at least about 60%, 75%, 80%, 85%, 90%, 95% or more demethylase activity as compared with the native protein).

The demethylase proteins of the invention can be derived from any species of interest, including without limitation, mammalian (*e.g*., human, non-human primate, mouse, rat, lagomorph, bovine, ovine, caprine, porcine, equine, feline, canine, *etc*.), insect (*e.g.,* Drosophila), avian, fungal, plant, yeast (*e.g*., *S. pombe* or *S*. *cerevisiae*), *C. elegans, D. rerio* (zebrafish), *etc.* as well as allelic variations, isoforms, splice variants and the like. The demethylase sequences can further be wholly or partially synthetic.

In particular embodiments, a functional fragment or variant of a JMHD protein comprises a JmjC domain and, optionally, further comprises a JmjN domain, a Tudor domain, a zinc finger domain (*e.g*., a Zf-CXXC motif and/or a Zf-C2HC4 motif), zinc finger-like domain, a PHD domain, an FBOX domain, a tetratricopeptide repeat (TPR), an AT-Rich Interactive Domain (Arid/Bright), a coiled coil motif and/or a Leucine Rich Repeat (LRR) domain. The JmjC domain can comprise amino acid residues that coordinate with Fe(II) and/or α-ketoglutarate, which amino acids can be naturally occurring in JmjC domains or can be variants thereof (*see*, *e.g.,* the Examples and Klose et al., (2006) Nature Reviews/Genetics 7:715-727).

For example, a functional fragment or variant of a JHDM1 protein can comprise a JmjC domain and additionally a LRR, FBOX domain, PHD domain and/or zinc-finger domain.

In representative embodiments, a functional fragment or variant of a PHF2/PHF8 family protein comprises a JmjC domain and a PHD domain.

In other representative embodiments, a functional fragment or variant of a JARID family protein comprises a JmjC domain and additionally a PHD domain, a JmjN domain, an AT-rich interactive domain, and/or a zinc finger domain.

In representative embodiments, a functional fragment or variant of a JHDM3 family protein comprises a JmjC domain and additionally a JmjN domain, a PHD domain and/or a zinc finger domain. Optionally, the functional fragment or variant further comprises a Tudor domain.

In representative embodiments, a functional fragment or variant of a UTX/UTY family protein comprises a JmjC domain and a TPR domain.

In representative embodiments, a functional fragment or variant of a JHDM2 family protein comprises a JmjC domain and a zinc finger like domain.

As used herein a "JHDM1 protein" includes the human JHDM1A and JHDM1B proteins (*see*, *e.g*., the Examples and the protein and nucleic acid sequences of JHDM1A and JHDM1B found at NCBI Accession Nos. NM_012308, BC047371, BC064360, AY409191, NP_036440, AAH47486, AAH47371, AAH64360, NP_115979, NP_001005366, NM_032590, NM_001005366, and BC008735), as well as homologues thereof including but not limited to homologues from mammals (*e.g*., rat, mouse), *Xenopus, D. rerio, C. elegans, S. pombe* and *S*. *cerevisiae* (*see*, *e*.*g*., NCBI Accession Nos. AAH82636, NP_649864, AAN65291, CAA21872, NP_010971, NM_001001984, NM_001011176, XM_341983, BC076576, AY409193, NP_001001984, AAH57051, NP_001005866, NP_001003953, NP-998332, NP_038938, AAH57622, AAH82040, AAH65090, NM_013910, NM_001005866, NM_001003953, NM_213167, XM_222177, BC057622, BC082040, and BC065090) and further including variants and functional fragments of the foregoing that retain substantial demethylase activity (*e.g*., at least about 60%, 75%, 80%, 85%, 90%, 95% or more demethylase activity as compared with the native protein). For a further description and listing of JHDM1 proteins, see Klose et al. (2006) Nature Reviews/Genetics 7:715-727.

As used herein, a "JHDM2 protein" includes the human JHDM2A, JHDM2B and JHDM2C proteins (*see*, *e.g*., the Examples and the protein and nucleic acid sequences of JHDM2A, JHDM2B and JHDM2C found at NCBI Accession Nos. NP_060903, NP_057688, NP-004232, NM_018433, NM_016604 and NM_004241), as well as homologues thereof including but not limited to homologues from mammals (*e.g*., rat, mouse), *Xenopus,* and *Drosophila melanogaster* (*see, e.g*., NCBI Accession Nos. NP_786940, AAH59264, AAH70558, NP_788611, BC070558, NM_175764, BC059264 and NM_176434) and further including variants and functional fragments of the foregoing that retain substantial demethylase activity (*e.g*., at least about 60%, 75%, 80%, 85%, 90%, 95% or more demethylase activity as compared with the native protein). For a further description and listing of JHDM2 proteins, see Klose et al. (2006) Nature Reviews/Genetics 7:715-727.

As used herein, a "JHDM3 protein" includes the human JHDM3A, JHDM3B, JHDM3C and JHDM3D proteins (*see*, *e.g*., the protein and nucleic acid sequences of the human JHDM3A/JMJD2A, JHDM3B/JMJD2B, JHDM3C/JMJD2C and JHDM3D/JMJD2D found at NCBI Accession Nos. NP_055478, NM_014663, NP_055830, NM_015015, AAl04862, BC104861, NP_060509 and NM_018039), as well as homologues thereof including but not limited to homologues from mammals, *C*. *elegans* and *S*. *cerevisiae,* and further including variants and functional fragments of the foregoing that retain substantial demethylase activity (*e.g*., at least about 60%, 75%, 80%, 85%, 90%, 95% or more demethylase activity as compared with the native protein). Homologues in other organisms can be identified by routine techniques, *e.g*., by a blast search in the NCBI database. For a further description and listing of JHDM3 proteins, *see* Klose et al. (2006) Nature Reviews/Genetics 7:715-727.

As used herein, a "JARID protein" includes proteins in the JARID1 subfamily (e.g., RBP2 [JARID1A], JARID1B [PLU1], JARID1C [SMCX] and JARID1D [SMCY] proteins) and the JARID2 subfamily (*see, e.g.,* the protein and nucleic acid sequences of the human RBP2, JARID1B, JARID1C, JARID1D and JARID2 proteins found at NCBI Accession Nos. NM_001042603, NM_005056, NM_006618, BC054499, NM_004653 and BC046246), as well as homologues thereof including but not limited to homologues from mammals (*e.g.,* dog, mouse), *Drosophila melanogaster* (for example, *Lid*), *S*. *pombe, S*. *cerevisiae, C. elegans* (*see, e.g.,* NCBI Accession Nos. NM_078762, NM164671, NM_001031029, NM_001048032, NM_013668, and NM_011419), and further including variants and functional fragments of the foregoing that retain substantial demethylase activity (*e.g*., at least about 60%, 75%, 80%, 85%, 90%, 95% or more demethylase activity as compared with the native protein). Homologues in other organisms can be identified by routine techniques, *e.g*., by a blast search in the NCBI database. For a further description and listing of JARID proteins, *see* Klose et al. (2006) Nature Reviews/Genetics 7:715-727.

As used herein a "Hairless protein" includes the human proteins (*see, e.g.,* NCBI Accession Nos. CAB86602, CAB87577, NP_060881, and AAH67128) as well as homologues thereof including but not limited to homologues from mammals (*e.g*., rat, mouse, pig, sheep) and *Drosophila* (*see, e.g.,* NCBI Accession Nos. NP_077340, AAN05753, AAP33389, CAB38221 and CAA47664) and further including variants and functional fragments of the foregoing that retain substantial activity (*e.g*., at least about 60%, 75%, 80%, 85%, 90%, 95% or more demethylase activity as compared with the native protein).

For a further description of other JmjC proteins, see Klose et al. (2006) Nature Reviews/Genetics 7:715-727.

A "demethylase" or "protein demethylase" for use in the practice of the present invention comprises a JmjC domain, and can be a methyl-lysine or methyl-arginine demethylase. In particular embodiments, the demethylase is a histone demethylase, *e.g*., a histone H3 or H4 demethylase. For example, the H3 demethylase can demethylate H3-K4, H3-K9, H3-K27, H3-K36 and/or H3-K79. As another alternative, the demethylase can demethylate histone H4-K20. The demethylase can demethylate mono-, di- and/or tri-methylated substrates. Further, histone demethylases can act on a methylated core histone substrate, mononucleosome substrate, dinucleosome substrate and/or oligonucleosome substrate, peptide substrate and/or chromatin (*e.g*., in a cell-based assay).

As used herein, the term "modulate," "modulates" or "modulation" or grammatical variations thereof refers to enhancement (*e.g*., an increase) or inhibition (*e.g*., a reduction) in the specified activity.

The term "enhancement," "enhance," "enhances," or "enhancing" or grammatical variations thereof refers to an increase in the specified activity (*e.g*., at least about a 1.1-fold, 1.25-fold, 1.5-fold, 2-fold, 3-fold, 4-fold, 5-fold, 6-fold, 8-fold, 10-fold, 12-fold, or even 15-fold or more increase).

The terms "inhibition," "inhibit", "inhibits" or "reduction," "reduce," "reduces" or grammatical variations thereof as used herein refer to a decrease or diminishment in the specified activity of at least about 10%, 25%, 35%, 40%, 50%, 60%, 75%, 80%, 90%, 95% or more. In particular embodiments, the inhibition or reduction results in no or essentially no (*i.e*., an insignificant amount, *e.g*., less than about 10% or even 5%) detectible activity.

By the terms "treat," "treating" or "treatment of" (or grammatically equivalent terms), it is meant that the severity of the subject's condition is reduced or at least partially improved or ameliorated and/or that some alleviation, mitigation or decrease in at least one clinical symptom is achieved and/or there is a delay in the progression of the condition and/or prevention or delay of the onset of a disease or illness. Thus, the terms "treat," "treating" or "treatment of" (or grammatically equivalent terms) refer to both prophylactic and therapeutic regimens.

### Protein Demethylation Assays.

The inventors have discovered a new demethylation mechanism and developed a novel demethylase assay based on detection of reaction products (*e.g*., formaldehyde and/or succinate). In particular embodiments, the invention provides a method of detecting demethylase activity in a composition, the method comprising:
(a) contacting the composition with (i) a methylated protein substrate, (ii) Fe(II) and (III) α-Ketoglutarate under conditions sufficient for demethylation of the methylated protein substrate; and (b) detecting the release of formaldehyde and/or succinic acid from the demethylation reaction; wherein the release of formaldehyde and/or succinic acid is an indicator of demethylase activity. The method can be practiced for any purpose, including without limitation as an assay for demethylase activity of a composition known to contain a demethylase or to determine if the composition contains a demethylase.

Any suitable sample that contains or is suspected of containing a demethylase can be evaluated. For example, the sample can be a protein fraction, a cellular extract, and/or a protein fraction derived from a cellular extract. Cellular extracts can further be derived from particular subcellular compartments including but not limited to a nuclear extract, lysosomal extract, chloroplast extract, endosome extract and/or cytosol extract.

The invention can also be practiced to identify and/or to isolate a demethylase in a composition, the method comprising: (a) contacting the composition with (i) a methylated protein substrate, (ii) Fe(II) and (iii) α-ketoglutarate under conditions sufficient for demethylation of the methylated protein substrate; (b) detecting the release of formaldehyde and/or succinic acid from the demethylation reaction; wherein the release of formaldehyde and/or succinic acid is an indicator of demethylase activity; and optionally (c) one or more protein purification steps which may be accompanied by one or more iterations of steps (a) and (b) above to track the demethylase activity during the purification process. Further, the purified protein can optionally be partially or completely sequenced.

As another aspect, the invention provides a method of detecting demethylase activity, the method comprising: (a) contacting a protein with (i) a methylated protein substrate, (ii) Fe(II), and (iii) α-ketoglutarate under conditions sufficient for demethylation of the methylated protein substrate; and (b) detecting the release of formaldehyde and/or succinic acid from the demethylation reaction; wherein the release of formaldehyde and/or succinic acid is an indicator of demethylase activity. The method can be practiced for any purpose, including without limitation as an assay for demethylase activity of a known protein demethylase or to determine if a protein is a demethylase.

In particular embodiments of the methods described above, the reaction mixture further comprises ascorbate.

The method can be practiced as a cell-based or cell-free assay. Further, the invention can be practiced to determine the presence of, to identify and/or to isolate any demethylase. In particular embodiments, the demethylase is a methyl-lysine demethylase and/or a methyl-arginine demethylase. According to particular aspects of the invention, the demethylase is a histone demethylase, and can further be a histone H3 demethylase (e.g., H3-K4, H3-K9, H3-K27, H3-K36 and/or H3-K79 demethylase) and/or a histone H4 demethylase (e.g., H4-K20 demethylase). Suitable methylated histone substrates include but are not limited to a methylated core histone substrate, mononucleosome substrate, dinucleosome substrate and/or oligonucleosome substrate, peptide substrate and/or chromatin (*e.g*., in a cell-based assay).

The methylated protein or histone substrate can further comprise one or more methyl groups at a single lysine or arginine residue (*e.g*., mono-, di- and/or tri-methylated substrates).

In particular embodiments, a methylated histone substrate can comprise a methylated H3-K36 substrate, which may further be a mono-, di- and/or tri-methylated H3-K36 substrate.

In other representative embodiments, a methylated histone substrate can comprise a methylated H3-K9 substrate; which may further be a mono-, di- and/or tri-methylated H3-K9 substrate.

In other representative embodiments, a methylated histone substrate can comprise a methylated H3-K4 substrate, which may further be a mono-, di- and/or tri-methylated H3-K4 substrate.

According to this aspect of the present invention, demethylation can be evaluated by detecting the release of reaction product, for example, formaldehyde and/or succinate, either directly or indirectly. In particular embodiments, detecting the release of formaldehyde comprises converting the formaldehyde to 3,5-diacethyl-1,4-dihydrolutidine (DDL) and detecting the DDL, for example, by detecting radiolabeled DDL (*e.g*., ³H-DDL). To illustrate, the starting substrate can be labeled such that a labeled reaction product is released (*e.g.*, formaldehyde and/or succinate) by the demethylation reaction. For example, the protein or histone substrate can be methylated with ³H-SAM (S-adenosylmethionine), which results in the release of ³H-formaldehyde in the demethylation reaction, which can be detected directly or by conversion to ³H-DDL, which can then be detected.

Reaction products such as formaldehyde and/or succinate can be detected by any other suitable method in the art, for example, mass spectrometry, gas chromatography, liquid chromatography, immunoassay, electrophoresis, and the like, or any combination of the foregoing. For example, formaldehyde and/or succinate can be detected by mass spectrometry (*e.g*., by detection of the protonated form of formaldehyde or by detection of succinic acid).

The inventors have identified a family of demethylases comprising JmjC domains. Thus, the invention encompasses methods of demethylating a methylated protein, the method comprising contacting the methylated protein with a demethylase comprising a JmjC domain under conditions sufficient for demethylation. Also encompassed is the use of a JmjC domain-containing protein as a demethylase (*e.g*., as a laboratory reagent). In particular embodiments, the demethylase is a lysine demethylase and the protein is methylated on a lysine residue(s). The demethylase can be a histone demethylase and the methylated protein can be a methylated histone (*e.g*., histone H3 or H4). Suitable methylated histone substrates include but are not limited to a methylated core histone substrate, mononucleosome substrate, dinucleosome substrate and/or oligonucleosome substrate, a peptide substrate and/or chromatin. Further, the methylated protein or histone can comprise one or more methyl groups at a single lysine or arginine residue (*e.*g., mono-, di- and/or tri-methylated proteins or histones). In representative embodiments, the methylated protein comprises a methylated H3-K36, which may further be a mono-, di-and/or tri-methylated H3-K36, and the demethylase is a H3-K36 demethylase. In other embodiments, the methylated protein comprises a methylated H3-K9, which may further be a mono-, di- and/or tri-methylated H3-K9, and the demethylase is a H3-K9 demethylase. In other illustrative embodiments, the methylated protein comprises a methylated H3-K4, which may further be a mono-, di- and/or tri-methylated H3-K4, and the demethylase is a H3-K4 demethylase.

In some exemplary embodiments of the invention, the demethylase is a JHDM1 protein. According to this aspect of the invention, the methylated protein substrate can be a methylated H3-K36 and optionally a mono- and/or di-methylated H3-K36.

Alternatively, the demethylase can be a JHDM2 protein (*e.g*., JHDM2A). According to this aspect of the invention, the methylated protein substrate can be a methylated H3-K9 and optionally a mono- and/or di-methylated H3-K9.

In other embodiments, the demethylase can be a JHDM3 protein (*e.g*., JDHM3A). According to this aspect of the invention, the methylated protein substrate can be a methylated H3-K9 and/or H3-K36, and optionally is a mono-, di- and/or tri-methylated H3-K9 and/or H3-K36.

In yet other representative embodiments, the demethylase is a JARID protein (as described herein, *e.g*., RBP2 and/or JARID1B). According to this aspect of the invention, the methylated protein substrate can be a methylated H3-K4 and optionally a mono-, di- and/or tri-methylated H3-K4.

The invention also encompasses a kit for carrying out the inventive assays, the kit comprising: (a) a demethylase comprising a JmjC domain; and (b) written instructions for methods of using the JmjC domain-containing protein to carry out a demethylation reaction, and optionally additional reagents or apparatus for using the JmjC domain-containing protein to carry out a demethylation reaction. In particular embodiments, the JmjC domain-containing protein is a JHDM1 protein, JDHM2 protein and/or a JHDM3 protein. The kit can optionally comprise Fe(II), α-ketoglutarate or ascorbate, or any combination of the foregoing. The kit can further comprise ³H-SAM.

In some embodiments, the kit further comprises a histone substrate. The histone substrate can comprise histone H3, optionally H3-K36 (e.g., mono-, di-methyl and/or tri-methyl H3-K36) and/or H3-K9 (e.g., mono-, di- and/or tri-methyl H3-K9). Suitable histone substrates include but are not limited to a methylated core histone substrate, mononucleosome substrate, dinucleosome substrate, oligonucleosome substrate and/or a peptide substrate.

The inventors have identified and functionally characterized a novel family of demethylases comprising a JmjC domain. Accordingly, as yet a further aspect, the invention provides a method of identifying a protein as a candidate demethylase, optionally a lysine demethylase, comprising determining the presence of a JmjC domain in the protein (*e.g*., by identifying a protein associated with demethylase activity and then sequencing the protein [or coding nucleic acid] or by evaluating the amino acid or nucleic acid coding sequence of a known protein). In particular embodiments, the method is practiced to identify a candidate histone demethylase, optionally a H3-K36 demethylase, a H3-K9 demethylase and/or a H3-K4 demethylase. The method can further comprise steps to confirm the enzymatic activity and/or substrate specificity of the candidate demethylase, for example, by using a demethylase assay according to the present invention and as described herein.

### Screening Methods.

The present invention further provides methods of identifying a compound that modulates the demethylase activity of a demethylase comprising a JmjC domain. Optionally, the method can be practiced to identify a compound that modulates the demethylase activity of a histone demethylase (*e.g*., a histone H3 or H4 demethylase).

Any suitable assay for detecting or determining demethylase activity can be used to identify compounds that modulate demethylase activity.

In particular emdodiments, the invention provides a method of identifying a compound that modulates the demethylase activity of a demethylase comprising a JmjC domain, the method comprising: contacting the demethylase with a methylated protein substrate in the presence of a test compound; and detecting the level of demethylation of the protein substrate under conditions sufficient for demethylation, wherein a change in demethylation of the protein substrate as compared with the level of demethylation in the absence of the test compound indicates that the test compound is a modulator of the demethylase activity of the demethylase. In particular embodiments, the demethylase is a histone demethylase, and the methylated protein substrate is a methylated histone substrate. The methylated histone substrate can be a methylated histone H3, including methylated H3-K36, H3-K9 and/or H3-K4. Methylated histone substrates can be mono-, di- or tri-methylated at a particular residue, which can be a lysine or arginine. One exemplary substrate is a mono-, di- and/or tri-methylated H3-K36. Another illustrative substrate is a mono-, di- and/or tri-methylated H3-K9. A further representative substrate is a mono-, di- and/or tri-methylated H3-K4. In addition, the methylated histone substrate can be a methylated core histone substrate, mononucleosome substrate, dinucleosome substrate and/or oligonucleosome substrate, a peptide substrate and/or chromatin (*e.g*., in cell based assays).

The invention can be practiced with any JHDM protein, *i.e*., a demethylase (including a histone demethylase) that comprises a JmjC domain. In particular embodiments, the JHDM protein is a JHDM1 protein, a JHDM2 protein, a JHDM3 protein, a JARID protein (*e.g*., a JARID1 subfamily protein such as RBP2, JARID1B [PLU1], JARID1C [SMCX], and JARID1D [SMCY] or a JARID2 subfamily protein), a UTX/UTY protein, a PHF2/PHF8 subfamily protein, or a JmjC domain only subfamily protein.

According to the present invention, "detecting the level of demethylation" may be performed by any method known in the art. In particular embodiments, demethylation may be detected directly (*e.g*., by detecting reaction products of the demethylation reaction such as formaldehyde and/or succinate). Alternatively, the level of methylation can be detected and the level of demethylation determined therefrom (*e.g*., by detecting methylated protein by detecting labeled methylated protein (*e.g*., methylated with ³H-SAM) or by using an antibody specific for the methylated protein.

Methylated protein (including histone) substrates can be prepared by any method known in the art. For example, histones can be methylated using histone methyltransferases, which can be specific for a particular methylation site of interest. Exemplary histone methyltransferases include EZH2, SET7, G9a, PRMT1, Set2, hDOT1L, Dim5, Suv39H1 and Suv4-20h1. In some embodiments, the protein or histone substrate is methylated in its native form.

A reduction in demethylation activity as compared with the level of demethylation in the absence of the test compound indicates that the test compound is an inhibitor of the demethylase activity of the demethylase. Conversely, an increase in demethylation activity as compared with the level of demethylation in the absence of the test compound indicates that the test compound is an activator of the demethylase activity of the demethylase.

Modulation of the demethylase activity of the demethylase can be determined by any method known in the art, for example by a demethylation assay as described herein comprising the steps of contacting the demethylase with a protein substrate, Fe(II), α-ketoglutarate and, optionally, ascorbate, and detecting the release of reaction product (*e.g*., formaldehyde and/or succinate) by detecting a label (*e.g*., radioactivity), by mass spectrometry or any other method known in the art.

Alternatively, an antibody that is specific for the methylated form of the protein can be used to detect the level of demethylation, *e.g*., by immunoprecipitation, by ELISA, or to identify bands in a Western blot. The methylation state of the protein can also be determined using antibodies specific to mono-, di- and tri-methylated proteins. Antibodies against mono-, di- and tri-methylated H3-K36, H3-K9 and H3-K4 are described herein.

As a further possibility, a protein substrate methylated with labeled methyl groups can be bound to a surface (*e.g*., the bottom of a multi-well plate, a filter, a matrix or a bead). The bound protein substrate can be contacted with the demethylase, the test compound, and cofactors (*e.g*., Fe(II) and α-ketoglutarate and, optionally, ascorbate). Demethylation can then be determined by release of the label or by a reduction in label bound to the surface.

Any detectable label can be used with the present invention including but not limited to radiolabels (*e.g*., ³H), fluorescence labels, colorimetric labels, and the like. Alternatively, demethylation can be detected by detecting the release of formaldehyde and/or succinate, as described herein.

In particular embodiments of the inventive screening methods, a reduction in demethylase activity (*e.g*., as determined by detecting formaldehyde release) as compared with the level of demethylase activity detected in the absence of the test compound indicates that the test compound is an inhibitor of the demethylase activity of the demethylase, *e.g*., as compared to the level of activity in the absence of the test compound.

In other embodiments, an enhancement of demethylase activity compared with the level of demethylase activity detected in the absence of the test compound indicates that the test compound is an activator of the demethylase activity of the demethylase, as compared to the level of activity in the absence of the test compound.

Inhibitors or activators identified in the first round of screening can optionally be evaluated further to determine the IC₅₀ and specificity using demethylase assays as described herein or any other suitable assay. Compounds having a relatively low IC₅₀ and exhibiting specificity for the protein substrate of interest can be further analyzed in tissue culture and/or in a whole organism to determine their *in vivo* effects on demethylase activity, cell proliferation, hair growth, and/or toxicity.

The inventive screening methods can be cell-based or cell-free. Cell-based methods can be carried out in cultured cells or in whole organisms. In representative embodiments, the method provides high throughput screening capability to identify modulators of the demethylase(s). To illustrate, a cell-based, high throughput screening assay for use in accordance with the methods disclosed herein includes that described by Stockwell et al. ((1999) Chem. Bio. 6:71-83), wherein biosynthetic processes such as DNA synthesis and post-translational processes are monitored in a miniaturized cell-based assay.

Compounds that modulate demethylase activity can also be identified by identifying compounds that bind to the demethylase. High throughput, cell-free methods for screening small molecule libraries for candidate protein-binding molecules are well-known in the art and can be employed to identify molecules that bind to the demethylase and modulate the demethylase activity and/or bind to the methylated protein substrate. For example, a protein substrate, free histones or nucleosomal substrates purified from HeLa cells can be coated on a multi-well plate or other suitable surface and a reaction mix containing the demethylase added to the substrate. Prior to, concurrent with and/or subsequent to the addition of the demethylase, a test compound can be added to the well or surface containing the substrate (*e.g*., filter, well, matrix, bead, *etc*.). The reaction mixture can be washed with a solution, which optionally reflects physiological conditions to remove unbound or weakly bound test compounds. Alternatively, the test compound can be immobilized and a solution of demethylase can be contacted with the well, matrix, filter, bead or other surface. The ability of a test compound to modulate binding of the demethylase to the substrate can be determined by any method in the art including but not limited to labeling (*e.g*., radiolabeling or chemiluminescence) or competitive ELISA assays.

Test compounds that can be screened in accordance with the methods provided herein encompass numerous chemical classes including, but not limited to, synthetic or semi-synthetic chemicals, purified natural products, proteins, antibodies, peptides, peptide aptamers, nucleic acids, oligonucleotides, carbohydrates, lipids, or other small or large organic or inorganic molecules. Small molecules are desirable because such molecules are more readily absorbed after oral administration and have fewer potential antigenic determinants. Non-peptide agents or small molecule libraries are generally prepared by a synthetic approach, but recent advances in biosynthetic methods using enzymes may enable one to prepare chemical libraries that are otherwise difficult to synthesize chemically.

Small molecule libraries can be obtained from various commercial entities, for example, SPECS and BioSPEC B.V. (Rijswijk, the Netherlands), Chembridge Corporation (San Diego, CA), Comgenex USA Inc., (Princeton, NJ), Maybridge Chemical Ltd. (Cornwall, UK), and Asinex (Moscow, Russia). One representative example is known as DIVERSet^{™}, available from ChemBridge Corporation, 16981 Via Tazon, Suite G, San Diego, Calif. 92127. DIVERSet^{™} contains between 10,000 and 50,000 drug-like, hand-synthesized small molecules. The compounds are preselected to form a "universal" library that covers the maximum pharmacophore diversity with the minimum number of compounds and is suitable for either high throughput or lower throughput screening. For descriptions of additional libraries, *see, e.g.,* Tan et al., (1998) Am. Chem Soc. 120: 8565-8566; and Floyd et al., (1999) Prog Med Chem 36:91-168. Other commercially available libraries can be obtained, *e.g*., from AnalytiCon USA Inc., P.O. Box 5926, Kingwood, Tex. 77325; 3-Dimensional Pharmaceuticals, Inc., 665 Stockton Drive, Suite 104, Exton, Pa. 19341-1151; Tripos, Inc., 1699 Hanley Rd., St. Louis, Mo., 63144-2913, etc. In certain embodiments of the invention, the methods are performed in a high-throughput format using techniques that are well known in the art, *e.g*., in multiwell plates, using robotics for sample preparation and dispensing, *etc*. Representative examples of various screening methods may be found, for example, in U.S. Pat. Nos. 5,985,829, 5,726,025, 5,972,621, and 6,015,692. The skilled practitioner will readily be able to modify and adapt these methods as appropriate.

A variety of other reagents can be included in the screening assays of the instant invention. These include reagents like salts, ATP, neutral proteins, *e.g*., albumin, detergents, *etc*., which can be used to facilitate optimal protein-protein binding and/or enzymatic activity and/or reduce non-specific or background interactions. Also, reagents that otherwise improve the efficiency of the assay, such as protease inhibitors, nuclease inhibitors, anti-microbial agents, and the like may be used. The mixture of components can be added in any order that permits binding and/or enzymatic activity.

Histone methyltransferases have been linked to cancer, indicating that the enzymatic activity of histone demethylases is also a good target for drug development for cancer treatment. Accordingly, the invention also provides a method of identifying a candidate compound for treating cancer, the method comprising: contacting a histone demethylase comprising a JmjC domain with a methylated histone substrate in the presence of a test compound; and detecting the level of demethylation of the histone substrate under conditions sufficient for demethylation, wherein a change in demethylation of the histone substrate as compared with the level of demethylation in the absence of the test compound indicates that the test compound is a candidate compound for the treatment of cancer.

Exemplary cancers include malignant disorders such as breast cancers; osteosarcomas; angiosarcomas; fibrosarcomas and other sarcomas; leukemias; lymphomas; sinus tumors; ovarian, cervical, uterine, uretal, bladder, prostate and other genitourinary cancers; colon, esophageal and stomach cancers and other gastrointestinal cancers; lung cancers; myelomas; pancreatic cancers; liver cancers; kidney cancers; endocrine cancers; skin cancers; and brain or central and peripheral nervous (CNS) system tumors, malignant or benign, including gliomas and neuroblastomas.

In particular embodiments, JARID1B [PLU1] target to identify compounds to reduce proliferation of breast cancer cells and/or to treat breast cancer.

Histone substrates are as described above with respect to methods of identifying modulators of demethylase activity. Likewise, the level of demethylation can be determined by any method known to those in the art as described above.

The Hairless protein, which controls hair growth, is a JmjC-domain containing protein. This protein is mutated in individuals with *alopecia universalis.* Thus, if Hairless is a demethylase as well, compounds that modulate the demethylase activity of Hairless can be identified to treat hair loss. Accordingly, the invention also provides a method of identifying a candidate compound for treating hair loss, the method comprising: contacting a Hairless protein with a methylated protein substrate in the presence of a test compound; and detecting the level of demethylation of the protein substrate under conditions sufficient for demethylation, wherein a change in demethylation of the protein substrate as compared with the level of demethylation in the absence of the test compound indicates that the test compound is a candidate compound for the treatment of hair loss. In particular embodiments, the invention is practiced to identity compounds that activate the demethylase activity of Hairless and increase demethylation.

Protein substrates are as described with respect to the foregoing screening methods. Further, methods of determining the level of demethylation can be carried out by any method known to those in the art as described above.

### Methods of Modulating Demethylation.

The invention further provides compounds identified by the screening methods of the invention. In further embodiments, the invention provides pharmaceutical preparations comprising a compound identified by the screening methods of the invention and a pharmaceutically acceptable carrier. The present invention further provides the use of a compound identified by the screening methods of the invention for the preparation of a medicament (*e.g*., to treat cancer or hair loss).

Also encompassed by the present invention are methods of modulating demethylation in a cell or in a subject, either generally or with respect to one or more specific target genes. Demethylation can be modulated to control a variety of cellular functions, including without limitation: differentiation; proliferation; apoptosis; tumorigenesis, leukemogenesis or other oncogenic transformation events; hair loss; or sexual differentiation. For example, in particular embodiments, the invention provides a method of treating cancer in a subject that has cancer or is considered at risk for cancer by modulating the activity of a demethylase comprising a JmjC domain (*e.g*., a histone demethylase such as a JHDM protein(s)). To illustrate, the expression of an oncogene can be suppressed by modulating H3-K9, H3-K36 and/or H3-K4 demethylation of the oncogene and/or the expression of a tumor suppressor gene can be increased by modulating H3-K9, H3-K36 and/or H3-K4 demethylation of the tumor suppressor gene. Exemplary cancers that can be treated according to the present invention are as described above with respect to screening methods.

As another aspect, the invention provides a method of modulating the expression of a steroid hormone-dependent target gene (*i.e*., genes that are targets of steroid hormone receptors) by modulating the activity of a demethylase comprising a JmjC domain (*e.g*., a histone demethylase such as a JHDM protein(s)). Steroid hormone-dependent target genes include but are not limited to androgen-dependent, estrogen-dependent, progesterone-dependent, thyroid hormone-dependent, vitamin D-dependent, and/or corticosteroid-dependent target genes. This embodiment of the invention can be practiced to modulate the effects of sex steroids on target cells (*e.g*., to modulate sexual maturation and/or secondary sex characteristics) or to treat cancer, for example, hormone-sensitive cancers such as androgen-sensitive (*e.g*., prostate) and estrogen-sensitive (*e.g*., breast) cancers.

The inventors have also discovered that a number of pluripotency and differentiation markers are down-regulated by JHDM2 knock-down. In representative embodiments, the invention provides a method of modulating expression of pluripotency and differentiation markers, for example, to regulate cell lineage determination. Cell lineage markers include but are not limited to Nanog, Oct4, Lamb1, Hoxb1 and/or Stra6. Thus, the invention provides methods of modulating expression of a pluripotency or differentiation marker(s) by modulating the activity of a demethylase comprising a JmjC domain (*e.g*., a histone demethylase such as a JHDM protein(s)).

The invention also provides a method of treating hair loss in a subject that has hair loss or is considered at risk for hair loss by modulating the activity of a Hairless protein. In particular embodiments, the invention is practiced to enhance the activity of Hairless and thereby increase demethylation of a target gene.

The activity of the demethylase can be modulated using methods known in the art. For example, the activity of the demethylase can be enhanced by introducing an exogenous nucleic acid encoding the demethylase to increase the production of the demethylase in the subject. Optionally, the heterologous nucleic acid encodes a demethylase having enhanced activity as compared with the native form. Further, a small molecule can be administered to enhance the expression of the demethylase (from an endogenous and/or exogenous coding sequence) and/or the activity of the demethylase protein.

The activity of a demethylase can be reduced using methods known in the art. For example, a ribozyme, an inhibitory RNA (*e.g*., an siRNA or a shRNA), an antisense RNA, or an inhibitory antibody can be administered. Alternatively, a small molecule can be administered to reduce the expression of the demethylase and/or the activity of the demethylase.

Having described the present invention, the same will be explained in greater detail in the following examples, which are included herein for illustration purposes only, and which are not intended to be limiting to the invention.

### Example 1

### METHODS FOR CHARACTERIZATION OF JHDM1 PROTEINS

*Purification of the H3-K36 demethylase activity and Flag®-JHDM1A.* Separation of HeLa S3 nuclear proteins into nuclear extract and nuclear pellet and subsequent solubilization of nuclear pellet proteins, fractionation on DEAE52, and P11 columns were performed according to standard methods (Wang, et al. (2001) Science 293:853-857). The P11 fraction, which eluted with BC300 [40 mM HEPES-KOH (pH 7.9), 0.2 mM EDTA, 1 mM DTT, 0.2 mM PMSF, and 10% glycerol, 300 mM KCI] was dialyzed with buffer D [40 mM HEPES-KOH (pH 7.9), 0.2 mM EDTA, 1 mM DTT, 0.2 mM PMSF, and 10% glycerol] containing 20 mM ammonium sulfate (BD20) and loaded onto a 45 mL DE5PW column (TosoHaas, Montgomeryville, PA). The bound proteins were eluted with a 12-column volume (cv) liner gradient from BD50 to BD500. The fractions containing the demethylase activity, which eluted between 140-185 mM ammonium sulfate, were combined and adjusted to 700 mM ammonium sulfate before loading onto a 22 ml Phenyl Sepharose^{®} column (Pharmacia Biotech, Uppsala, Sweden). The bound proteins were eluted with an 8-cv linear gradient from BD700 to BD50. The active fractions, which eluted from the column between 450-360 mM ammonium sulfate, were pooled and concentrated to 0.5 ml before loading onto a 24 ml Superose^{®} 6 gel filtration column (Pharmacia). The Superose^{®} 6 column was eluted with BC400 (buffer C with 400 mM KCI). The active fractions, which eluted between 240-320 kDa, were then combined and adjusted to 200 mM KCI with BC50 before loading to a 0.1 ml MonoQ^{®} column (Pharmacia). The bound proteins were eluted with a 20-cv linear gradient from BC200 to BC500. The active fractions eluted from the column between 315-345 mM KCI. The proteins in the active fractions were combined and resolved in a 8-15% gradient SDS-PAGE. After Coomassie^{™} staining, candidate polypeptides were excised for protein identification. The generation of baculovirus expressing Flag^{®}-JHDM1A and purification of Flag^{®}-JHDM1A from infected SF9 cells were performed according to established methods (Cao and Zhang (2004) Mol. Cell. 15:57-67).

*Protein Identification and Mass Spectrometry Analysis.* For protein identification, the candidate polypeptides were digested with trypsin and the proteins identified using well-known methods (Wang, et al. (2004) Nature 431:873-878). For peptide substrate analysis, an aliquot (1 µL) of the reaction mixtures was diluted 100-fold with 0.1 % formic acid, and loaded onto a 2-µL bed volume of Poros 50 R2 (PerSeptive Biosystems, Framingham, MA) reversed-phase beads packed into an Eppendorf^{®} gel-loading tip. The peptides were eluted with 5 µL of 30% acetonitrile/0.1 % formic acid. A fraction (0.5 mL) of this peptide pool was analyzed by matrix-assisted laser-desorption/ionization (MALDI) time-of-flight (TOF) mass spectrometry (MS), using a BRUKER^{®} UltraFlex^{™} TOF/TOF instrument (Bruker Daltonics^{®}; Bremen, Germany), as described (Erdjument-Bromage, et al. (1998) J. Chromatogr. A 826:167-181*).* For detection of formaldehyde and succinate, the reaction mixture was diluted 1:10 with aqueous 0.1% triflouro acetic acid and directly analyzed by nano-electrospray mass spectrometry (ESI-MS) and tandem mass spectrometry (ESI-MS/MS) using an Applied Biosystems^{™} (Foster City, CA) QSTAR™ quadrupole time-of-flight instrument. A 5-minute acquisition time and Proxeon (Odense, Denmark) nanospray needles were used, under the conditions previously described (Kast, et al. (2003) Rapid Commun. Mass Spectrom. 17:1825-1834). For optimum sensitivity, only the masses of protonated formaldehyde and succinic acid were selected by the quadrupole and analyzed by the time-of-flight analyzer (selected ion monitoring). The fragmentation analysis of succinic acid by ESI-MS/MS was performed using established methods (Kast, et al. (2003) Rapid Commun. Mass Spectrom. 17:1825-1834).

*In vitro Histone Demethylase Assay.* For the preparation of ³H-labeled methyl-histone octamer or -oligonucleosome substrates, histone methyltransferases were expressed in *E*. *coli* (GST-SET7 for H3-K4, GST-G9a for H3-K9, CBP-Set2-Flag^{®} for H3-K36, GST-hDOT1L for H3-K79, GST-PRMT1 for H4-R3, and GST-Suv4-20h1 for H4-K20), or Sf9 cells (EZH2 complex for H3-K27). The HMTases were incubated with histone octamers (for SET7, G9a, PRMT1), oligonucleosomes (for Set2, hDOT1L, Suv4-20h1) purified from HeLa cells, or oligonucleosomes purified from chicken blood (for the EZH2 complex) in the presence of [³H]-SAM. After the HMTase reaction, the reaction mixtures were dialyzed into histone storage buffer [10 mM HEPES-KOH (pH 7.5), 10 mM KCI, 0.2 mM PMSF, and 10% glycerol] and used as substrates for the histone demethylase assay.

For the demethylase assay, histone octamers, oligonucleosomes (either ³H-labeled or not) or H3-K36 methylated peptide substrates were incubated with protein fractions or purified Flag^{®}-JHDM1A in histone demethylation reaction buffer [50 mM HEPES-KOH (pH 8.0), 7-700 µM Fe(NH₄)₂(SO₄)₂, 1 mM α-ketoglutarate, 2 mM ascorbate] at 37°C for 1-3 hours. The reaction mixtures were analyzed by NASH method, western blot, and mass spectrometry. For detection of ³H-labeled formaldehyde; a modified NASH method (Kleeberg and Klinger (1982) J. Pharmacol. Methods 8:19-31) was used. After TCA precipitation, equal volume of NASH reagent (3.89 M ammonium acetate, 0.1 M acetic acid, 0.2% 2,4-pentanedione) was added into the supernatant and the mixtures were incubated at 37°C for 50 minutes. The reaction mixture was then extracted with an equal volume of 1-pentanol. The radioactivity of the 1-pentanol-phase was measured by scintillation counting. For detection of demethylation with peptide substrates, peptides in the reaction mixture were desalted on a RP micro-tip and analyzed by MALDI-TOF as described above. For detection of histone demethylation using western blot analysis, demethylation reactions were subjected to western blotting using methyl-specific antibodies.

*Constructs and Antibodies.* Plasmids encoding GST-SET7, GST-hDOT1L (1-416), GST-PRMT1 and components of the EZH2 complex have previously been described (Cao and Zhang (2004) Mol. Cell. 15:57-67; Min, et al. (2003) Genes Dev. 17:1823-1828; Wang, et al. (2001) Mol. Cell. 8:1207-1217; Wang et al. (2001) Science 293:853-857). Plasmids encoding GST-G9a (621-1000), CBP-Set2-Flag^{®} (*S. pombe*), and GST-Suv4-20h1 were kindly provided by Drs. Shinkai, Strahl, and Jenuwein, respectively. A plasmid encoding Flag^{®}-JHDM1A (human) was constructed by PCR amplification from I.M.A.G.E. cDNA clone (5534384). The full-length coding sequence was inserted into *Not*l and *Xba*l sites of N-terminal Flag^{®}-tagged pcDNA3 vector and N-terminal Flag^{®}-tagged pFASTBAC™ vector. The pcDNA3-Flag^{®}-JHDM1A (H212A), the deletion constructs in the JmjC (148-316 aa) domain, zf-CXXC (563-609 aa) motif, PHD (619-676 aa) domain, FBOX (893-933 aa) domain, and LRRs (1000-1118 aa) were generated by two-step PCR. Plasmids encoding GST-scJHDM1 (*S. cerevisiae*) were constructed by PCR amplification of *S*. *cerevisiae* genomic DNA. The GST-scJHDM1 (H305A) and GST-scJHDM1 (Y315A) mutants were generated by two-step PCR. All of the constructs generated through PCR were verified by DNA sequence analysis.

The antibodies against H3 monomethyl-K36 and trimethyl-K36 were purchased from Abcam^{®} (Cambridge, MA). The antibody against H3 dimethyl-K36 was generated in rabbits by injection of a synthetic peptide (STGGVKKPHRY-C; **SEQ ID NO:1**), in which K36 (underlined) was dimethylated. The antibodies against H3 dimethyl-K4 have previously been described (Feng, et al. (2002) Curr. Biol. 12:1052-1058). The antibody against Flag^{®} and secondary antibodies for immunofluorescence were purchased from Sigma™ (St. Louis, MO) and Jackson ImmunoResearch Laboratories (West Grove, PA), respectively. The antibody against H3 was kindly provided by Dr. Verreault.

*Protein Expression in Mammalian Cells and Immunopurification.* All of the GST and CBP fusion proteins were expressed in *E*. *coli* and purified on glutathione-immobilized agarose beads (Sigma™), or calmodulin affinity resin (Stratagene^{®}, La Jolla, CA). The expression and purification of the EZH2 complex was performed according to known methods (Cao and Zhang (2004) Mol. Cell. 15:57-67). For immunoprecipitation of wild-type and mutant Flag^{®}-JHDM1A proteins, COS-7 cells were transiently transfected with plasmids by FuGENE™ 6 following the manufacture's protocol. Two days after transfection, cells were washed with phosphate-buffered saline (PBS) before being lysed with lysis buffer (20 mM HEPES-NaOH, pH 7.5, 3 mM MgCl₂, 100 mM NaCl, 1 mM Na₃VO₄, 10 mM NaF, 20 mM β-Glycerophosphate, 1 mM EGTA, and 0.5% NP-40) containing protease inhibitor cocktail (Roche Applied Science, Nutley, NJ) and 1 mM phenylmethyl sulfonate fluoride. The lysates were cleared by centrifugation, and the amounts of lysate for immunoprecipitation were adjusted based on protein expression level. The adjusted amounts of cell lysate were incubated with M2 α-Flag^{®} agarose (Sigma™) for 3 hours at 4°C. After centrifugation, the beads were washed with lysis buffer once and with BC50 without EDTA twice. The immunoprecipitated proteins were used for demethylase assay and western blot analysis.

*Immunostaining.* 293T cells were plated onto glass coverslips in a 12-well plate and cultured for 1 day. After washing with PBS, cells were fixed in 4% paraformaldehyde for 10 minutes. The cells were then washed once with cold PBS permeabilized for 5 minutes with cold PBS containing 0.2% Triton^{®} X-100. Permeabilized cells were then washed three times with blocking buffer (1% bovine serum albumin in PBS) and blocked for 30 minutes and subsequently incubated with primary antibodies for 1 hour in a humidified chamber. After three consecutive 5-minute washes with PBS, cells were incubated with secondary antibodies for 1 hour. The cells were then washed with PBS and stained with 4,6-diamidino-2-phenylindole dihydrochloride (DAPI) in PBS. Cells were washed again twice with PBS and then mounted in fluorescent mounting medium (Dako, Glostrup, Denmark) before being viewed under a fluorescence microscope.

### Example 2

### HISTONE DEMETHYLATION BY A FAMILY OF JmjC DOMAIN-CONTAINING PROTEINS

*Identification of a Histone Demethylase Activity in HeLa Extracts.* Methyl-groups of 1-methyladenine (1-meA) and 3-methylcytosine (3-meC) in DNA can be removed by the AlkB family of proteins through oxidative demethylation (**Scheme 1**)(Falnes, et al. (2002) Nature 419:178-182; Trewick, et al. (2002) EMBO Rep. 6:315-320). 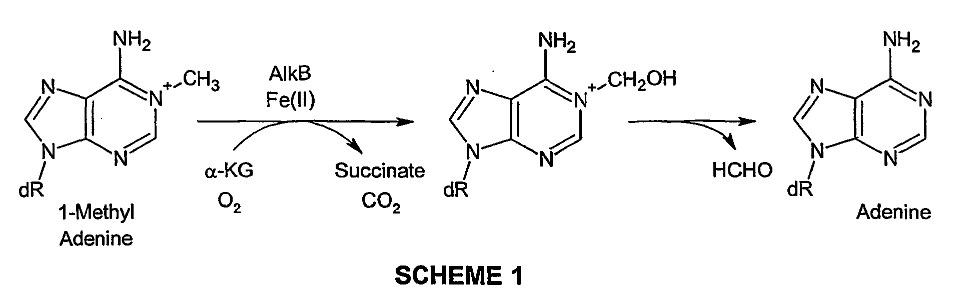 This suggested that a similar mechanism might be employed for the removal of methyl-groups from methylated histones (Scheme 2).

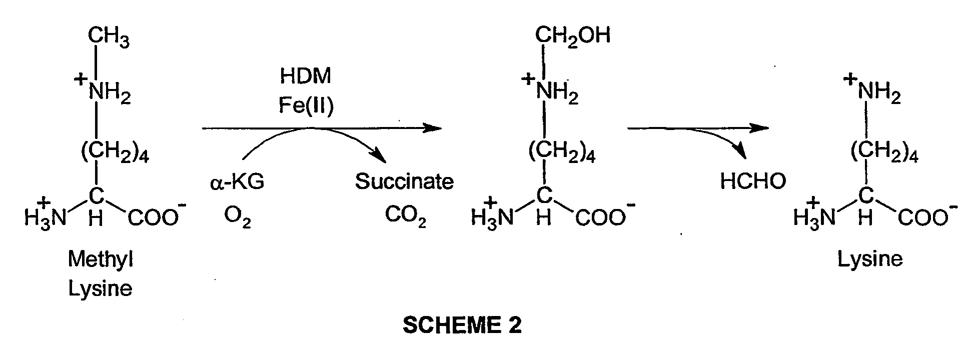

To demonstrate this, an *in vitro* assay was developed based on detection of one of the predicted release products, formaldehyde. To maximize detection sensitivity, nucleosomal histone substrates were radiolabeled by incubation with the histone H3 lysine 36 (H3-K36)-specific methyltransferase Set2 and [³H]-SAM. As outlined in **Scheme 3,** unincorporated [³H]-SAM was removed by dialysis, then the labeled substrates were subjected to demethylation reactions in the presence of cofactors Fe(II) and α-ketoglutarate (α-KG). To detect the released [³H]-formaldehyde, we first removed contaminating labeled histone and proteins by TCA precipitation. Then, through a chemical reaction, we converted the predicted reaction product formaldehyde to 3,5-diacethyl-1 ,4-dihydrolutidine (DDL), which was detected by scintillation counting after extraction in organic solvents (**Scheme 3**).

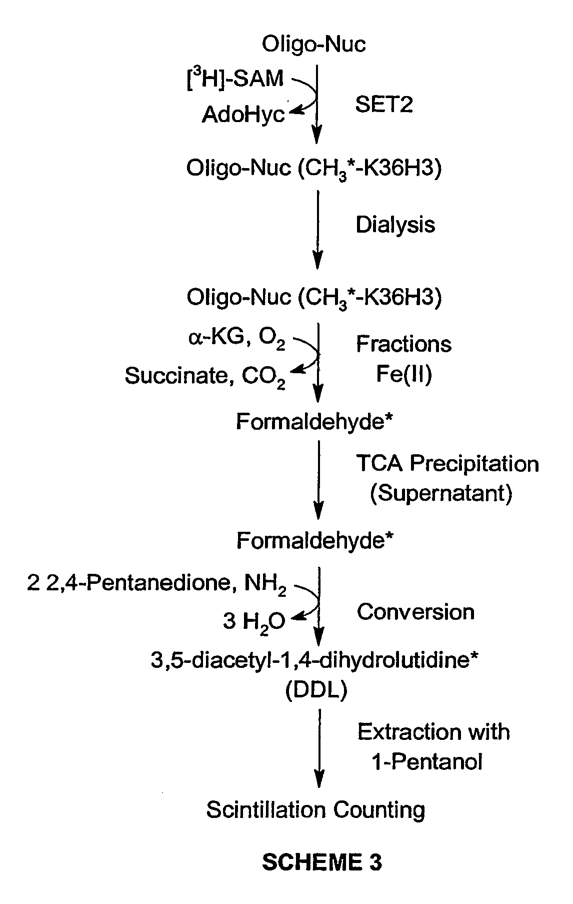

Using the assay described above, we analyzed the protein fractions derived from HeLa nuclear extracts (NE) and nuclear pellet (NP) (Wang, et al. (2001) Science 293:853-857). Results shown in **Figure 1A** indicate that a demethylase activity is enriched in the nuclear pellet-derived 0.3 M P11 fraction. To ascertain whether the activity detected is the result of a real demethylase activity, we examined its dependency on the predicted co-factors and reaction conditions. Results shown in **Figure 1** **B** demonstrate that release of formaldehyde not only requires the presence of the protein fraction (lane 1), but also the co-factors Fe(II) and α-KG (lanes 3 and 4). In addition, ascorbate is also required for optimal activity, probably due to its ability to regenerate Fe(II) from Fe(III). These results demonstrate that the enzyme(s) present in the 0.3 M P11 fraction can use the indicated mechanism for histone demethylation.

*Idenfification of a Novel JmjC Domain-Containing Protein as a Histone Demethylase.* To identify the protein(s) responsible for the demethylase activity, we monitored the enzymatic activity through six chromatography columns (**Scheme 4**), wherein numbers represent the salt concentrations (mM) at which the histone demethylase activity elutes from the column.

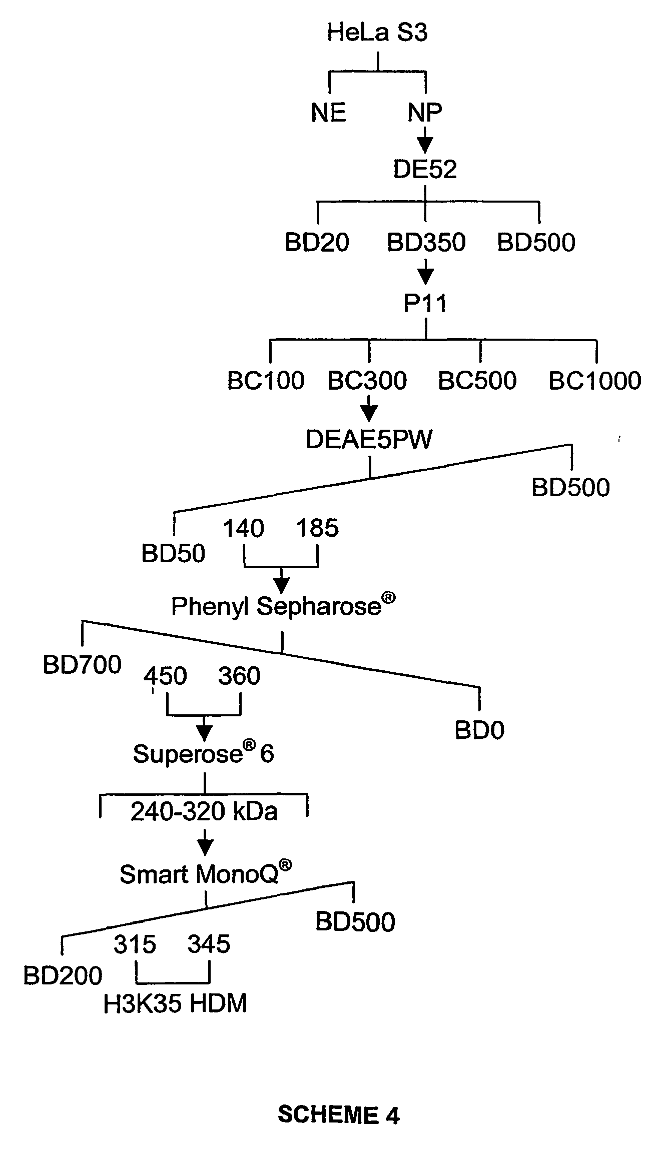

After purification of the 0.3 M P11 fraction through DEAE5PW and Phenyl Sepharose^{®} columns, we determined the relative mass of the enzymatic activity on a Superose^{®} 6 gel-filtration column and found its native size to be about 300 kDa (**Figure 2A**). Further purification on a MonoQ^{®} column allowed us to correlate two protein bands, marked by * (**Figure 2B****,** top panel), with the enzymatic activity (**Figure 2B**, second panel). To identify the two proteins, we pooled the MonoQ^{®} samples between fractions 27-30. After concentration, the samples were resolved on SDS-PAGE and the two candidate protein bands were recovered for identification. Mass spectrometry analysis identified the larger of the two proteins as F-box and leucine-rich repeat protein 11 (FBXL11) (**Figure 2C**).

FBXL11 was originally identified by searching the human expressed sequence tag (EST) database for F-box-containing proteins (Cenciarelli, et al. (1999) Curr. Biol. 9:1177-1179; Winston, et al. (1999) Curr. Biol. 9:1180-1182), but the function of FBXL11 has not been characterized. In addition to an F-box, FBXL11 contains several interesting domains including a JmjC domain, a CxxC (**SEQ ID NO:2**) zinc-finger, a PHD domain, and three leucine-rich repeats (**Figure 3A**). Previously, JmjC domain-containing proteins have been predicted to be metalloenzymes that regulate chromatin function (Clissold and Ponting (2001) Trends Biochem. Sci. 26:7-9). This prediction, in combination with the fact that the demethylase activity requires Fe(II) as a co-factor, indicate that FBXL11 is likely responsible for the purified histone demethylase activity in **Figure 2****.** To demonstrate this, we transfected COS-7 cells with a Flag^{®}-tagged FBXL11 mammalian expression vector. After immunoprecipitation with the anti-Flag^{®}-coated beads, half of the immunoprecipitates were used for western blot analysis and half for enzymatic activity assays. Results shown in **Figure 3B** (compare lanes 1 and 2) revealed a robust histone demethylase activity in the Flag^{®}-FBXL11 immunoprecipitates.

To evaluate the importance of the various domains of FBXL11 for its enzymatic activity, a series of expression constructs was generated with deletions of the JmjC domain, the CxxC (**SEQ ID NO:2**) zinc-finger, the PHD domain, the F-box, or the leucine-rich repeat, respectively (**Figure 3A**). After transfection and immunoprecipitation, these mutant proteins were subjected to western blot analysis and demethylase activity assays. After normalizing for protein expression levels, the activities of the various deletion mutants were determined (**Figure 3B**). The results demonstrate that only the JmjC domain is absolutely required, although deletion of the CxxC (**SEQ ID NO:2**) zinc-finger, PHD domain, and the leucine rich repeats also partially impaired the enzymatic activity (compare lanes 4-8 with 2; **Figure 3B**). To further demonstrate the importance of the JmjC domain for enzymatic activity, a single amino acid mutant H212A was generated. We chose to mutate H212 because this histidine is highly conserved in the JmjC domain of FBXL11 orthologs (**Figure 7B**). In addition, the corresponding histidine in FIH [factor-inhibiting HIF (hypoxia-inducible factor)], a known Fe(II) dependent oxygenase, was found to directly bind to Fe(II) (Elkins, et al. (2003) J. Biol. Chem. 278:1802-1806). Fe(II)-dependency of the histone demethylase activity (**Figure 1B**) indicates that the H212A mutation will disrupt Fe(II) binding, and thus impair the enzymatic activity of FBXL11. Results shown in **Figure 3B** confirmed this (compare lanes 2 and 3). Based on the above results it was concluded that FBXL11 is a novel histone demethylase and that the JmjC domain is critical for its enzymatic activity. Since histone demethylase activity is the first demonstrated function for FBXL11 and because FBXL11 is the first JmjC domain-containing protein shown to possess histone demethylase activity, this protein has been named JHDM1A (JmjC domain-containing histone demethylase 1A.) to reflect its newly identified function. The highly related protein FBXL10 (**Figure 7A**), which we have named JHDM1B, is also an active H3-K36 demethylase (data not shown).

*JHDM1A Preferentially Demethylates H3 Dimethyl-K36.* To further characterize JHDM1A, we generated a baculovirus expressing a Flag^{®}-tagged JHDM1A and purified the protein from infected Sf9 cells by affinity chromatography. After evaluating the purity and quantifying the Flag^{®}-JHDM1A protein (**Figure 4A**), we analyzed its site specificity using histone substrates radiolabeled at all known methylated sites in histones H3 (K4, K9, K27, K36, K79) and H4 (K20). As a representative of methyl-arginine, we also generated H4-R3-methylated substrates. Of the seven substrates, only H3-K36 methylated by Set2 was a substrate for JHDM1A (**Figure 4B**). Thus it was concluded that JHDM1A is an H3-K36-specific demethylase.

Lysine residues exist in three methylation states (mono-, di-, and tri-methylation). To determine whether JHDM1A preferentially demethylates a particular methylation state, we prepared unlabeled core histones, mononucleosomes, and oligonucleosomal substrates (Fang, et al. (2004) Methods Enzymol. 377:213-226). After subjecting these substrates to demethylation reactions with or without enzyme, the methylation levels were measured by western blot analysis using antibodies specific for mono-, di-, and tri-methylated H3-K36. Results shown in **Figure 4C** indicate that JHDM1A preferentially demethylates dimethyl-K36 (second panel, compare lanes 2, 4, 6 with 1, 3, 5) although a decrease in monomethyl K36 levels was also observed (first panel). In contrast, no change in trimethyl-K36 levels was detected. Under these assay conditions, JHDM1A was capable of demethylating H3-K36 regardless of whether it was in free, mono-, or oligo-nucleosome form. To analyze the dimethyl-K36 selectivity of JHDM1A further, synthetic peptides corresponding to the histone H3 N-terminal tail were di- or trimethylated at K36 and subjected to demethylation reactions with or without recombinant Flag^{®}-JHDM1A. Mass spectrometry analysis of the reaction containing dimethyl-peptide substrates revealed the generation of mono- and unmethylated forms of the peptide in a JHDM1A-dependent manner (**Figure 4D**) indicating that the dimethyl-K36 peptide can serve as a substrate for JHDM1A. In contrast, we did not detect any demethylation when a trimethyl-K36 peptide was subjected to a parallel reaction (data not shown). Based on the above results, it was concluded that JHDM1A selectively demethylates H3-K36 with a preference for the dimethylated form.

*JHDM1A Demethylates H3 Dimethyl-K36 in vivo*. Having demonstrated demethylase activity for JHDM1A *in vitro*, we sought to test its activity *in vivo*. Since our attempts at generating stable JHDM1A knock-down cell lines were unsuccessful, it was determined whether H3 K36 methylation levels were affected by over expression of JHDM1A. Data presented in **Figure 5B** indicates that over-expression of Flag^{®}-JHDM1A in 293T cells resulted in a significant decrease in dimethyl-K36 levels, but did not alter the level of monomethyl-K36, trimethyl-K36, nor the level of dimethyl-K4 (**Figures 5A**, **5D**, **5E**). The effect of Flag^{®}-JHDM1A on the dimethyl-K36 level depends on its enzymatic activity as over expression of an enzymatically defective mutant did not affect dimethyl-K36 levels (**Figure 5C**). From these results it was concluded that JHDM1A demethylates H3 dimethyl-K36 *in vivo*.

*JHDM1A-Mediated Histone Demethylation Generates Formaldehyde and Succinate*. Having demonstrated the enzymatic activity of JHDM1A *in vitro* and *in vivo*, we then verified the reaction mechanism. As shown in **Scheme 2**, the demethylation reaction generates formaldehyde and succinate. Although conversion of 2,4-pentanedione into DDL is consistent with formaldehyde as a reaction product (**Scheme 3**), it does not directly prove its presence. Therefore, we used mass spectrometry to directly detect formaldehyde. Under the assay conditions, formaldehyde would exist in its protonated form with a mass-to-charge (m/z) ratio of 31.0184. The results shown in **Figure 6A** demonstrate that formaldehyde is the reaction product based on the appearance of an ion in the reaction mixture at the molecular weight of protonated formaldehyde (m/z 31.0239). Formation of formaldehyde is dependent on the presence of JHDM1A in the reaction (compare top and bottom panels). Using a similar approach, we also detected an ion (at m/z 119.0778) that correlates with protonated succinic acid (C₄O₄H₇) in a JHDM1A-dependent manner (**Figure 6B**). Because an ion of similar m/z, 119.1215 (but lower abundance ~20%) was also detected in the reaction mixture in the absence of JHDM1A (**Figure 5B**, bottom panel), we sought to verify that the ion detected in the presence of JHDM1A was indeed succinic acid. Using a MS/MS reference spectrum of pure succinic acid (**Figure 6C**, top panel), we analyzed the fragmentation pattern of the predicted succinic acid ion produced in the demethylation reaction. The fragmentation pattern matched the reference spectrum of succinic acid indicating that the reaction produced succinic acid (**Figure 6C**, compare top and middle panels). Importantly, no signal was detected from the reaction in the absence of JHDM1A (bottom panel), indicating that the lower abundance ion detected in the control reaction (**Figure 6B**, bottom panel) is not succinic acid. Taken together, these mass spectrometric analysis demonstrate that JHDM1A-mediated histone demethylation generates formaldehyde and succinic acid, thus confirming the demethylation mechanism outlined in **Scheme 2**.

*JmjC Domain-Mediated Histone Demethylation is Conserved from Human to Yeast*. Having established a critical role for the JmjC domain in JHDM1A-mediated histone H3-K36 demethylation (**Figure 3**), it was determined whether the function of JmjC domain is evolutionarily conserved. A search of the SMART database revealed 538 JmjC domain-containing proteins in organisms from bacteria to human. The number of JmjC domain-containing proteins in each organism appeared to correlate to genome complexity with 109 in human, 86 in mouse, 19 in *Drosophila,* 15 in *C. elegans*, 7 in *S. pombe*, and 5 in *S. cerevisiae*. In addition to the highly related JHDM1B found in human and mouse, potential JHDM1A homologs in each of the organisms mentioned above were identified and their domain structures are presented in **Figure 7A**. Although the domain structures are not completely conserved among these proteins, their JmjC domains are highly conserved (**Figure 7B**). Importantly, alignment of their JmjC domains with that of FIH revealed strict conservation of the amino acids, marked by * and #, that are involved in Fe(II) and α-KG-binding, respectively (Elkins, et al. (2003) J. Biol. Chem. 278:1802-1806). This indicates that these proteins are likely to be active demethylases. To demonstrate this, we cloned JHDM1B and showed its H3-K36 demethylase activity (data not shown). Then, we amplified the open reading frame (ORF) of YER051W that encodes the *S. cerevisiae* homolog scJHDM1 and expressed it in *E. coli* as a GST-fusion protein. Recombinant scJHDM1 specifically demethylated methyl-K36, but not methyl-K4 or methyl-K79 (**Figure 7C**). The detected demethylase activity was dependent upon the JmjC domain, as a point mutation in the JmjC domain (H305A) completely abolished enzymatic activity (**Figure 7D**). Collectively, these results demonstrate a critical role for the JmjC domain in histone demethylation and that this function is conserved from human to yeast.

*A Link Between H3-K36 Demethylase Activity and Epe1 Function.* Having demonstrated the functional conservation of the JmjC domain, we sought to establish a link between the known function of the JHDM1 family proteins and their demethylase activity. A search of the literature indicated that of the JHDM1 family members shown in **Figure 7A**, only the *S. pombe* homolog Epe1 has been functionally characterized (Ayoub, et al. (2003) Mol. Cell. Biol. 23:4356-4370). Epe1 was identified in a genetic screen for mutations that promote spreading of silencing outside a heterochromatin barrier (Ayoub, et al. (2003) Mol. Cell. Biol. 23:4356-4370). Epe1 mutation also suppresses mutations in histone deacetylases *clr3*, *clr6* and stabilizes the repressed epigenetic state at the *mat* locus. Over expression of Epe1 disrupts heterochromatin structure and impairs centromere function. Normal function of Epe1 requires an intact JmjC domain, as a JmjC domain mutant Y307A failed to complement a loss-of-function epe1 mutant. Interestingly, a tyrosine corresponding to Y307 in Epe1 is present in all the JHDM1 family proteins (**Figure 7B**, marked by $) indicating involvement of this residue in a conserved function. To determine whether this residue is important for H3-K36 demethylase activity, we generated a mutation (Y315A) in scJHDM1 that mimics the Epe1 Y307A mutation. This mutation significantly reduced the H3-K36 demethylase activity (**Figure 7D**). This result indicates that *epe1* phenotypes likely result from impaired H3-K36 demethylation capability. Therefore, one of the major functions of H3-K36 demethylation is to maintain heterochromatin stability.

### Example 3

### METHODS FOR THE CHARACTERIZATION OF JHDM2 PROTEINS

*Histone Demethylase Assay.* The histone demethylase assay was performed as described in Example 1.

*Purification of the Native and Recombinant JHDM2A.* The procedure for conventional purification of JHDM2A is outlined in **Scheme 5**.

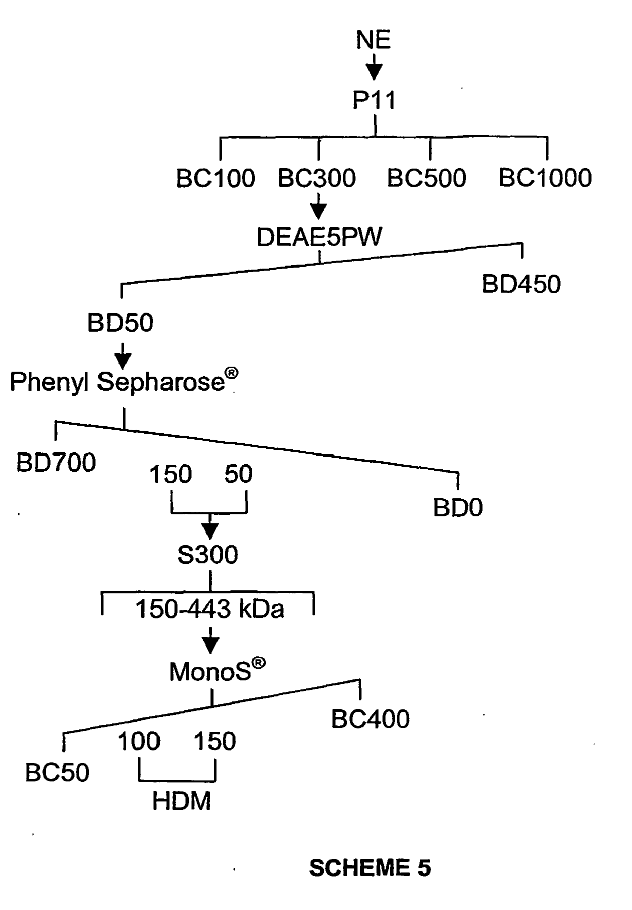

Preparation and fractionation of HeLa cell nuclear extracts on a P11 phosphocellulose column was carried out according to established methods (Wang, et al. (2003) Mol. Cell 12:475-487). The P11 fraction eluted with BC300 was dialyzed into buffer D (40 mM HEPES-KOH pH 7.9, 0.2 mM EDTA, 1 mM DTT, 0.2 mM PMSF, and 10% glycerol) containing 50 mM ammonium sulfate (BD50) and loaded to a 45 mL DE5PW column (TosoHaas). The bound proteins were eluted with a 12-cv linear gradient from BD50 to BD450. The flow-through containing the HDM activity was adjusted to 700 mM ammonium sulfate before it was loaded onto a 22 mL Phenyl Sepharose^{®} column (Pharmacia). The bound proteins were eluted with a 10-cv linear gradient from BD700 to BD0. The active fractions, which eluted from BD150-BD50, were combined and concentrated to 5 mL before they were loaded onto a 120 mL Sephacyl^{®} S300 gel filtration column (Pharmacia). The active fractions, which eluted around 300 kDa, were pooled and loaded onto a 1 mL MonoS^{®} column (Pharmacia) equilibrated with BC50. Bound proteins were eluted with a 20-cv linear gradient from BC50 to BC400. The active fractions eluted from BC100 to BC150. The proteins in the active fractions were pooled and resolved in a 6.5-12% gradient SDS-PAGE. After Coomassie^{®} staining, candidate polypeptides were excised for protein identification.

Generation of baculovirus expressing Flag^{®}-JHDM2A and purification of Flag^{®}-JHDM2A from infected Sf9 cells were performed according to well-known methods (Cao and Zhang (2004) Mol. Cell 15:57-67). Purification of wild-type and deletion mutant Flag^{®}-JHDM2A from COS-7 cells was as described for mutant Flag^{®}-JHDM1A proteins (see Example 1). Similarly, protein identification and mass spectrometry was carried out according to the methods set forth in Example 1.

*Constructs and Antibodies.* Plasmids encoding GST-SET7, GST-hDOT1 L (1-416), components of the EZH2 complex, GST-G9a (621-1000), and CBP-Set2-Flag^{®} were as described in Example 1. Plasmid encoding GST-SET8 has been described previously (Cao and Zhang (2004) Mol. Cell. 15:57-67). A plasmid encoding hJHDM2A was constructed by PCR amplification from a human KIAA clone (KIAA 0742), and was inserted into *Xhol* sites of an N-terminal Flag^{®}-tagged pcDNA3 vector or an N-terminal Flag^{®}-tagged pFASTBAC™ vector. pcDNA3-Flag^{®}-JHDM2A (H1120Y), and deletion mutants (489-1321 aa), (766-1321 aa), (1-1009 aa) were generated by PCR. All the constructs generated through PCR were verified by sequence analysis. RNAi constructs were made by synthesizing oligonucleotides encoding 19 bp short-hairpin RNA that targeted *mJhdm2a* (RNAi1: 5'-GTA CAA GAA GCA GTA ATA A-3', **SEQ ID NO:3**; RNAi2: 5'-AGG TGT CAC TAG CCT TAA T-3', **SEQ ID NO:4**) and cloned into pMSCVneo retrovirus vector (Clonetech™, Palo Alto, CA) under the regulation of H1 RNA promoter as described in the art (Okada, et al. (2005) Cell 121:167-178).

The sources of the antibodies used are as follows: H3 trimethyl-K4 (Abcam), H3 monomethyl-K9 (Abcam), H3 dimethyl-K9 (Upstate Biotechnology, Lake Placid, NY), and H3 dimethy-K27 (Upstate Biotechnology). H3 trimethyl-K9 antibody is known in the art (Plath, et al. (2003) Science 300:131-135).The antibody against Flag^{®} and secondary antibodies for immunofluorescence were as described in Example 1. Antibodies against hJHDM2A were generated in rabbits using the first 495 amino acids of the protein as antigen.

*Immunostaining.* COS-7 cells were plated onto glass coverslips in a 12-well plate and cultured for 1 day. Cells were transiently transfected with plasmids by FuGENE™ 6. Two days after transfection, cells were washed with PBS and fixed in 4% paraformaldehyde for 10 minutes. The cells were then washed three times with cold PBS and permeabilized for 5 minutes with cold PBS containing 0.2% Triton^{®} X-100. Permeabilized cells were then washed three times with blocking buffer (1% bovine serum albumin in PBS) and blocked for 30 minutes before incubation with primary antibodies for 1 hour in a humidified chamber. After three consecutive 5-minute washes with PBS, cells were incubated with secondary antibodies for 1 hour before being washed with PBS and stained with 4,6-diamidino-2-phenylindole dihydrochloride (DAPI) in PBS. Cells were washed again twice with PBS and then mounted in fluorescent mounting medium (Dako) before viewing under an immunofluorescence microscope.

*Generation of a Stable JHDM2A Knockdown Cell Line.* F9 cells were cultured in DMEM media supplied with 10% FBS on 0.1% gelatin-coated plates. The MSCVneo-JHDM2A siRNA vector was cotransfected with pGag-pol and pVSVG into 293T cells by calcium phosphate-mediated transfection. At 48 to 72 hours post-transfection, the supernatants were collected and were used for transduction of F9 cells by spinoculation. Stable transfectants were selected in the presence of 500 µg/mL G418 (Gibco-BRL^{®}, Gaithersburg, MD). Cells derived from these transfectants were used for western blot, real-time PCR, and CHIP analyses.

*Real-time PCR and ChIP Assays.* Real-time PCR was performed in triplicate using SYBR^{®} Green PCR Master Mix (Applied Biosystems™) and the ABI Prism^{®} 7900 sequence detection system (Applied Biosystems™). Quantitative PCR reactions were performed under conditions standardized for each primer. Standard curves were generated using 10-fold dilutions of standard plasmids. To compare the relative amount of target in different samples, all values were normalized to the appropriately quantified 36B4 control. The primers used in quantitative PCR were as follows: mJhdm2a-F, 5'-TGA GTA CAC CAG GCG AGA TG-3' (**SEQ ID NO:5**) and mJhdm2a-R, 5'-GGT CCC ATA TTT CCG ATC CT-3' (**SEQ ID NO:6**); 36B4-F, 5'-CTG ATG GGC AAG AAA ACC AT-3' (**SEQ ID NO:7**) and 36B4-R, 5'-GTG AGG TCC TCC TTG GTG AA-3' (**SEQ ID NO:8**); Nanog-F, 5'-AAG CAG AAG ATG CGG ACT GT-3' (**SEQ ID NO:9**) and Nanog-R and 5'-ATC TGC TGG AGG CTG AGG TA-3' (**SEQ ID NO:10**); Oct4-F, 5'-CCA ATC AGC TTG GGC TAG AG-3' (**SEQ ID NO:11**) and Oct4-R, 5'-CCT GGG AAA GGT GTC CTG TA-3' (**SEQ ID NO:12**); Sox2-F, 5'-GAA CGC CTT CAT GGT ATG GT-3' (**SEQ ID NO:13**) and Sox2-R, 5'-TTG CTG ATC TCC GAG TTG TG-3' (**SEQ ID NO:14**); LamininB1-F, 5'-GTT CGA GGG AAC TGC TTC TG-3' (**SEQ ID NO:15**) and LamininB1-R, 5'-GTT CAG GCC TTT GGT GTT GT-3' (**SEQ ID NO:16**); Hoxa1-F,5'-GCC CTG GCC ACG TAT AAT AA-3' (**SEQ ID NO:17**) and Hoxa1-R, 5'-TCC AAC TTT CCC TGT TTT GG-3' (**SEQ ID NO:18**); Stra6-F, 5'-GTT CAG GTC TGG CAG AAA GC-3' (**SEQ ID NO:19**), Stra6-R, 5'-CAG GAA TCC AAG ACC CAG AA-3' (**SEQ ID NO:20**).

For ChIP assays, 90% confluent F9 cells in 150-mm dishes were treated with DMEM containing 1% formaldehyde for 10 minutes. Cross-linking was stopped by the addition of 0.125 M glycine for 5 minutes. After washing twice with PBS, the cells were resuspended in 1 mL of cell lysis buffer (10 mM HEPES [pH 7.9], 0.5% NP-40, 1.5 mM MgCl₂, 10 mM KCI, 0.5 mM DTT) by pipetting and kept on ice for 10 minutes. After centrifugation at 4,000 rpm for 5 minutes, the cell pellets were resuspended in nuclear lysis buffer (20 mM HEPES [pH 7.9], 25% glycerol, 0.5% NP-40, 0.42 M NaCl, 1.5 mM , 0.2 mM EDTA) containing protease inhibitors to extract nuclear proteins at 4°C for 20 minutes. Chromatin was sonicated into fragments with an average length of 1 kb. After centrifugation at 13,000 rpm for 10 minutes, the supernatants were diluted in an equal volume of dilution buffer containing 1% Triton^{®} X-100, 2 mM EDTA, 20 mM Tris-HCl [pH 7.9], 50 mM NaCl, and protease inhibitors. ChIP assays were then performed with anti-JHDM2A, anti-dimethyl-K9, and anti-trimethyl-K4 antibodies. For all ChIP experiments, quantitative PCR analyses were performed in real-time using the ABI Prism^{®} 7900 sequence detection system and SYBR^{®} Green master mix. Quantity of DNA was determined following the algebraic formula of 2^{-Ct} (where Ct is the cycle threshold number). The relative amount of immunoprecipitated DNA to input DNA was calculated. Primer pairs were as follows: LamininB1-F, 5'-CTT TTC TCC CCG CTA CCT CT-3' (**SEQ ID NO:21**) and LamininB1-R, 5'-CTA GGA CAC CAA AGG CGA AC-3' (**SEQ ID NO:22**); Stra6-F, 5'-TGG AAG AGG AGG GTC TCT GA-3' (**SEQ ID NO:23**) and Stra6-R, 5'-CTC CTG CCA TGG AGT CTC TC-3' (**SEQ ID NO:24**); Hoxa1-F, 5'-ACT GCC AAG GAT GGG GTA TT-3' (**SEQ ID NO:25**) and 5'-CTT CGC AGG ATC CAA TCA CT-3' (**SEQ ID NO:26**).

For ChIP assay in LNCaP cells, the cells were cultured in charcoal-stripped serum medium for three days before treatment with R1881 (50 nM) for 1 hour. The ChIP assay was performed essentially as previously described (Yoon, et al. (2005) Mol. Cell Biol. 25:324-335). Primers for RT-PCR analysis of the PSA mRNA were 5'-GCC CAC CCA GGA GCC AGC ACT-3' (**SEQ ID NO:27**) and 5'-GGC CCC CAG AAT CAC CCG AGC AG-3' (**SEQ ID NO:28**).

*AR-JHDM2A Interaction.* The *in vitro* translated ³⁵S-methionine labeled AR (10 µL) was mixed with 3 µL (300 ng) of purified recombinant JHDM2A in binding butter (20 mM Hepes, pH 7.6, 50 mM KCI, 1 mM DTT, 0.5 mM PMSF and 10% glycerol) in the presence or absence of 100 nM R1881 in a 100 µL reaction. The mixture was rotated in the cold room for 2 hours and Protein-A agarose beads and anti-JHDM2A antibody (10 µL) were added. After a 1 hour incubation, the beads were extensively washed with the binding buffer, the AR was resolved by a 10% SDS-PAGE and visualized by autoradiography.

### Example 4

### JHDM2A FACILITATES TRANSCRIPTIONAL ACTIVATION THROUGH A NUCLEAR HORMONE RECEPTOR

*Purification and Identification of a Histone Demethylase Activity.* A SET2-methylated nucleosomal histone substrate has been used to monitor histone demethylase activity. Thus, in a parallel study, we also used histone substrates methylated by other histone methyltransferases, including the H3-K9 methyltransferase G9a (Tachibana, et al. (2002) Genes Dev. 16:1779-1791). When the G9a-methylated histone substrates were subjected to demethylation assays using the protein fractions derived from HeLa nuclear extracts (NE) and nuclear pellet (NP) (Wang, et al. (2001) Science 293:853-857), we detected a H3-K9 demethylase activity in the nuclear extract derived 0.3 M P11 protein fraction (**Figure 8A**). To ascertain whether this activity was the result of a genuine demethylase, we examined its dependency on the required co-factors Fe(II) and α-KG. Results shown in **Figure 8B** demonstrate that release of formaldehyde not only requires the presence of the protein fraction (compare lanes 1 and 5), but also the co-factors Fe(II) and α-KG (compare lanes 2 and 3 with lane 1). Addition of ascorbate, which is involved in regenerating Fe(II) from Fe(III), appears to stimulate formaldehyde production (compare lanes 1 and 4). These results indicate that a histone demethylase activity is present in the nuclear extract derived 0.3 M P11 fraction and the candidate enzyme likely uses the same oxidative demethylation mechanism used by JHDM1A for histone demethylation (Example 2).

To identify the protein(s) responsible for this demethylase activity, we monitored the enzymatic activity through five chromatography columns (**Scheme 5**). After purification of the 0.3 M P11 fraction through DEAE5PW and Phenyl Sepharose^{®} columns, we determined that the native mass of the enzymatic activity was about 300 kDa as assessed by a Sephacyl^{®} S300 column (**Figure 9A**). Further purification on a MonoS^{®} column allowed us to correlate the enzymatic activity (**Figure 9B**, bottom panel) to a protein of about 150 kDa, marked by * (**Figure 9B**, top panel). Because there was a dramatic difference in the enzymatic activities between fractions 17 and 20 (**Figure 9B**), we concentrated these two fractions and compared their protein composition by SDS-PAGE (**Figure 9C**). After recovering protein bands unique to fraction 20, they were subjected to mass spectrometry analysis which revealed the protein, marked by *, to be a novel JmjC domain-containing protein named JMJD1A (jumonji domain containing 1A) or TSGA (testis-specific gene A) (Hoog, et al. (1991) Mol. Reprod. Dev. 30:173-181) (**Figure 9C**). To verify that JMJD1A was responsible for the detected demethylase activity, an antibody against this protein was generated and used to immunoprecipitate JMJD1A from the MonoS^{®} column fractions 21-29. The immunoprecipitated sample was divided for silver staining, western blot, and demethylase assay analyses. Results shown in **Figure 9D** demonstrate that the demethylase activity (bottom panel) was completely depleted from the supernatant (S), which correlated with depletion of the JMJD1A (middle panel). Importantly, silver staining (top panel) revealed a single protein corresponding to JMJD1A. These results collectively indicate that JMJD1A is responsible for the detected demethylase activity. Given the size is about 300 kDa (**Figure 9A**), JMJD1A appears to function as a homodimer. Because histone demethylase activity is the first identified activity for the protein and it is the second JmjC domain-containing histone demethylase that we have identified, we have renamed the protein JHDM2A (JmjC domain-containing histone demethylase 2A) to reflect its newly identified function. Accordingly, we have named the other two JHDM2A-related human proteins JHDM2B and JHDM2C, respectively. (**Figure 10C**).

JHDM2A was first identified in a testis cDNA library (Hoog, et al. (1991) Mol. Reprod. Dev. 30:173-181). *In situ* hybridization studies indicated that JHDM2A is mainly expressed in male germ cells and its steady-state transcript levels are the highest during the meiotic and the post-meiotic stages of germ cell development (Hoog, et al. (1991) Mol. Reprod. Dev. 30:173-181). Domain structure analysis using the SMART program revealed the presence of a JmjC domain and a zinc-finger (**Figure 10A**). Given the demonstration that JmjC domain is a signature motif for histone demethylases (Example 2), the presence of a JmjC domain in JHDM2A indicates that JHDM2A is responsible for the detected histone demethylase activity. To directly demonstrate the demethylase activity of JHDM2A, we transfected COS-7 cells with a Flag^{®}- tagged mammalian expression vector. After immunoprecipitation with anti-Flag^{®}-conjugated beads, half of the immunoprecipitate was used for western blot analysis and half for enzymatic activity assays. Results shown in **Figure 10B** (lanes 1) revealed a robust histone demethylase activity dependent on the Flag^{®}-JHDM2A protein.

To evaluate the importance of the JmjC and the zinc-figure domains for the enzymatic activity of JHDM2A, we generated three expression constructs with deletions of the N-terminus, zinc-finger, and JmjC domain, respectively (**Figure 10A**). After transfection and immunoprecipitation, these mutant proteins were subjected to western blot analysis and demethylase activity assays. Results shown in **Figure 10B** indicate that both the zinc-finger and the JmjC domain are critical for the enzymatic activity (compare lane 1 with lanes 3-5). To further demonstrate the importance of the JmjC domain for enzymatic activity, we generated a single amino acid substitution, H1120Y, in the JmjC domain. We chose to mutate amino acid H1120 because this histidine is highly conserved in the JmjC domain of JHDM2A-related proteins (**Figure 10D**), and the corresponding histidine in FIH [factor-inhibiting HIF (hypoxia-inducible factor)], a known Fe(II) dependent oxygenase, was found to directly bind to Fe(II) (Elkins, et al. (2003) J. Biol. Chem. 278, 1802-1806). Fe(II)-dependency of the histone demethylase activity (**Figure 8B**) indicated that the H1120Y mutation would disrupt Fe(II) binding, and thus impair the enzymatic activity of JHDM2A. Results shown in **Figure 10B** confirmed this (compare lanes 1 and 2). Therefore, it was concluded that JHDM2A is a novel histone demethylase and the JmjC domain is critical for enzymatic activity.

*JHDM2A Demethylates H3 Mono- and Dimethyl-K9 in vitro.* To further characterize JHDM2A, we generated a baculovirus expressing a Flag^{®}-tagged JHDM2A and purified the protein from infected Sf9 cells by affinity chromatography. After evaluating the purity and quantifying the Flag^{®}-JHDM2A protein (**Figure 11A**), we analyzed and compared its enzymatic activity with that of the native JHDM2A protein. Results shown in **Figure 11B** demonstrate that recombinant Flag^{®}-JHDM2A and native JHDM2A had similar activity when equal amounts of protein were compared (compare lane 1 with lanes 4 and 5). To analyze the site-specificity of JHDM2A, histone substrates radiolabeled at all known methyl-lysine sites in histones H3 (K4, K9, K27, K36, K79) and H4 (K20) were subjected to a demethylation assay containing purified recombinant Flag^{®}-JHDM2A. Of the six substrates, only H3-K9 methylated by G9a was a substrate for JHDM2A (**Figure 11C**). In addition to H3-K9, previous studies indicated that G9a can also methylate H3-K27 *in vitro* (Tachibana, et al. (2002) Genes Dev. 16:1779-1791). To verify that JHDM2A specifically demethylates H3-K9, but not H3-K27, we generated radiolabeled substrate histone H3 that contains either a K9R or a K27R mutation using the G9a HMT. In parallel, we also included radiolabeled wild-type recombinant H3 and HeLa core histones in the assay. Results shown in **Figure 11D** demonstrate that JHDM2A was not able to demethylate H3 when K9 was mutated (compare lanes 5 and 6), while the K27 mutation did not affect the activity of JHDM2A (compare lanes 7 and 8). Thus it was concluded that JHDM2A is an H3-K9-specific demethylase.

Lysine residues exist in three methylation states (mono-, di-, and tri-methylation). To determine whether JHDM2A preferentially demethylates a particular methylation state, we performed a demethylation assay using H3-K9-methylated peptide substrates and analyzed demethylation products by mass spectrometry. Results shown in **Figure 11E** demonstrate that demethylation of a dimethyl-K9 peptide depends on the presence of JHDM2A and generated products with masses that correlate with both mono-methyl and unmethylated forms of the peptide, indicating that both mono- and dimethyl-K9 can serve as substrates. In contrast, no demethylation was detected when a trimethyl-K9 peptide, that has the same sequence, was subjected to parallel analysis (**Figure 11F**). Based on the above results, it was concluded that JHDM2A selectively demethylates H3-mono- and dimethyl-K9.

*JHDM2A Demethylates H3 Mono- and Dimethyl-K9 in vivo.* Having demonstrated demethylase activity for JHDM2A *in vitro,* we sought to test its activity *in vivo*. To this end, we investigated the effect of over-expression JHDM2A on the H3-K9 methylation levels by immunostaining. Over-expression of JHDM2A was found to greatly reduce the H3-dimethyl-K9 level (**Figure 12A**). This effect was not simply due to inaccessibility of modification specific antibody due to the presence of Flag^{®}-JHDM2A, as over-expression of an enzymatically defective mutant did not affect dimethyl-K9 levels (**Figure 12B**). A decrease in the level of monomethyl-K9 was observed in cells that over-expressed JHDM2A (**Figure 12C**). However, no effect was observed on the levels of trimethyl-K9 (**Figure 12D**) or dimethyl-K27. In addition, overexpression of JHDM2A does not alter the expression level of several known H3K9 methyltransferases (data not shown). These results indicate that JHDM2A demethylates H3 mono- and dimethyl-K9, with a preference for dimethyl-K9, *in vivo*.

*JHDM2A Knockdown Leads to Decreased Transcription Concomitant with Increased Promoter H3-K9 Dimethylation* Having demonstrated the enzymatic activity of JHDM2A *in vitro* and *in vivo*, we attempted to address whether JHDM2A has a role in transcriptional regulation. Studies have linked H3-K9 methylation to transcriptional repression and heterochromatin formation (Martin and Zhang (2005) Nat. Rev. Mol. Cell. Biol. 6:838-849). Therefore, a H3-K9 demethylase could potentially antagonize gene silencing. To test this, we generated stable *Jhdm2a* knockdown cells using a vector-mediated RNAi approach (Okada, et al. (2005) Cell 121:167-178). We chose to perform the knockdown in F9 cells because this cell line exhibits the highest *Jhdm2a* expression of the three cell lines that we analyzed (**Figure 13A**). Quantitative PCR (**Fig. 13B**, top two panels) and western blot (**Figure 13B**, bottom two panels) analyses confirmed that the knockdown efficiency was about 80% at the RNA level and about 70% at the protein level. Compared to the parental F9 cells, no apparent morphological changes were observed in the knockdown cells (data not shown), indicating either that *Jhdm2a* does not play a critical role in maintaining the undifferentiated state of F9 cells, or that the highly related *Jhdm26* which is also expressed in F9 cells (data not shown) may compensate for *Jhdm2a* function. Although no apparent cell differentiation was observed in response to *Jhdm2a* knockdown, we have nevertheless analyzed changes in expression levels of a few genes in response to *Jhdm2a* knockdown. The genes that we analyzed included the pluripotency marks (Nanog, Oct4 and Sox2) and differentiation marks (Lamb1, Hoxa1, Hoxb1, and Stra6). Results shown in **Figure 13C** indicate that, in response to *Jhdm2a* knockdown, Nanog and Oct4 were down-regulated about 20%, while the differentiation marks Lamb1, Hoxb1, and Stra6 were down-regulated about 40-60%. These results are consistent with *Jhdm2a* functioning as a H3-K9. demethylase to positively regulate transcription. In contrast, Hoxa1 expression was up-regulated by *Jhdm2a* knockdown perhaps due to indirect effects.

To investigate whether the observed transcriptional effects due to *Jhdm2a* knockdown represent a direct effect, we analyzed binding of the JHDM2A protein to the LamB1 and Stra6 promoters by ChIP assay. As a control, we also analyzed its binding to the Hoxa1 gene promoter. Results shown in **Figure 13D** indicate that JHDM2A binds to the LamB1 and Stra6 promoters, but not the Hoxa1 promoter (compare columns 1 and 2 with 3). Knockdown of *Jhdm2a* significantly decreased JHDM2A binding indicating the detected CHIP signals were specific (columns 1 and 2). Consistent with the observation that JHDM2A functions as a dimethyl-K9 demethylase, knockdown *Jhdm2a* increased the dimethyl-K9 levels at the LamB1 and Stra6 promoters, but had little effect on the Hoxa1 promoter (columns 4-6). Consistent with the fact that *Jhdm2a* is a dimethyl-K9-specific demethylase, knockdown *Jhdm2a* did not cause a significant alteration in the trimethyl-K4 levels on any of the three promoters analyzed (columns 7-9). From these results it was concluded that JHDM2A is targeted to a subset of genes to demethylate dimethyl-K9 which in turn positively regulate their gene expression.

*Hormone-Dependent Recruitment of JHDM2A Correlates with H3-K9 Demethylation and Transcriptional Activation.* JHDM2A has two closely related homologs JHDM2B and JHDM2C (**Figure 10C**). Interestingly, JHDM2C was first identified in a yeast two-hybrid screen as a thyroid hormone receptor (TR) interacting protein named TRIP8 (Lee, et al. (1995) Mol. Endocrinol. 9:243-254). In addition, JHDM2A contains a ⁸⁸⁵LXXLL⁸⁸⁹ (**SEQ ID NO:29**) sequence, which is a signature motif involved in nuclear hormone receptor interaction (Heery, et al. (1997) Nature 387:733-736). Thus, the involvement of JHDM2A in transcriptional regulation by nuclear receptors was determined. While a hormone-dependent interaction with TR was not detected by either *in vitro* pull-down or co-immunoprecipitation assays (data not shown), we found that JHDM2A interacted with androgen receptor (AR) in a ligand-dependent manner (**Figure 14A**). To investigate the *in vivo* relevance of this *in vitro* interaction, we asked whether JHDM2A is recruited to known AR target genes in a hormone-dependent manner. Thus, we performed ChIP assays on two well-characterized AR target genes, prostate-specific antigen (PSA) and NKX3.1, in LNCaP cells in the presence or absence of hormone. Results shown in **Figure 14B** demonstrate a strong binding of the AR to the PSA enhancer upon R1881 treatment (third panel, compare lanes 1 and 2). Similarly, strong hormone-dependent binding of the AR to a region containing a function ARE (AR enhancer) located at ~3 kb upstream of the NKX3.1 transcriptional start site was also detected (third panel, compare lanes 3 and 4). Consistent with hormone-induced transcriptional activation (data not shown), an increase in histone acetylation was detected in both cases (4^{th} panel). Importantly, R1881 treatment also led to increased association of JHDM2A with AR target genes (**Figure 14B**, 5^{th} panel), concomitant with decreases in the level of dimethyl- and trimethyl-K9 (**Figure 17B**, 6^{th} and 7^{th} panels). In contrast to JHDM2A, which binds to PSA and NKX3.1 genes in a hormone-dependent manner, association of LSD1 with these two genes was not affected by hormone treatment (**Figure 14B**, last panel), consistent with the teachings in the art (Metzger, et al. (2005) Nature 437:436-439).

To directly test and compare the role of JHDM2A and LSD1 in hormone-dependent transcriptional activation, we used siRNA to knockdown JHDM2A and LSD1 in LNCaP cells (**Figure 14C**). We then evaluated the effects of JHDM2A or LSD1 knockdown on the hormone induced activation of three AR target genes, PSA, NKX3.1 and TMPRSS22, by quantitative RT-PCR. Results shown in **Figure 14D** demonstrate that while treatment with R1881 for 8 hours robustly activated transcription from all three AR target genes, knockdown of JHDM2A led to significant reduction in hormone response for all three AR target genes. Although knockdown of LSD1 also led to reduced hormone response, the effect was not as significant when compared to knockdown of JHDM2A. It was therefore concluded that JHDM2A is critically important for optimal hormone-dependent transcriptional activation by AR.

Having established a role for JHDM2A in hormone-dependent activation by AR, we next examined whether JHDM2A is important for the hormone-induced H3K9 demethylation observed in **Figure 14B**. For this purpose, LNCaP cells were first treated with JHDM2A siRNA, followed by treatment with R1881 for 1 hour before ChIP analysis. Results shown in **Figure 14E** demonstrate that the hormone-induced recruitment of JHDM2A to the PSA enhancer and NKX3.1 gene was largely abrogated upon siJHDM2A treatment (second panel, compare lane 3 with lanes 2 and 4; compare lane 7 with lanes 6 and 8). Importantly, knockdown of JHDM2A significantly impaired the hormone-induced reduction of dimethyl-H3K9 at the PSA enhancer (4^{th} panel, compare lanes 2 and 3). Although to a lesser extent, knockdown of JHDM2A also affected the hormone-induced reduction of dimethyl-H3K9 at NKX3.1 (4^{th} panel, compare lanes 6 and 7). These results indicate that JHDM2A is required for efficient demethylation of repressive dimethyl-H3K9 at AR target genes. Interestingly, although JHDM2A had no activity toward trimethyl-H3K9 (**Figures 11F**, **12D**, **13D**), knockdown of JHDM2A also affected hormone-induced reduction of trimethyl-H3K9 for both PSA enhancer and NKX3.1 (**Figure 14E**, last panel). It was of interest that knockdown of JHDM2A also appeared to reduce the hormone-induced H3 acetylation levels (**Figure 14E**, third panel, compare lanes 2 and 3, 6 and 7). One explanation for this observation is that H3K9 demethylation is a prerequisite for H3K9 acetylation. On the other hand, knocking down JHDM2A did not affect hormone-induced binding of AR to both target genes (**Figure 14E**, first panel), indicating that JHDM2A contributes to AR-mediated transcription activation subsequent to its binding to target genes.

### Example 5

### METHODS FOR THE CHARACTERIZATION OF JHDM3 PROTEINS

*Constructs and Recombinant Protein.* JHDM3A was PCR-amplified from EST clone (IMAGE3138875) and cloned into the *Bam*HI and *Not*I sites of modified FastbacHTb™ (Invitrogen™, Carlsbad, CA) and pCDNA3 (Invitrogen™) vectors engineered to contain an N-terminal Flag^{®}-tag. The H197A substitution mutation was generated by site-directed mutagenesis using the QuikChange^{®} mutagenesis kit (Stratagene^{®}, La Jolla, CA). The deletion constructs were generated using established methods (Zhang, et al. (2005) Mol. Cell. Biol. 25:6404-14). In all cases, the sequences of PCR-amplified clones were confirmed by sequence analysis. Generation of baculoviruses that express Flag^{®}-JHDM3A and purification of the recombinant protein from infected SF9 cells were performed as described in Example 3.

*Demethylation Assay and Mass Spectrometry.* All histone substrates were radioactively labeled as described in Example 1. Likewise, histone demethylation assays and mass spectrometry were performed as described in Example 1. Peptide substrates used in the assay encompass amino acids 1-18 of histone H3 and contained either a di-methyl or tri-methyl modification on lysine 9.

*Immunofluorescence Microscopy.* NIH 3T3 cells were grown in DMEM containing 10% FBS and penicillin/streptomycin. Cells grown on coverslips in 6-well plates were transfected with 2 µg of Flag^{®}-JHDM3A expression plasmid using FuGENE™ 6 transfection reagent (Roche). In experiments using GFP-HP1β, 250 ng of expression vector was included in the transfection. Cells were fixed 24 hours post-transfection for 20 minutes in 4% paraformaldehyde, washed 3 times with PBS, and subsequently permeabilized for 20 minutes in 0.5% Triton® X-100/PBS. Permeablized cells were washed 2 times in PBS and blocked in 3% BSA/PBS for 30 minutes. Cells were incubated with primary antibody in a humidified chamber for 1-3 hours using histone modification antibodies [tri-methyl H3K9 (Plath, et al. (2003) Science 300:131-5), di-methyl H3K9 (Upstate Biotechnology), mono-methyl H3K9 (Abcam), and tri-methyl H3K27 (Plath, et al. (2003) Science 300:131-5)] at a dilution of 1:100 and the Flag^{®} monoclonal M2 antibody (Sigma^{®}) at a dilution of 1:1000. After primary antibody incubation, cells were washed 3 times and incubated with FITC- or Rhodamine-conjugated secondary antibodies (Jackson ImmunoResearch Laboratories). Cells were washed twice with PBS, stained with 4,6-diamidino-2-phenylindole dihydrochloride (DAPI) and mounted on glass slides in fluorescent mounting medium (Dako). Slides were analyzed on a fluorescent microscope.

*JHDM3A siRNA, RT-PCR Analysis, and ChIP.* siRNA-mediated JHDM3A knock-down, RT-PCR analysis of ASCL2, and ChIP analysis were carried out as described in Example 3.

### Example 6

### JHDM3 DIRECTLY REVERSES HISTONE H3 LYSINE 9 TRI-METHYLATION AND ANTAGONIZES TRI-METHYL-LYSINE 9-MEDIATED HP1 RECRUITMENT

To identify additional histone lysine demethylases, we compared the JmjC domains of other JmjC family members to the known histone demethylases, JHDM1A/B and JHDM2A (Examples 2 and 4, respectively), focusing on similarities in the proposed Fe(II) and α-KG binding sites. A related protein hydroxylase, FIH (factor-inhibiting hypoxia-inducible factor), was included in the alignment because the structure of FIH in complex with Fe(II) and α-KG is available and thus can serve as a reference point (Elkins, et al. (2003) J. Biol. Chem. 278:1802-6). Of the JmjC domain-containing proteins analyzed, the JMJD2 family of proteins (Katoh & Katoh (2004). Int. J. Oncol. 24:1623-8) were excellent candidates for demethylase activity because amino acids predicated to be involved in Fe(II) and α-KG binding are conserved (**Figures 15A** and **15B**). Since JMJD2A is the only JMJD2 family member characterized (Gray, et al. (2005) J. Biol. Chem. 280:28507-18 ; Zhang, et al. (2005) Mol. Cell. Biol. 25:6404-14), we have focused our efforts in defining whether this protein is an active histone demethylase.

To determine whether JMJD2A is an active histone demethylase, we produced recombinant Flag^{®}-tagged JMJD2A in insect cells using a baculovirus expression system and purified the recombinant protein to homogeneity (**Figure 15C**). Demethylation analysis using different histone substrates containing radioactively labeled methyl groups corresponding to multiple characterized methyl-lysine sites on histone H3 and H4 indicated that only Dim5-labeled histone substrates resulted in release of labeled formaldehyde when incubated with purified recombinant JMJD2A (**Figure 15D**). This indicated that JMJD2A has demethylase activity toward methylated histone H3K9. To verify that JMJD2A utilizes an oxidative demethylation mechanism with Fe(II) and α-KG as co-factors, each co-factor was independently omitted from the demethylation reaction. Full enzymatic activity was only observed when the complete co-factor/enzyme complement was present in the reaction (**Figure 15E**). Together, these data indicate that recombinant JMJD2A catalyzes H3K9 demethylation via an oxidative demethylation reaction using Fe(II) and α-KG as co-factors. Having demonstrated that JMJD2A is an active histone demethylase, we have renamed the protein JmjC domain-containing histone demethylase 3A (JHDM3A) to reflect its enzymatic function and conform to the existing naming convention.

Given that the fungal H3K9 methyltransferase Dim5 can produce mono, di, and trimethylated histone H3K9 substrates *in vitro* (Tamaru, et al. (2003) Nat. Genet. 34:75-9), we sought to determine the modification state-specificity of the JHDM3A. To this end, Flag^{®}-tagged JHDM3A was expressed in NIH3T3 cells. The effect of JHDM3A over-expression on H3K9 methylation levels was analyzed by indirect immuno-florescence staining. In agreement with previous observations (Tamaru, et al. (2003) Nat. Genet. 34:75-9), Flag^{®}-tagged JHDM3A protein exhibited diffuse nuclear staining (**Figure16**, left panels). Interestingly, cells expressing JHDM3A displayed a near complete loss of trimethyl H3K9 at pericentric heterochromatin and throughout euchromatic regions of the nucleus (**Figure 16A**, upper panel). The observed loss of trimethyl-K9 was dependent upon an intact JmjC domain as a mutation (H197A) in the predicted Fe(II) binding site abrogated the effect (**Figure 16A**, bottom panel). The observed demethylation was also trimethyl H3K9-specific as no effects were observed on other histone modification states including: di-methyl K9, mono-methyl K9, or trimethyl K27 (**Figure 16B**). Thus, it was concluded that JHDM3A preferentially demethylates trimethyl-H3K9 *in vivo.*

Next, we sought to determine whether this substrate-specificity was intrinsic to JHDM3A. Thus, we incubated recombinant JHDM3A with peptides containing either di-methyl or trimethyl modifications at the H3K9 position. Following the demethylation reaction, peptide substrates were analyzed by mass spectrometry. Consistent with JHDM3A acting as a trimethyl-K9-specific demethylase, the trimethyl-K9 peptide was converted to a dimethyl-K9 peptide (**Figure 16C**), indicating loss of a single methyl group. The fact that no mono- or unmodified peptides were detected indicates that JHDM3A specifically demethylates trimethyl K9 to the di-methyl state. Under the same assay condition, no demethylation was observed on a di-methyl H3K9 peptide (**Figure 16D**) which is efficiently demethylated by the related H3K9 demethylase, JHDM2A (Example 4). Therefore, JHDM3A displays remarkable enzymatic specificity toward trimethyl H3K9 both *in vitro* and *in vivo*, resulting in the removal of one methyl group and leaving di-methyl H3K9.

Having verified the JHDM3A is a trimethyl-K9 specific demethylase, we determined the domain requirements for demethylase activity *in vivo*. We generated mutants of JHDM3A each harboring deletion of a single predicted functional domain (**Figure 17A**). Transfection of these mutant expression vectors into NIH 3T3 cells followed by trimethyl-K9 staining revealed that only the JmjC domain is required for demethylation activity (**Figures 17B-17G**). This indicates that other domains of JHDM3A are likely involved in targeting and protein-protein interaction function.

In mouse cells, Suv39H1 is the major histone methyltransferase responsible for H3K9 trimethylation at pericentric heterochromatin (Peters, et al. (2003) Mol. Cell 12:1577-89; Rice, et al. (2003) Mol. Cell 12:1591-8). HP1 (heterochromatin protein 1) preferentially binds to trimethylated H3K9 *in vitro* (Jacobs & Khorasanizadeh (2002) Science 295:2080-3; Bannister, et al. (2001) Nature 410:120-4; Lachner, et al. (2001) Nature 410:116-20) and localizes to pericentric heterochromatin in a Suv39H1-dependent manner (Peters, et al. (2003) Mol. Cell 12:1577-89; Rice, et al. (2003) Mol. Cell 12:1591-8). Given the ability of JHDM3A to actively demethylate trimethyl-H3K9, we determined whether JHDM3A levels may influence HP1 localization pattern through modulating H3K9 trimethylation levels. Consistent with this, the pericentric HP1 was redistributed throughout the nucleus when JHDM3A was co-expressed with HP1 (compare **Figure 18A** and **18B**). The ability of JHDM3A to cause HP1 redistribution was dependent upon its H3K9 demethylase activity, as co-expression of the JHDM3A(H197A) mutant failed to cause HP1 redistribution (**Figure 18C**). These data indicate that elevated levels of JHDM3A and H3K9 demethylation can function to antagonize HP1 recruitment to pericentic heterochromatin.

In addition to participating in heterochromatin formation (Peters, et al. (2003) Mol. Cell 12:1577-89; Rice, et al. (2003) Mol. Cell 12:1591-8), H3K9 trimethylation has also been linked to transcriptional regulation in euchromatin (Vakoc, et al. (2005) Mol. Cell 19:381-391; Schultz, et al. (2002). Genes Dev. 16:919-932; Wang, et al. (2003) Mol. Cell 12:475-87). To examine whether JHDM3A plays a role in removing trimethyl H3K9 at euchromatin genes, we utilized siRNA-mediated knock-down to manipulate JHDM3A levels and analyzed its effects on the only known JHDM3A target gene ASCL2 (Zhang, et al. (2005) Mol. Cell. Biol. 25:6404-14). Previous studies have indicated that JHDM3A can transiently interact with NCoR co-repressor complex to repress the ASCL2 gene (Zhang, et al. (2005) Mol. Cell. Biol. 25:6404-14). Consistent with JHDM3A functioning as a negative regulator of transcription, siRNA-mediated knock-down resulted in ASCL2 up-regulation (**Figures 19B** and **19C**). ChIP analysis demonstrated that JHDM3A normally binds to the ASCL2 promoter region at 1.1 kb up-stream of the transcription start site (**Figures 19A** and **19D**). After siRNA treatment, JHDM3A occupancy was reduced (**Figures 19D**, third panel). Consistent with the role of JHDM3A functioning as a trimethyl H3K9 demethylase, a significant increase in the levels of trimethyl H3K9 at the ASCL2 gene was observed upon JHDM3A knock-down (**Figures 19D**, 4^{th} panel). In contrast, no significant changes in H3K27 methylation were observed (**Figures 19D**, bottom three panels). Given the documented role of H3K9 trimethylation as a silencing mark (Martin & Zhang (2005) Nat. Rev. Mol. Cell. Biol. 6:838-849), the observation that a trimethyl-K9 demethylase functions in transcriptional repression indicates that JHDM3A may function to antagonize trimethyl-H3K9 which has been linked to transcriptional activation (Vakoc, et al. (2005) Mol. Cell 19:381-391). These data clearly indicate that occupancy of endogenous JHDM3A at specific target gene is required to maintain reduced levels of trimethyl H3K9 in concert with transcriptional repression.

The identification of JHMD3A provides molecular basis for dynamic regulation of trimethyl-H3K9. Although a significant amount of trimethyl H3K9 resides in heterochromatic regions (Peters, et al. (2003) Mol. Cell 12:1577-89; Rice, et al. (2003) Mol. Cell 12:1591-8), this modification also plays a role in silencing genes found in euchromatic regions (Schultz, et al. (2002) Genes Dev. 16:919-932; Sarraf & Stancheva (2004) Mol. Cell 15:595-605). Because JHDM3A preferentially removes trimethyl H3K9 and does not subsequently remove di or mono methyl-K9 modifications, histone demethylases, much like histone methyltransferase enzymes (Wang, et al. (2003) Mol. Cell 12:475-87; Manzur, et al. (2003) Nat. Struct. Biol. 10:187-96; Xiao, et al. (2005) Genes Dev. 19:1444-54), may be specifically tailored to regulate distinct modification states. In this regard, biochemical studies have defined a role for the Tandem Tudor domain of JHDM3A in recognition of methyl-lysine residues in histones H3 and H4. Not wishing to be bound by theory, it is believed that the Tandem Tudor domain of JHDM3A facilitates recruitment of the demethylase activity of JHDM3A to chromatin-containing specific histone modifications.

### Example 7

### METHODS FOR THE CHARACTERIZATION OF THE DEMETHYLASE ACTIVITY OF RBP2

*Constructs and Recombinant Protein.* pcDNA3/HA-Flag^{®}-RBP2 was generated by inserting a Flag^{®}-tag into the *Cla*I site of pcDNA3/HA-RBP2. The H483A substitution mutation was introduced into pCDNA3/HA-Flag^{®}-RBP2 by site-directed mutagenesis as described in Example 5. For production of baculovirus-expressed protein, RBP2 was cloned into the *Sal*I and *Xba*I sites of a modified FastbacHTb™ (Invitrogen™) vector engineered to contain an N-terminal Flag^{®}-tag. Generation of baculovirus that expresses Flag^{®}-RBP2 and purification of the recombinant protein from infected SF9 cells were performed as described in Example 3.

*Antibodies.* The RBP2 antibodies 1416 and 2471 have previously been described (Benevolenskaya, et al. (2005) Mol. Cell 18:623-635). The anti-RBP2 polyclonal antibody 2470 was raised in rabbits against glutathione S-transferase (GST)-RBP2 (1311-1358). Flag^{®} monoclonal M2 antibody and α-tubulin antibody (clone B-5-1-2) were from Sigma^{®}. H3K4me3 antibody, H3K4me1 antibody, H4K20me3 antibody, and H3 antibody were from Abcam^{®}. H3K4me2 antibody, H3K9me2 antibody, and H4R3me2 antibody were from Upstate Biotechnology. In some experiments, H3K4me2 antibody was also used (Feng, et al. (2002) Curr. Biol. 12:1052-1058).

*Purification of the RBP2 complexes from HeLa cells.* HeLa S3 nuclear extract was prepared as described (Dignam, et al. (1983) Nucleic Acids Res. 11:1475-1489). Protein fractions containing RBP2 were identified by western blot analysis using RBP2 antibody. The nuclear extract was brought to a conductivity equivalent to that of Buffer D (40 mM HEPES·NaOH (pH 7.9), 0.5 mM EDTA, 1 mM DTT, 0.5 mM AEBSF and 10% (v/v) glycerol) containing 100 mM KCI, and loaded to a 20 ml HiPrep^{®} 16/10 SP FF column (Amersham), the bound proteins were eluted with a 10-cv linear gradient from 100 mM to 500 mM KCI in Buffer D. The fractions containing RBP2, which eluted from 350 mM to 410 mM KCI (complex 1) and from 280 mM to 310 mM KCI (complex 2), were pooled separately and brought to a conductivity equivalent to that of Buffer H (20 mM Na₃PO₄ (pH 6.8), 0.5 mM EDTA, 1 mM DTT, and 10% (v/v) glycerol) containing 300 mM NaCl, and loaded into a 1 ml HiTrap^{®} Heparin HP column (Amersham), the bound proteins were eluted with a 12-cv linear gradient from 300 mM to 1.6 M NaCl in Buffer H. The fractions containing RBP2 complex 1 were eluted from 540 mM to 1 M NaCl, and the fractions containing RBP2 complex 2 were eluted from 540 mM to 820 mM NaCl. Both fractions were brought to a conductivity equivalent to that of Buffer C (40 mM Tris·HCl (pH 7.9), 0.5 mM EDTA, 1 mM DTT and 10% (v/v) glycerol) containing 100 mM NaCl, and loaded into a 0.6 mL MonoQ^{®} HR 5/5 (Amersham), the bound proteins were eluted with a 10-cv linear gradient from 100 mM to 500 mM KCI in Buffer C. The fractions containing RBP2 complex 1 were eluted from 350 mM to 430 mM NaCl, and the fractions containing complex 2 were eluted from 360 mM to 400 mM NaCl.

*Western Blot Analysis.* Cells were lysed in lysis buffer E (50 mM Tris (pH 7.9), 400 mM NaCl, 0.5% NP-40) supplemented with complete protease inhibitor cocktail (Roche Molecular Biochemicals). For the analysis of histones, cells were lysed in SDS lysis buffer (50 mM Tris (pH 7.9), 10 mM EDTA, 0.5% SDS) supplemented with complete protease inhibitor cocktail (Roche Molecular Biochemicals) and sonicated before loading onto the gel. Approximately 30 µg of cell extract per lane, as determined by the Bradford method, was resolved by SDS-polyacrylamide gel electrophoresis and transferred to nitrocellulose membranes. After blocking in Tris-buffered saline with 4% nonfat milk, the membranes were probed with the indicated antibodies, diluted in Tris-buffered saline with 4% nonfat milk. Bound antibody was detected with the appropriate horseradish peroxidase-conjugated goat anti-rabbit IgG or goat anti-mouse IgG (Pierce, Rockland, IL) and SuperSignal^{®} West Pico chemiluminescent substrate (Pierce) or Immobilon Western chemiluminescent substrate (Millipore^{®}) according to the manufacturer's instructions.

*Histone Demethylase Assays.* Histone demethylase assays analyzing formaldehyde release were carried out using equal counts of labeled histone substrate and fractionated cell extracts as described in Example 1. Histone demethylase assays using modified histone peptide substrates were carried out using peptides corresponding to amino acids 1-18 of histone H3 (Upstate 12-563(me1), Upstate 12-460 (me2), and Upstate 12-564(me3)).

*Immunofluorescence.* Indirect immunofluorescence was carried out using NIH3T3 cells grown in DMEM containing 10% FBS and penicillin/streptomycin. Cells were grown, permeabilized, stained and analyzed as described above.

### Example 8

### RETINOBLASTOMA BINDING PROTEIN RBP2 IS AN H3K4 DEMETHYLASE

Retinoblastoma Binding Protein 2 (RBP2), a member of the JARID1 subfamily of mammalian JmjC domain-containing proteins, was observed to contain a JmjC domain sharing extensive similarity to the JmjC domain of the JHDM3 demethylases (**Figure 20A**), which targets removal of H3K9/36 methylation and can remove the trimethyl modification state. Conservation of residues within the predicted co-factor binding sites of the RBP2 JmjC domain and the apparent role of RBP2 as a transcriptional regulator indicated that RBP2 possessed histone demethylase activity and may exhibit activity toward a histone methylation mark that was not recognized by the previously characterized JmjC family members.

Thus, endogenous RBP2 was enriched from HeLa nuclear extract by sequential fractionation over SP-Sepharose^{®}, Heparin-Sepharose^{®}, and MonoQ^{®} chromatographic columns (data not shown). RBP2 containing fractions were identified at each chromatographic step by western blot analysis using RBP2 specific antibodies. RBP2 was detected in two distinct fractions following SP-Sepharose^{®} chromatography (data not shown). Both fractions were further purified in parallel using Heparin-Sepharose^{®} and MonoQ^{®} columns (data not shown). To determine whether RBP2-containing protein complexes possess histone demethylase activity, partially purified RBP2 fractions from the MonoQ^{®} column were incubated with various labeled histone substrates and histone demethylase activity was monitored by release of labeled formaldehyde (data not shown). Formaldehyde release was only observed when RBP2-containing fractions were incubated with H3K4 labeled substrate, indicating that the RBP2-containing complexes specifically demethylate methylated H3K4.

Lysine-specific demethylase 1 (LSD1) is characterized as an H3K4 demethylase, but its catalytic requirement for a protonated nitrogen on the lysine amine group limits its enzymatic activity to H3K4me1/me2-modified substrates (Lee, et al. (2005) Nature 437:432-435; Metzger, et al. (2005) Nature 437:436-439; Shi, et al. (2004) Cell 119:941-953; Shi, et al. (2005) Mol. Cell 19:857-864). The inability of LSD1 to reverse H3K4me3 suggested that this modification state is refractory to enzymatic demethylation. In contrast to LSD1, the JmjC domain-containing histone demethylases exploit a direct hydroxylation reaction to remove histone methylation, suggesting that RBP2 might catalyze the removal of H3K4me3.

To verify that RBP2 is an H3K4 demethylase and to examine the modification state-specificity of RBP2, Flag^{®}-tagged RBP2 was expressed in SF9 cells using a baculovirus expression system and affinity purified to homogeneity. Purified Flag^{®}-RBP2 resolved as a single band following SDS-PAGE and Coomassie^{®} blue staining (**Figure 20B**). Recombinant RBP2 was then incubated with modified histone H3 peptide substrates corresponding to the H3K4 me3, me2 and me1 modification states (**Figures 20C-20E**). RBP2 displayed robust H3K4 demethylase activity against H3K4me3 and me2 (**Figures 20C** and **20D**), resulting in 80-90% demethylation of the modified substrate (**Figures 20F and 20G**), but failed to catalyze removal of the me1 modification state (**Figure 20E**). Although RBP2 failed to initiate demethylation of H3K4me1 *in vitro* (**Figure 20E**) it was capable of processively demethylating the H3K4me3 and me2 modifications to the unmodified state (**Figure 20C** and **20D**). This enzymatic property of RBP2 is similar JHDM3A, which also catalyses processive removal of all three modification states but fails to initiate demethylation on the me1 modification state *in vitro*. Together, these observations indicate that the H3K4me3 modification state is enzymatically reversible and that histone demethylation may contribute to transcriptional regulation by RBP2.

*RBP2 Demethylates H3K4 in vivo.* To demonstrate that RBP2 functions as an active H3K4 demethylase *in vivo,* a Flag^{®}-tagged RBP2 expression plasmid was transfected into NIH3T3 cells and its effect on H3K4 methylation was analyzed by indirect immunofluorescence using H3K4 methylation-specific antibodies (Figure 21). Cells over-expressing RBP2 showed a uniform reduction of H3K4me1, H3K4me2 and H3K4me3 modifications (Figures 21A-21C, top panels). Removal of H3K4 methylation was dependent upon the demethylase activity of RBP2 as a point mutation in the predicted JmjC domain iron-binding site (H483A) abrogated the H3K4 demethylation (Figures 21A-21C, bottom panels). Unexpectedly, given its inability to demethylate the H3K4me1 *in vitro,* RBP2 also efficiently catalyzed removal of the H3K4me1 *in vivo.* This observation indicates that the capacity of the RBP2 to catalyze H3K4me1-specific demethylation may be regulated by additional factors *in vivo*. Collectively, these data indicate that RBP2 is able to demethylate all the H3K4 methylation states *in vivo*, and demonstrates the H3K4me3 is a readily reversible histone modification.

The foregoing is illustrative of the present invention, and is not to be construed as limiting thereof. The invention is defined by the following claims, with equivalents of the claims to be included therein.

## Claims

1. A method of identifying a compound that modulates the demethylase activity of a demethylase comprising a JmjC domain, wherein the demethylase comprises amino acid residues that coordinate with Fe(II) and α-ketoglutarate, the method comprising:
(a) contacting the demethylase with a methylated protein substrate in the presence of a test compound, α-ketoglutarate and Fe(II); and
(b) detecting the level of demethylation of the protein substrate under conditions sufficient for demethylation, wherein a change in demethylation of the protein substrate as compared with the level of demethylation in the absence of the test compound indicates that the test compound is a modulator of the demethylase activity of the demethylase wherein the demethylase is:
(i) a JHDM1 protein, optionally JHDM1A or JHDM1 B,
(ii) a JHDM2 protein, optionally JHDM2A , JHDM2B, or JHDM2C; or
(iii) a JHDM3 protein, optionally JHDM3A.

2. The method of claim 1, wherein the method is a cell-based method.

3. The method of claim 1, wherein the method is a cell-free method.

4. The method of any of claims 1 to 3, wherein a reduction in demethylation activity as compared with the level of demethylation in the absence of the test compound indicates that the test compound is an inhibitor of the demethylase activity of the demethylase and wherein an enhancement of demethylation activity as compared with the level of demethylation in the absence of the test compound indicates that the test compound is an activator of the demethylase activity of the demethylase.

5. The method of any of the preceding claims, wherein the methylated histone substrate is a methylated core histone substrate, mononucleosome substrate, dinucleosome substrate, oligonucleosome substrate or peptide substrate.

6. The method of any of the preceding claims, wherein the methylated histone substrate is a methylated histone H3 substrate.

7. The method of claim 6 wherein the methylated histone H3 substrate is methylated at lysine 36 of histone H3 (H3-K36), lysine 9 of histone H3 (H3-K9) or lysine 4 of histone H3 (H3-K4).

8. The method of claim 7 wherein the methylated histone H3 substrate is mono- and/or di-methylated at H3-K36 or at H3-K9.

9. The method of claim 8 wherein the methylated histone H3 substrate is mono-, di- and/or tri-methylated at H3-K4.

10. The method of any of the preceding claims, wherein the demethylation is detected at lysine 36 of histone H3 (H3-K36), lysine 9 of histone H3 (H3-K9) or lysine 4 of histone H3 (H3-K4).

11. The method of any of claims 1 to 7, wherein when the demethylase is a JHDM1 protein, optionally JHDM1A or JHDM1 B, demethylation of mono- and/or di-methylated H3-K36 is detected.

12. The method of any of claims 1 to 7, wherein when the demethylase is a JHDM2 protein, optionally a JHDM2A protein, a JHDM2B protein, or a JHDM2C protein, demethylation of mono- and/or di-methylated H3-K9 is detected.

13. The method of any of claims 1 to 7, wherein when the demethylase is a JHDM3 protein, optionally a JHDM3A protein, demethylation of di- and/or tri-methylated H3-K9 is detected.

14. A method of identifying a candidate compound for treating cancer, the method comprising:
(a) contacting a histone demethylase comprising a JmjC domain with a methylated histone substrate in the presence of a test compound, α-ketoglutarate and Fe(II), wherein the histone demethylase comprising a JmjC domain is:
(i) a JHDM1 protein, optionally JHDM1A or JHDM1 B;
(ii) a JHDM2 protein, optionally a JHDM2A protein, a JHDM2B protein, or a JHDM2C protein; or
(iii) a JHDM3 protein, optionally a JHDM3A protein; and
(b) detecting the level of demethylation of the histone substrate under conditions sufficient for demethylation, wherein a change in demethylation of the histone substrate as compared with the level of demethylation in the absence of the test compound indicates that the test compound is a candidate compound for the treatment of cancer.

## Patentansprüche

1. Verfahren zum Identifizieren einer Verbindung, welche die Demethylase-Aktivität einer Demethylase moduliert, die eine JmjC-Domäne umfasst, wobei die Demethylase Aminosäurereste umfasst, die mit Fe(II) und α-Ketoglutarat koordiniert, wobei das Verfahren folgendes umfasst:
(a) das Kontaktieren der Demethylase mit einem methylierten Proteinsubstratin Gegenwart einer Testverbindung, α-Ketoglutarat und Fe(II); und
(b) das Detektieren der Höhe der Demethylierung des Proteinsubstrats unter ausreichenden Bedingungen für die Demethylierung, wobei eine Veränderung der Demethylierung des Proteinsubstrats im Vergleich zu der Höhe der Demethylierung in Abwesenheit der Testverbindung anzeigt, dass es sich bei der Testverbindung um einen Modulator der Demethylase-Aktivität der Demethylase handelt, wobei es sich bei der Demethylase um folgendes handelt:
(i) ein JHDM1-Protein, optional JHDM1A oder JHDM1B;
(ii) ein JHDM2-Protein, optional JHDM2A, JHDM2B oder JHDM2C; oder
(iii) ein JHDM3-Protein, optional JHDM3A.

2. Verfahren nach Anspruch 1, wobei es sich bei dem Verfahren um ein zellbasiertes Verfahren handelt.

3. Verfahren nach Anspruch 1, wobei es sich bei dem Verfahren um ein zellfreies Verfahren handelt.

4. Verfahren nach einem der Ansprüche 1 bis 3, wobei eine Reduktion der Demethylierungs-Aktivität im Vergleich zur Höhe der Demethylierung in Abwesenheit der Testverbindung anzeigt, dass die Testverbindung ein Hemmer für die Demethylase-Aktivität der Demethylase ist, und wobei eine Erhöhung der Demethylierungs-Aktivität im Vergleich zu der Höhe der Demethylierung in Abwesenheit der Testverbindung anzeigt, dass es sich bei der Testverbindung um einen Aktivator der Demethylase-Aktivität der Demethylase handelt.

5. Verfahren nach einem der vorstehenden Ansprüche, wobei es sich bei dem methylierten Histon-Substrat um ein methyliertes Kern-Histon-Substrat, ein Mononukleosom-Substrat, ein Dinukleosom-Substrat, ein Oligonukleosom-Substrat oder ein Peptid-Substrat handelt.

6. Verfahren nach einem der vorstehenden Ansprüche, wobei es sich bei dem methylierten Histon-Substrat um ein methyliertes Histon-H3-Substrat handelt.

7. Verfahren nach Anspruch 7, wobei das methylierte Histon-H3-Substrat mit Lysin 36 von Histon H3 (H3-K36), Lysin 9 von Histon H3 (H3-K9) oder Lysin 4 von Histon H3 (H3-K4) methyliert wird.

8. Verfahren nach Anspruch 7, wobei das methylierte Histon-H3-Substrat mono- und/oder dimethyliert wird mit H3-K36 und H3-K9.

9. Verfahren nach Anspruch 7, wobei das methylierte Histon-H3-Substrat mono-, di- und/oder trimethyliert wird mit H3-K4.

10. Verfahren nach einem der vorstehenden Ansprüche, wobei die Demethylierung detektiert wird bei Lysin 36 des Histons H3 (H3-K36), dem Lysin 9 von Histon H3 (H3-K9) oder Lysin 4 von Histon H3 (H3-K4).

11. Verfahren nach einem der Ansprüche 1 bis 7, wobei für den Fall, dass es sich bei der Demethylase um ein JHDM1-Protein handelt, optional JHDM1A oder JHDM1B, die Demethylierung von mono- und/oder dimethyliertem H3-K36 detektiert wird.

12. Verfahren nach einem der Ansprüche 1 bis 7, wobei für den Fall, dass es sich bei der Demethylase um ein JHDM2-Protein handelt, optional ein JHDM2A-Protoein, ein JHDM2B-Protein oder ein JHDM2C-Protein, die Demethylierung von mono- und/oder dimethyliertem H3-K9 detektiert wird.

13. Verfahren nach einem der Ansprüche 1 bis 7, wobei für den Fall, dass es sich bei der Demethylase um ein JHDM3-Protein handelt, optional ein JHDM3A-Protein, die Demethylierung von mono-, di- und/oder trimethyliertem H3-K9 detektiert wird.

14. Verfahren zum Identifizieren einer möglichen Verbindung zur Krebsbehandlung, wobei das Verfahren folgendes umfasst:
(a) das Kontaktieren einer Histon-Demethylase, die eine JmjC-Domäne mit methyliertem Histon-Substrat in Gegenwart einer Testverbindung, α-Ketoglutarat und Fe(II) umfasst, wobei es sich bei der Histon-Demethylase, die eine JmjC-Domäne umfasst, um folgendes handelt:
(i) ein JHDM1-Protein, optional JHDM1A oder JHDM1B;
(ii) ein JHDM2-Protein, optional ein JHDM2A-Protein, ein JHDM2B-Protein oder ein JHDM2C-Protein; oder
(iii) ein JHDM3-Protein, optional ein JHDM3A-Protein; und
(b) das Detektieren der Höhe der Demethylierung des Histon-Substrats unter ausreichenden Bedingungen für die Demethylierung, wobei eine Veränderung der Demethylierung des Histon-Substrats im Vergleich zu der Höhe der Demethylierung in Abwesenheit der Testverbindung anzeigt, dass es sich bei der Testverbindung um eine mögliche Verbindung zur Krebsbehandlung handelt:

## Revendications

1. Procédé d'identification d'un composé qui module l'activité de déméthylase d'une déméthylase comprenant un domaine JmjC, dans lequel la déméthylase comprend des résidus d'acide aminé qui se coordonnent avec Fe(II) et l'α-cétoglutarate, le procédé comprenant :
(a) la mise en contact de la déméthylase avec un substrat protéique méthylé en présence d'un composé test, d'α-cétoglutarate et de Fe(II) ; et
(b) la détection du niveau de déméthylation du substrat protéique dans des conditions suffisantes pour la déméthylation, dans lequel un changement de déméthylation du substrat protéique par rapport au niveau de déméthylation en l'absence du composé test indique que le composé test est un modulateur de l'activité de déméthylase de la déméthylase, dans lequel la déméthylase est :
(i) une protéine JHDM1, en option JHDM1A ou JHDM1B,
(ii) une protéine JHDM2, en option JHDM2A, JHDM2B ou JHDM2C ; ou
(iii) une protéine JHDM3, en option JHDM3A.

2. Procédé selon la revendication 1, dans lequel le procédé est un procédéà base cellulaire.

3. Procédé selon la revendication 1, dans lequel le procédé est un procédé exempt de cellules.

4. Procédé selon l'une quelconque des revendications 1 à 3, dans lequel une réduction de l'activité de déméthylation par rapport au niveau de déméthylation en l'absence du composé test indique que le composé test est un inhibiteur de l'activité de déméthylase de la déméthylase et dans lequel une amplification de l'activité de déméthylation par rapport au niveau de déméthylation en l'absence du composé test indique que le composé test est un activateur de l'activité de déméthylase de la déméthylase.

5. Procédé selon l'une quelconque des revendications précédentes, dans lequel le substrat d'histone méthylée est un substrat d'histone de noyau méthylée, un substrat de mononucléosome, un substrat de dinucléosome, un substrat d'oligonucléosome ou un substrat peptidique.

6. Procédé selon l'une quelconque des revendications précédentes, dans lequel le substrat d'histone méthylée est un substrat d'histone H3 méthylée.

7. Procédé selon la revendication 6, dans lequel le substrat d'histone H3 méthylée est méthylé au niveau de la lysine 36 de l'histone H3 (H3-K36), la lysine 9 de l'histone H3 (H3-K9) ou la lysine 4 de l'histone H3 (H3-K4).

8. Procédé selon la revendication 7, dans lequel le substrat d'histone H3 méthylée est mono- et/ou diméthylé à H3-K36 ou à H3-K9.

9. Procédé selon la revendication 8, dans lequel le substrat d'histone H3 méthylée est mono-, di- et/ou triméthylé à H3-K4.

10. Procédé selon l'une quelconque des revendications précédentes, dans lequel la déméthylation est détectée au niveau de la lysine 36 de l'histone H3 (H3-K36), la lysine 9 de l'histone H3 (H3-K9) ou la lysine 4 de l'histone H3 (H3-K4).

11. Procédé selon l'une quelconque des revendications 1 à 7, dans lequel lorsque la déméthylase est une protéine JHDM1, en option JHDM1A ou JHDM1B, la déméthylation de H3-K36 mono- et/ou diméthylée est détectée.

12. Procédé selon l'une quelconque des revendications 1 à 7, dans lequel lorsque la déméthylase est une protéine JHDM2, en option une protéine JHDM2A, une protéine JHDM2B ou une protéine JHDM2C, la déméthylation de H3-K9 mono- et/ou diméthylée est détectée.

13. Procédé selon l'une quelconque des revendications 1 à 7, dans lequel lorsque la déméthylase est une protéine JHDM3, en option une protéine JHDM3A, la déméthylation de H3-K9 di- et/ou triméthylée est détectée.

14. Procédé d'identification d'un composé candidat pour le traitement du cancer, le procédécomprenant :
(a) la mise en contact d'une histone déméthylase comprenant un domaine JmjC avec un substrat d'histone méthylée en présence d'un composé test, d'α-cétoglutarate et de Fe(II), dans lequel l'histone déméthylase comprenant un domaine JmjC est :
(i) une protéine JHDM1, en option JHDM1A ou JHDM1B ;
(ii) une protéine JHDM2, en option une protéine JHDM2A, une protéine JHDM2B ou une protéine JHDM2C ; ou
(iii) une protéine JHDM3, en option une protéine JHDM3A ;
et
(b) la détection du niveau de déméthylation du substrat d'histone dans des conditions suffisantes pour la déméthylation, dans lequel un changement de déméthylation du substrat d'histone par rapport au niveau de déméthylation en l'absence du composé test indique que le composé test est un composé candidat pour le traitement du cancer.
